# EUROPEAN PATENT APPLICATION

(11) **EP 3 141 235 A1**
(43) Date of publication of application: **15.03.2017**
(21) Application number: 16177674.5
(22) Date of filing: 02.12.2013
(51) Int. Cl.: A61J 1/00, A61K 9/50, A61K 9/16, A61K 31/44

(54) **N-(6-((2R,3S)-3,4-DIHYDROXYBUTAN-2-YLOXY)-2-(4-FLUOROBENZYLTHIO)PYRIMIDIN-4-YL)-3-METHYLAZETIDINE-L -SULFONAMIDE**

(30) Priority: 06.12.2012 EP 12195865; 06.12.2012 EP 12195877; 20.12.2012 EP 12198772; 03.01.2013 EP 13150181; 03.01.2013 EP 13150189; 03.01.2013 EP 13150188; 17.01.2013 EP 13151631; 17.01.2013 EP 13151751; 17.01.2013 EP 13151752; 24.01.2013 EP 13152599; 24.01.2013 EP 13152603; 24.01.2013 EP 13152606; 24.01.2013 EP 13152609; 31.01.2013 EP 13153449; 31.01.2013 EP 13153473
(62) Divisional of application: 13195300.2
(71) Applicant: IP Gesellschaft für Management mbH, 50931 Köln (DE)
(72) Inventor: Trinius, Frank, 50931 Köln (DE)

(57) **Abstract**

The invention relates to an administration unit comprising 0.1 µg to 2 g of a crystalline form or pharmaceutical acceptable salt of N-(6-((2R,3S)-3,4-dihydroxybutan-2-yloxy)-2-(4-fluorobenzylthio)pyrimidin-4-yl)-3-methylazetidine-1-sulfonamide. The invention also relates to a packaging comprising one or more such administration units.

## Description

### CROSSREFERENCES

The present application claims priorities of European patent application no. EP12195865, filed on 06 December 2012; European patent application no. EP12195877, filed on 06 December 2012; European patent application no. EP12198772, filed on 20 December 2012; European patent application no. EP13150181, filed on 03 January 2013; European patent application no. EP13150189, filed on 03 January 2013; European patent application no. EP13150188, filed on 03 January 2013; European patent application no. EP13151631, filed on 17 January 2013; European patent application no. EP13151751, filed on 17 January 2013; European patent application no. EP13151752, filed on 17 January 2013; European patent application no. EP13152599, filed on 24 January 2013; European patent application no. EP13152603, filed on 24 January 2013; European patent application no. EP13152606, filed on 24 January 2013; European patent application no. EP13152609, filed on 24 January 2013; European patent application no. EP13153449, filed on 31 January 2013; and European patent application no. EP13153473, filed on 31 January 2013.

### BACKGROUND ART

Many pharmaceuticals are marketed without any packaging. In various countries, small drug shops are a common source of medicine where stocking of prescription-only medicines and unpackaged tablets is the norm (Goodman C et al., Health Policy Plan. 2007 Nov;22(6):393-403). Furthermore, self-medication is common practice in various countries. For example, the most frequently proposed drugs to treat male urethritis are: ampicillin 250-mg capsules (44%); oxytetracyline 250-mg capsules (24%); and cotrimoxazole 450-mg tablets (12%), and these drugs are frequently sold unpackaged (Sow PSet al., Int J Infect Dis. 2002 Jun;6(2): 108-12).

In Germany and other European countries, numerous pharmaceuticals are marketed in packaging wherein every single administration unit is individually packaged in a single packaging. Examples include but are not limited to Frubiase^{®} Calcium (Boehringer Ingelheim) marketed as a liquid administration unit that is individually packaged in a glass ampoule; Orthomol Immun^{®} Direktgranulat (Orthomol) marketed as administration unit in granulate form that is individually packaged in a bag; Orthomol Vital m^{®} (Orthomol) marketed as a liquid administration unit that is individually packaged in a bottle having a lid containing an individually packaged tablet; Vitasprint^{®} (Pfizer) and Celestamine^{®} (MSD Sharp & Dohme) each marketed as a liquid administration unit that is individually packaged in a bottle; Alka-Seltzer^{®} (Bayer) marketed as effervescent tablet that is individually packaged in a bag; Aspirin® Complex (Bayer), Aspirin^{®} Effect (Bayer), Helmex^{®}

(Infectopharm), Monuril^{®} Granulat (Pierre Fabre Pharma) each marketed as administration unit in granulate form that individually packaged in a bag; ellaOne^{®} (HRA Pharma), Levonelle^{®} (Bayer Schering Pharma); Pidana^{®} (HRA Pharma), Fungata^{®} (Pfizer), and Canesten^{®} Gyn once (Bayer) each marketed as a single tablet that is individually packaged in a blister. Furthermore, numerous single-to-use syringes are marketed individually packaged.

It is an object of the invention to provide administration units containing drugs that have advantages compared to conventional administration units and conventional drugs, respectively. It is also an object of the invention to provide packaging containing administration units containing drugs that have advantages compared to conventional packaging. These objects have been achieved by the present invention.

### SUMMARY OF THE INVENTION

The present invention relates to different drugs (active pharmaceutical ingredients) and certain forms of said drugs, such as stereoisomers, salts and/or polymorphs thereof. The present invention covers various aspects that are numbered (v), (vii), (viii), (ix), and (xi). Aspects (v), (vii), (viii), (ix), and (xi) are separate from one another.

The present invention relates to
⊙ an administration unit comprising (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d};;
⊙ a packaging comprising one or more of such administration units;
⊙ a method for manufacturing such an administration unit;
⊙ a method for manufacturing such a packaging; and
⊙ particles of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.

Certain embodiments of the invention are also disclosed in WO 2012/164286, WO 2012/166420, WO 2013/001294, WO 2013/003249, WO 2013/003386, WO 2012/172449, WO 2013/008002, WO 2013/007518, WO 2013/007519, WO 2013/012909, WO 2013/013114, WO 2013/011402, WO 2013/012118, WO 2013/016155, and WO 2013/016156, respectively. Any embodiment disclosed in any of these references is incorporated herein by reference as a preferred embodiment of the invention.

### GENERAL DEFINITIONS

To avoid unnecessary repetitions, the description refers to the following abbreviations the meaning of which is defined hereinafter:
Aspect (v) of the present invention relates to 3,5-Diamino-6-chloro-N-(N-(4-(4-(2-(hexyl(2,3,4,5,6-pentahydroxyhexyl)-amino)ethoxy)phenyl)butyl)carbamimidoyl)pyrazine-2-carboxamide and pharmaceutical acceptable salts thereof, abbreviated as '{drug5}'. In particular, the present invention relates to one or more stereoisomer(s) of 3,5-Diamino-6-chloro-N-(N-(4-(4-(2-(hexyl(2,3,4,5,6-pentahydroxyhexyl)amino)ethoxy)phenyl)butyl)carbamimidoyl)pyrazine-2-carboxamide and/or pharmaceutical salts thereof, abbreviated as '{drug5a}'. {drug5a} is a specific form falling within the definition of {drug5}. Aspect (v) of the invention is separate from aspects (vii), (viii), (ix), and (xi) of the invention. Thus, all embodiments of {drug5a} are only related to {drug5}, but neither to {drug1}, nor to {drug2}, nor to {drug3}, nor to {drug4}, nor to {drug6}, nor to {drug7}, nor to {drug8}, nor to {drug9}, nor to {drug10}, nor to {drug11}, nor to {drug12}, nor to {drug13}, nor to {drug14}, nor to {drug15}.
Aspect (vii) of the present invention relates to N-(6-((2R,3S)-3,4-dihydroxybutan-2-yloxy)-2-(4-fluorobenzylthio)pyrimidin-4-yl)-3-methylazetidine-1 -sulfonamide, abbreviated as '{drug7}'. In particular, the present invention relates to crystalline form or pharmaceutical acceptable salt of N-(6-((2R,3S)-3,4-dihydroxybutan-2-yloxy)-2-(4-fluorobenzylthio)pyrimidin-4-yl)-3-methylazetidine-1-sulfonamide, abbreviated as '{drug7a}', and to crystalline form or pharmaceutical acceptable salt of N-(6-((2R,3S)-3,4-dihydroxybutan-2-yloxy) -2-(4-fluorobenzylthio)pyrimidin-4-yl)-3-methylazetidine-1-sulfonamide with at least one peak at 2-Theta (in degrees) selected from 8.5, 9.7, 10.6, 17.1, 19.9, and 21.2, abbreviated as '{drug7b}'. {drug7a} and {drug7b} are specific forms falling within the definition of {drug7}. Aspect (vii) of the invention is separate from aspects (v), (viii), (ix), and (xi) of the invention. Thus, all embodiments of {drug7a} and {drug7b}, respectively, are only related to {drug7}, but neither to {drug1}, norto {drug2}, norto {drug3}, norto {drug4}, nor to {drug5}, nor to {drug6}, nor to {drug8}, nor to {drug9}, nor to {drug10}, nor to {drug11}, nor to {drug12}, nor to {drug13}, nor to {drug14}, nor to {drug15}.
Aspect (viii) of the present invention relates to 6-(Piperidin-4-yloxy)-2H-isoquinolin-1-one hydrochloride, abbreviated as '{drug8}'. In particular, the present invention relates to 6-(Piperidin-4-yloxy)-2H-isoquinolin-1-one hydrochloride hydrate, abbreviated as '{drug8a}', to 6-(Piperidin-4-yloxy)-2H-isoquinolin-1-one hydrochloride dihydrate, abbreviated as '{drug8b}', to 6-(Piperidin-4-yloxy)-2H-isoquinolin-1-one hydrochloride polymorph 1, abbreviated as '{drug8c}', to 6-(Piperidin-4-yloxy)-2H-isoquinolin-1-one hydrochloride polymorph 2, abbreviated as '{drug8d}', to 6-(Piperidin-4-yloxy)-2H-isoquinolin-1-one hydrochloride polymorph 3, abbreviated as '{drug8e}', to 6-(Piperidin-4-yloxy)-2H-isoquinolin-1-one hydrochloride polymorph 4, abbreviated as '{drug8f}', to 6-(Piperidin-4-yloxy)-2H-isoquinolin-1-one hydrochloride 1,4-dioxane solvate, abbreviated as '{drug8g}', to 6-(Piperidin-4-yloxy)-2H-isoquinolin-1-one hydrochloride acetonitrile solvate, abbreviated as '{drug8h}', and to 6-(Piperidin-4-yloxy)-2H-isoquinolin-1-one hydrochloride methyl acetate solvate, abbreviated as'{drug8i}'. {drug8a}, {drug8b}, {drug8c}, {drug8d}, {drug8e}, {drug8f}, {drug8g}, {drug8h}, and {drug8i} are specific forms falling within the definition of {drug8}. Aspect (viii) of the invention is separate from aspects (v), (vii), (ix), and (xi) of the invention. Thus, all embodiments of {drug8a}, {drug8b}, {drug8c}, {drug8d}, {drug8e}, {drug8f}, {drug8g}, {drug8h}, and {drug8i}, respectively, are only related to {drug8}, but neither to {drug1}, nor to {drug2}, nor to {drug3}, nor to {drug4}, nor to {drug5}, nor to {drug6}, nor to {drug7}, nor to {drug9}, nor to {dmg10}, nor to {drug11}, norto {drug12}, nor to {drug13}, nor to {drug14}, nor to {drug15}.
Aspect (ix) of the present invention relates to 6-(Piperidin-4-yloxy)-2H-isoquinolin-1-one hydrochloride, abbreviated as '{drug9}'. In particular, the present invention relates to crystalline polymorph of 6-(Piperidin-4-yloxy)-2H-isoquinolin-1-one hydrochloride wherein the polymorph exhibits in an X-ray powder diffractogram using CuKa radiation at least a characteristic reflection within the two ranges selected from 1) 15.4 -15.8 and 2) 16.5 -16.8 ± 0.2 degrees 2theta, abbreviated as '{drug9a}'. {drug9a} is a specific form falling within the definition of {drug9}. Aspect (ix) of the invention is separate from aspects (v), (vii), (viii), and (xi) of the invention. Thus, all embodiments of {drug9a} are only related to {drug9}, but neither to {drug1}, {drug2}, nor to {drug3}, nor to {drug4}, nor to {drug5}, nor to {drug6}, nor to {drug7}, nor to {drug8}, nor to {drug10}, nor to {drug11}, nor to {drug12}, nor to {drug13}, nor to {drug14}, nor to {drug15}.
Aspect (xi) of the present invention relates to a compound of the formula (I) or a stereoisomer or pharmaceutically acceptable salt from thereof 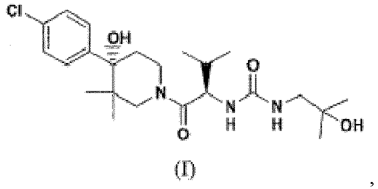 abbreviated as '{drug 11}'. In particular, the present invention relates to an amorphous solid dispersion comprising compound of the formula (I) or a stereoisomer or pharmaceutically acceptable salt from thereof and a PVP polymer, abbreviated as '{drug11a}'; to an amorphous solid dispersion comprising Compound of the formula (I) or a stereoisomer or pharmaceutically acceptable salt from thereof and a PVP-vinylacetatcopolymer, abbreviated as '{drug11b}'; to an amorphous solid dispersion comprising Compound of the formula (I) or a stereoisomer or pharmaceutically acceptable salt from thereof and a HPMC-AS, abbreviated as '{drug11c}'; and to an amorphous solid dispersion comprising Compound of the formula (I) or a stereoisomer or pharmaceutically acceptable salt from thereof and HPMC, abbreviated as '{drug11d}', {drug11a}, {drug11b}, {drug11c}, and {drug11d} are specific forms falling within the definition of {drug11}. Aspect (xi) of the invention is separate from aspects (v), (vii), (viii), and (ix) of the invention. Thus, all embodiments of {drug11a}, {drug11b}, {drug11c}, and {drug11d}, respectively, are only related to {drug11}, but neither to {drug1}, nor to {drug2}, nor to {drug3}, nor to {drug4}, nor to {drug5}, nor to {drug6}, nor to {drug7}, nor to {drug8}, nor to {drug9}, nor to {drug10}, nor to {drug12}, nor to {drug13}, nor to {drug14}, nor to {drug15}.

Summarizing therefore the following abbreviations have the following meanings:

### Aspect (v):

{drug5} = 3,5-Diamino-6-chloro-N-(N-(4-(4-(2-(hexyl(2,3,4,5,6-pentahydroxyhexyl)amino)ethoxy)phenyl)-butyl)carbamimidoyl)pyrazine-2-carboxamide and pharmaceutical acceptable salts thereof;
{drug5a} = one or more stereoisomer(s) of 3,5-Diamino-6-chloro-N-(N-(4-(4-(2-(hexyl(2,3,4,5,6-pentahydroxyhexyl)-amino)ethoxy)phenyl)butyl)carbamimidoyl)pyrazine-2-carboxamide and/or pharmaceutical salts thereof.

### Aspect (vii):

{drug7} = N-(6-((2R,3S)-3,4-dihydroxybtttan-2-yloxy) -2-(4-fluorobenzylthio)pyrimidin-4-yl)-3-methylazetidine-1-sulfonamide;
{drug7a} = crystalline form or pharmaceutical acceptable salt of N-(6-((2R,3S)-3,4-dihydroxybutan-2-yloxy) -2-(4-fluorobenzylthio)pyrimidin-4-yl)-3-methylazetidine-1-sulfonamide;
{drug7b} = crystalline form or pharmaceutical acceptable salt of N-(6-((2R,3S)-3,4-dihydroxybutan-2-yloxy) -2-(4-fluorobenzylthio)pyrimidin-4-yl)-3-methylazetidine-1-sulfonamide with at least one peak at 2-Theta (in degrees) selected from 8.5, 9.7, 10.6, 17.1, 19.9, and 21.2 (Form A).

### Aspect (viii):

{drug8} = 6-(Piperidin-4-yloxy)-2H-isoquinolin-1-one hydrochloride;
{drug8a} = 6-(Piperidin-4-yloxy)-2H-isoquinolin-1-one hydrochloride hydrate;
{drug8b} = 6-(Piperidin-4-yloxy)-2H-isoquinolin-1-one hydrochloride dihydrate;
{drug8c} = 6-(Piperidin-4-yloxy)-2H-isoquinolin-1-one hydrochloride polymorph 1;
{drug8d} = 6-(Piperidin-4-yloxy)-2H-isoquinolin-1-one hydrochloride polymorph 2;
{drug8e} = 6-(Piperidin-4-yloxy)-2H-isoquinolin-1-one hydrochloride polymorph 3;
{drug8f} = 6-(Piperidin-4-yloxy)-2H-isoquinolin-1-one hydrochloride polymorph 4;
{drug8g} = 6-(Piperidin-4-yloxy)-2H-isoquinolin-1-one hydrochloridel,4-dioxane solvate;
{drug8h} = 6-(Piperidin-4-yloxy)-2H-isoquinolin-1-one hydrochloride acetonitrile solvate;
{drug8i} = 6-(Piperidin-4-yloxy)-2H-isoquinolin-1-one hydrochloride methyl acetate solvate.

### Aspect (ix):

{drug9} = 6-(Piperidin-4-yloxy)-2H-isoquinolin-1-one hydrochloride;
{drug9a} = crystalline polymorph of 6-(Piperidin-4-yloxy)-2H-isoquinolin-1-one hydrochloride wherein the polymorph exhibits in an X-ray powder diffractogram using CuKa radiation at least a characteristic reflection within the two ranges selected from 1) 15.4 -15.8 and 2) 16.5 -16.8 ± 0.2 degrees 2theta.

### Aspect (xi):

{drug11} = compound of the formula (I) or a stereoisomer or pharmaceutically acceptable salt from thereof; 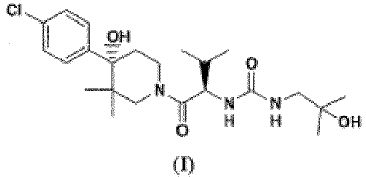
{drug11a} = amorphous solid dispersion comprising compound of the formula (I) or a stereoisomer or pharmaceutically acceptable salt from thereof and a PVP polymer;
{drug11b} = amorphous solid dispersion comprising compound of the formula (I) or a stereoisomer or pharmaceutically acceptable salt from thereof and a PVP-vinylacetatcopolymer;
{drug11c} = amorphous solid dispersion comprising compound of the formula (I) or a stereoisomer or pharmaceutically acceptable salt from thereof and a HPMC-AS;
{drug11d} = amorphous solid dispersion comprising compound of the formula (I) or a stereoisomer or pharmaceutically acceptable salt from thereof and HPMC.

### SPECIFIC INORMATION CONCERNING ASPECT (V) OF THE INVENTION

Aspect (v) of the invention concerns forms of {drug5}, especially a form of 3,5-diamino-6-chloro-N-(N-(4-(4-(2-(hexyl(2,3,4,5,6-pentahydroxyhexyl)amino)ethoxy)phenyl)butyl)carbamimidoyl)pyrazine-2-carboxamide and pharmaceutical acceptable salts thereof, namely to one or more stereoisomer(s) of 3,5-diamino-6-chloro-N-(N-(4-(4-(2-(hexyl(2,3,4,5,6-pentahydroxyhexyl)amino)ethoxy)phenyl)butyl)carbamimidoyl)pyrazine-2-carboxamide and/or pharmaceutical salts therof, which is useful for treating chronic obstructive pulmonary disease (COPD), asthma, bronchiectasis, acute and chronic bronchitis, cystic fibrosis, emphysema, and pneumonia.

One or more stereoisomer(s) of 3,5-diamino-6-chloro-N-(N-(4-(4-(2-(hexyl(2,3,4,5,6-pentahydroxyhexyl)amino)ethoxy)-phenyl)butyl)carbamimidoyl)pyrazine-2-carboxamide and/or pharmaceutical salts therof is described in WO 2013/003386, which is incorporated by reference and which describes that the invention relates to the compound 3,5-Diamino-6-chloro-N-(N-(4-(4-(2-(hexyl(2,3,4,5,6-pentahydroxyhexyl)amino)ethoxy)phenyl)butyl)carbamimidoyl)pyrazine-2-carboxamide and pharmaceutical acceptable salts thereof of the formula shown below or pharmaceuticaily acceptable salts thereof, as well as compositions containing the same, processes for the preparation of the same, and therapeutic methods of use therefore in promoting hydration of mucosal surfaces and the treatment of diseases including chronic obstructive pulmonary disease (COPD), asthma, bronchiectasis, acute and chronic bronchitis, cystic fibrosis, emphysema, and pneumonia.

As used herein, one or more stereoisomer(s) of 3,5-diamino-6-chloro-N-(N-(4-(4-(2-(hexyl(2,3,4,5,6-pentahydroxyhexyl)-amino)ethoxy)phenyl)butyl)carbamimidoyl)pyrazine-2-carboxamide and/or pharmaceutical salts therof is a form of 3,5-diamino-6-chloro-N-(N-(4-(4-(2-(hexyl(2,3,4,5,6-pentahydroxyhexyl)amino)ethoxy)phenyl)butyl)carbamimidoyl)pyrazine-2-carboxamide and pharmaceutical acceptable salts thereof. 3,5-diamino-6-chloro-N-(N-(4-(4-(2-(hexyl(2,3,4,5,6-pentahydroxyhexyl)amino)ethoxy)phenyl)butyl)carbamimidoyl)pyrazine-2-carboxamide and pharmaceutical acceptable salts thereof can be also described by formula (I): 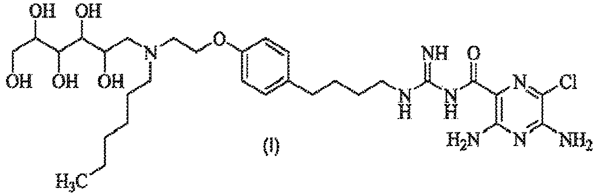 optionally in form of pharmaceutical acceptable salts.

The invention relates to an administration unit comprising one or more stereoisomer(s) of3,5-Diamino-6-chloro-N-(N-(4-(4-(2-(hexyl(2,3,4,5,6-pentahydroxyhexyl)amino)ethoxy)phenyl)butyl)carbamimidoyl)pyrazine-2-carboxamide and/or pharmaceutical salts thereof. The administration unit according to the invention is useful for treating various diseases and disorders which include but are not limited to chronic obstructive pulmonary disease (COPD), asthma, bronchiectasis, acute and chronic bronchitis, cystic fibrosis, emphysema, and pneumonia.

### SPECIFIC INORMATION CONCERNING ASPECT (VII) OF THE INVENTION

Aspect (vii) of the invention concerns forms of {drug7}, especially a solid form of N-(6-((2R,3S)-3,4-dihydroxybutan-2-yloxy)-2-(4-fluorobenzylthio)pyrimidin-4-yl)-3-methylazetidine-1-sulfonamide, namely to crystalline form or pharmaceutical acceptable salt of N-(6-((2R,3S)-3,4-dihydroxybutan-2-yloxy)-2-(4-fluorobenzylthio)pyrimidin-4-yl)-3-methylazetidine-1-sulfonamide, which is useful for treating of a chemokine mediated disease state, asthma, allergic rhinitis, chronic obstructive pulmonary disease, inflammatory bowel disease, irritable bowel syndrome, osteoarthritis, osteoporosis, rheumatoid arthritis or psoriasis. N-(6-((2R,3S)-3,4-dihydroxybutan-2-yloxy)-2-(4-fluorobenzylthio)pyrimidin-4-yl)-3-methylazetidine-1-sulfonamide can be decribed by formula:

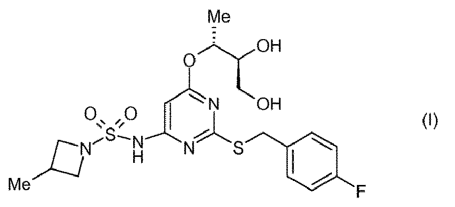

Crystalline forms or pharmaceutical acceptable salt of N-(6-((2R,3S)-3,4-dihydroxybutan-2-yloxy) -2-(4-fluorobenzylthio)-pyrimidin-4-yl)-3-methylazetidine-1-sulfonamide is described in WO 2013/008002, which is incorporated by reference. As used herein, crystalline form or pharmaceutical acceptable salt of N-(6-((2R,3S)-3,4-dihydroxybutan-2-yloxy)-2-(4-fluorobenzylthio)pyrimidin-4-yl)-3-methylazetidine-1-sulfonamide is preferred a crystalline form of N-(6-((2R,3S)-3,4-dihydroxybutan-2-yloxy)-2-(4-fluorobenzylthio)pyrimidin-4-yl)-3-methylazetidine-1-sulfonamide characterized by X-ray powder diffraction pattern, measured using a wavelength of X-rays 1.5418 A, with at least one peak at 2-Theta (in degrees) selected from 8.5, 9.7, 10.6, 17.1, 19.9, and 21.2.

Further preferred embodiments are the claims of WO 2013/008002, repeated as items 1 to 19 hereinafter:
1. A compound which is (a) a pyrimidine sulfonamide of formula (I), or (b) a pharmaceutically acceptable salt thereof: 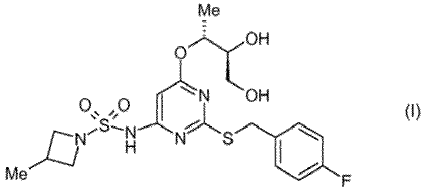
2. A compound according to item 1 of the formula (I).
3. A pharmaceutical composition which comprises a compound as defined in either item 1 or 2, and a pharmaceutically acceptable adjuvant, diluent or carrier.
4. A compound as defined in either item 1 or 2 for use in therapy.
5. A compound as defined in either item 1 or 2 in the manufacture of a medicament for use in therapy.
6. A compound as defined in either item 1 or 2 for the treatment of a chemokine mediated disease state.
7. A compound as defined in either item 1 or 2 for the treatment of asthma, allergic rhinitis, chronic obstructive pulmomary disease, inflammatory bowel disease, irritable bowel syndrome, osteoarthritis, osteoporosis, rheumatoid arthritis or psoriasis.
8. A method of treating a chemokine mediated disease state in a mammal suffering from, or at risk of, said disease, which comprises administering to a mammal in need of such treatment a therapeutically effective amount of a compound as claimed in either item 1 or 2.
9. A compound as defined in item 2 in a crystalline form.
10. A crystalline form as defined in item 9, characterised by an X-ray powder diffraction pattern, measured using a wavelength of X-rays 1.5418 A, with at least one peak at 2-Theta (in degrees) selected from 8.5, 9.7, 10.6, 17.1, 19.9, and 21.2.
11. A crystalline form as defined in item 9, characterised by an X-ray powder diffraction pattern, measured using a wavelength of X-rays 1.5418 A, with at least two or more peaks at 2-Theta (in degrees) selected from 8.5, 9.7, 10.6, 17.1, 19.9, and 21.2.
12. A crystalline form as defined in item 9, characterised by an X-ray powder diffraction pattern, measured using a wavelength of X-rays 1.5418 A, with at least three peaks at 2-Theta (in degrees) selected from 8.5, 9.7, 10.6, 17.1, 19.9, and 21.2.
13. A crystalline form as defined in item 9, characterised by an X-ray powder diffraction pattern, measured using a wavelength of X-rays 1.5418 A, with peaks at 2-Theta (in degrees) of 8.5, 9.7, 10.6, 17.1, 19.9, and 21.2.
14. A crystalline form as defined in item 9, characterised in that said form has an X-ray powder diffraction pattern, measured using a wavelength of X-rays 1.5418 A, substantially as shown in Figure 1 of WO 2013/008002.
15. A compound which is (a) an azetidine sulfonamide of formula (VIII), or (b) a salt thereof: 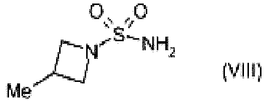
16. A process for preparing a compound of the formula (VI) 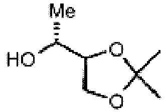 from a compound of the formula (XII): 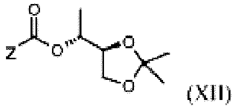 wherein Z is selected from 3,4,5-tri-Ci_₄alkyoxyphenyl, 4-phenylphenyl,4-Ci_₄alkyoxyphenyl, 2-nitrophenyl, 4-nitrophenyl and 3,5-dinitrophenyl.
17. A process according to item 16, wherein the compound of formula (XII) is prepared from a compound of formula (XI): 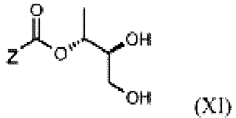 wherein Z is as defined in item 16.
18. A compound of the formula (XI) or a salt thereof: 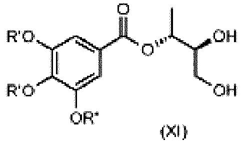 wherein R' is C₁₋₄alkyl.
19. A compound of the formula (XII) or a salt thereof: 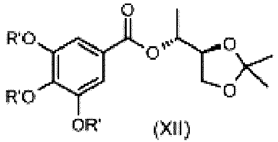 wherein R' is C₁₋₄alkyl.

As an example crystalline form or pharmaceutical acceptable salt of N-(6-((2R,3S)-3,4-dihydroxybtttan-2-yloxy) -2-(4-fluorobenzylthio)pyrimidin-4-yl)-3-methylazetidine-1-sulfonamide can be obtained by (as shown in WO 2013/008002 and cited here):
Example 1: N-(6-((2R,3S)-3,4-Dihydroxybutan-2-yloxy)-2-(4-fluorobenzylthio)pyrimidin-4-yl)-3-methylazetidine-1-sulfonamide

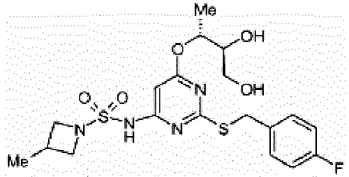

i) 2-(4-Fluorobenzylthio)pyrimidine-4,6-diol: Sodium acetate (113 g) was added to a suspension of 2-mercaptopyrimidine-4,6-diol (80 g) in water (900 mL) at room temperature. A solution of 1-(bromomethyl)-4-fluorobenzene (105 g) in acetonitrile (90 mL) was added dropwise over 2 hours. The reaction was allowed to stir for 20 hours before the suspension was filtered and washed with water (3x) and isohexane (3x). The solid was dried in vacuo for 2 hours and azeotroped with toluene (3x) to afford the sub-title product (125 g) as a white solid. ¹H NMR (500 MHz, d₆-DMSO) δ 11.62 (s, 2H), 7.57 - 7.36 (m, 2H), 7.14 (dd, J = 6.0, 14.8 Hz, 2H), 5.18 (s, 1H), 4.37 (d, J = 6.5 Hz, 2H). ii) 4,6-Dichloro-2-(4-fluorobenzylthio)pyrimidine: Phosphorus oxychloride (92 mL) was added to a suspension of the sub-title product of step (i) (100 g) and benzyltriethylammonium chloride (9 g) in dimethoxyethane (500 mL) and heated at reflux for 10 hours. The reaction was carefully poured onto stirred ice-water, partitioned between water (400 mL) and ethyl acetate (400 mL) and the organics recovered, dried over magnesium sulfate, filtered and evaporated. The crude product was purified by flash silica chromatography, elution gradient 1-40 % dichloromethane in isohexane. Pure fractions were evaporated to dryness to afford the sub-title product (86 g) as a red oil. ¹H NMR (500 MHz, CDCl₃) δ 7.46 - 7.34 (m, 2H), 7.08 - 6.94 (m, 3H), 4.34 (s, 2H). iii) 4-Chloro-6-((R)-1-((S)-2,2-dimethyl-1,3-dioxolan-4-yl)ethoxy)-2-(4-fluorobenzylthio)pyrimidine: The sub-title product of step (ii) (85.7 g) and 60% sodium hydride (14.2 g) were suspended in tetrahydrofuran (1000 mL) and cooled in ice/water for 30 minutes. A solution of (R)-1-((S)-2,2-Dimethyl-1,3-dioxolan-4-yl)ethanol (Intermediate A) in 2-methyltetrahydrofuran (53% w/v) (104 mL) was added dropwise over 20 minutes and the reaction was stirred at 0 °C to room temperature for 20 hours. The reaction was partitioned between water (500 mL) and ethyl acetate (500 mL). The aqueous was re-extracted with ethyl acetate (2x500 mL) and the combined organics were dried and evaporated in vacuo. The crude material was purified by flash silica chromatography with gradient elution 0-30% ethyl acetate in isohexane using Isolera LS. Pure fractions were evaporated to dryness to afford to afford the sub-title product (94 g) as a yellow oil. ¹H NMR (500 MHz, CDCl₃) δ 7.43 - 7.35 (m, 2H), 7.04 - 6.95 (m, 2H), 6.41 (d, J = 5.3 Hz, 1H), 5.27 - 5.17 (m, 1H), 4.33 (s, 2H), 4.20 - 4.15 (m, 1H), 4.07 - 4.02 (m, 1H), 3.83 -3.76 (m, 1H), 1.39 (dd, J = 6.9, 27.4 Hz,6H), 1.31 (d, J = 6.3 Hz, 3H). iv) N-(6-((R)-1-((S)-2,2-Dimethyl-1,3-dioxolan-4-yl)ethoxy)-2-(4-fluorobenzyl-thio)pyrimidin-4-yl)-3-methylazetidine-1-sufonamide: A solution of the sub-title product of step (iii) (94 g) dissolved in dioxane (700 mL) was treated with 3 -methylazetidine-1 -sulfonamide (Intermediate B) (42.5 g), potassium carbonate (65.1 g), dicyclohexyl(2',4',6'-triisopropylbiphenyl-2-yl)phosphine (11.2 g) and tris(dibenzylideneacetone)dipalladium(0) (10.8 g) under nitrogen. The resulting mixture was stirred at 100 °C for 60 minutes. The reaction mixture was diluted with water (500 mL) and extracted with ethyl acetate (500 mL). The organics were dried over magnesium sulfate, filtered and evaporated in vacuo to afford crude product. The crude product was purified by flash silica chromatography with elution 30% ethyl acetate in isohexane. Pure fractions were evaporated to dryness to afford the sub-title product (98 g) as a red oil. m/z [M+H]⁺= 513 (calc=512) (APCI) v) N-(6-((2R,3S)-3,4-Dihydroxybutan-2-yloxy)-2-(4-fluorobenzylthio)pyrimidin-4-yl)-3-methylazetidine-1-sulfonamide: The sub-title product of step (v) (98 g) was stirred in dichloromethane (200 mL) at 0°C and trifluoroacetic acid (200 mL) added. The reaction was allowed to warm to room temperature and was stirred for a further 18 hours (formed the TFA ester of the alcohol). The volatiles were removed in vacuo and the residue diluted in methanol (20 mL) and treated with 7 M ammonia in methanol (100 mL). The solution was stirred for 20 minutes and then evaporated to dryness to afford crude product. The crude product was purified by flash silica chromatography with gradient elution 50% to 100% ethyl acetate in isohexane. Pure fractions were evaporated to afford the title product as a white solid which was crystallised from acetonitrile to give a white crystalline solid (46.8 g). m/z [M+H]⁺ = 473 (calc=472) (APCI) ¹H NMR (500 MHz, d₆-DMSO) δ 11.06 (s, 1H), 7.49 (dd, 2H), 7.13 (t, 2H), 6.09 (s, 1H), 5.28 - 5.18 (m, 1H), 4.93 (d, 1H), 4.65 (t, 1H), 4.37 (q, 2H), 3.97 (t, 2H), 3.70- 3.60 (m, 1H), 3.58 - 3.49 (m, 2H), 3.37 (t, 2H), 2.59 (td, 1H), 1.20 (d, 3H), 1.09 (d, 3H). Crystals of the title product of Example 1 have been analysed by XRPD. The results are shown in Figure 1 of WO 2013/008002 and some of the characteristic peaks in the XRPD-diffractogram are tabulated below (RI represents relative intensity). A number of weak and very weak peaks have been omitted from the table. Due to preferred orientation effects some of the weak omitted peaks may become more significant. Position RI Position RI

| **Position °2-Theta** | **RI** | **Position °2-Theta** | **RI** |
|---|---|---|---|
| 8.5 | vs | 21.2 | s |
| 9.7 | vs | 23.3 | w |
| 10.6 | s | 23.5 | w |
| 12.9 | w | 24.0 | w |
| 16.1 | m | 25.7 | w |
| 17.1 | s | | |
| 19.9 | vs | | |
| 21.1 | m | | |

| | | | |
|---|---|---|---|
| Abbreviations: vs = very strong; s = strong; m = medium; w = weak | | | |

The differential scanning calorimetry profile of the title product of Example 1 is shown in Figure 2 of WO 2013/008002.

### Intermediate A

### (R)-1-((S)-2,2-Dimethyl-1,3-dioxolan-4-yl)ethanol

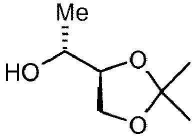

i) 5,6-O-Isopropylidene-L-ascorbic acid: To a mixture of L-ascorbic acid (65 kg, 369 mol), acetone (283 kg) and 2,2-dimethoxypropane (46 kg, 443 mol) was charged /p-toluenesulfonic acid (1.1 kg, 5.5 mol). Temperature was adjusted to 25±5°C. The slurry was stirred for 2 hours, during which time nitrogen was frequently flushed through the bottom valve to prevent material from settling at the bottom of the reactor. NMR analysis (solvent: D₂0) then showed 98.5 % conversion. Heptanes (222 kg) were charged and the temperature adjusted to 5±5°C. The reaction mixture was stirred for at least 30 minutes before filtering. Remains of the acetonide product in the reactor were rinsed onto the filter cake using the mother liquors. The filter cake was washed with heptanes (111 kg) and dried at 50°C to give 5,6-O-isopropylidene-L-ascorbic acid (73 kg, 336 mol) as an almost white powder. Yield: 91%. ¹H NMR (400 MHz, d₆-DMSO, with maleic acid and TFA) δ 4.71 (d, J= 3.0 Hz, 1H), 4.28 (m, 1H), 4.11 (dd, J= 7.0, 8.4 Hz, 1H), 3.90 (dd, J= 6.3, 8.4 Hz, 1H), 1.27 (s, 6H). ii) (R)-Methyl 2-((S)-2,2-dimethyl-1,3-dioxolan-4-yl)-2-hydroxyacetate: 5,6-O-Isopropylidene-L-ascorbic acid (58.8 kg, 272 mol) was charged to sodium hydroxide solution (27.5 kg, 50%, 340 mol) diluted with water (294 kg), and the temperature was adjusted to 30 ± 5°C. Sodium bicarbonate (57 kg, 680 mol) was charged and the mixture was agitated for 15 minutes before the temperature was increased to 40 ± 5°C. Hydrogen peroxide 35% (55 kg, 562 mol) was added to the mixture at 35 - 60°C over a period of more than 60 minutes. The reaction mixture was agitated for two hours before NMR analysis (solvent: D₂0) showed <1% residual starting material. Sodium sulfite (4.2 kg, 33 mol) was charged to the reactor and after stirring for 30 minutes, a test for peroxides was negative. After charging more sodium bicarbonate (34 kg, 408 mol), the mixture was heated to 70 ± 5°C and agitated for at least one hour before NMR analysis (solvent: D₂0) showed 98.5% conversion to the next intermediate, (2R)-[(4S)-2,2-dimethyl-1,3-dioxolan-4-yl](hydroxy)ethanoic acid. Approximately 150 L of water was stripped off under reduced pressure before filtering off salts. The filter cake was washed with water (30 L). NMP (330 kg) was charged to the combined mother liquors/wash and the temperature was adjusted to 30 ± 5°C. Methyl iodide (83 kg, 585 mol) was charged and the reactor closed. The temperature was adjusted to 55 ± 5°C and the reaction mixture was left to react for at least 120 minutes before NMR analysis (solvent: D₂0) showed 6% of the residual hydroxy ethanoic acid intermediate. Sodium sulfite (56 kg, 446 mol) dissolved in water (147 kg) was charged and the mixture was agitated for 30 minutes. The solution was extracted four times for 10 minutes at 30 ± 10°C using 406 kg toluene in each extraction. The combined organic phase was concentrated by stripping off solvent, under reduced pressure and a maximum temperature of 70°C, until a residual volume of approximately 350 L was reached. The solution was cooled to below 30°C and transferred to steel barrels over a Millipore filter to give (R)-methyl 2-((S)-2,2-dimethyl-1,3-dioxolan-4-yl)-2-hydroxyacetate solution in toluene (359 kg, 9.4%, 177 mol). Yield: 65%. ¹H NMR consistent with commercially available sample of the sub-title product, iii) (R)-Methyl 2-((S)-2,2-dimethyl- 1,3-dioxolan-4-yl)-2-(tosyloxy)acetate: From (R)-Methyl 2-((S)-2,2-dimethyl-1,3-dioxolan-4-yl)-2-hydroxyacetate solution in toluene (359 kg, 9.4%, 177 mol), toluene was distilled off under reduced pressure and a maximum temperature of 70°C until condensation ceased. Acetonitrile (153 kg) was charged and the temperature was adjusted to 25 ± 5°C. Triethylamine (41 kg, 405 mol), 4-(dimethyl amino)pyridine (1.12 kg, 9.2 mol) and then, over about 30 minutes, a solution of p-toluenesultonyl chloride (52.5 kg, 276 mol) in acetonitrile (146 kg) were added at 25 ± 5°C. After stirring the reaction mixture for an additional three hours, NMR analysis (solvent: ₆-DMSO) showed acceptable conversion (94%). MTBE (235 kg) and water (326 kg) were charged and the two-phase system was agitated for about 3 hours, after which time HPLC analysis showed the level of p-toluenesulfonyl chloride to be <0.1% of total peak area. The temperature was adjusted to 25 ± 5°C and then allowed to separate for 15 minutes. The aqueous phase was taken and extracted with further MTBE (156 kg) before discarding. The 2 organic phases were pooled together and washed with water (326 kg). Then the organic phase was washed 4 times with sodium chloride (16 kg each portion) solution in water (140 kg each portion), each for 5 - 10 minutes at 25 ± 5°C. Then the organic phase was washed twice with water (185 kg per portion) each for 5 - 10 minutes at 25 ± 5°C. NMR analysis (solvent: ₆-DMSO) then showed < 2% NMP (residual from the starting solution), by moles relative to the sulfonate ester intermediate. Activated carbon (6.0 kg) was charged and the slurry was agitated for 15 minutes at 25 ± 5°C before the carbon was filtered off in two parallel bag filter. A cartridge filter of 0.6µ 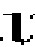 η was used after the bag filters. The filters and pipes were rinsed with MTBE (27 kg). The mother liquors and rinse were combined and reduced in volume by stripping off solvent, under reduced pressure and a maximum temperature of 50°C, until condensation ceased. Heptanes (106 kg) was charged and the solution was reduced once again by stripping off solvent, under reduced pressure and a maximum temperature of 50°C, until condensation ceased, leaving about 60 L solution in the reactor. MTBE (185 kg) was charged followed, after adjusting the temperature to 25 ± 5°C, by heptanes (75 kg). The solution was cooled to 0 - 5°C over no less than 30 minutes and heptanes (150 kg) was added over an additional 20 minutes. The slurry was agitated for one hour at 0 -5 °C and then filtered. The filter cake was washed with a mixture of MTBE (16 kg) and heptanes (30 kg). The wet product was charged to a vacuum tray dryer and dried at 35°C (at less than 100 mbar), to give (R)-methyl 2-((S)-2,2-dimethyl-1,3-dioxolan-4-yl)-2-(tosyloxy)acetate (51.3 kg, 154 mol) as a light brown powder. Yield: 87%. ¹H NMR (400 MHz, CDCL₃) δ 7.83 (m, 2H), 7.35 (m, 2H), 4.84 (d, J= 4.8 Hz, 1H), 4.46 (m, 1H), 4.04 (dd, J= 6.6, 9.1 Hz, 1H), 3.97 (dd, J= 5.2, 9.1 Hz, 1H), 3.70 (s, 3H), 2.45 (s, 3H), 1.30 (s, 3H), 1.29 (s, 3H). iv) (S)-2,2-Dimethyl-4-((R)-oxiran-2-yl)-1,3-dioxolane: (R)-Methyl 2-((S)-2,2-dimethyl-1,3-dioxolan-4-yl)-2-(tosyloxy)acetate (76.1 kg, 221 mol) was dissolved in methanol (57 kg) and dichloromethane (208 kg). Methanol (14 kg), dichloromethane (53 kg) and one -third of the starting material solution (74 mol) were charged to the reactor. The solution was tempered to 10-15°C. Then, sodium borohydride (6.3 kg, 169 mol) was charged in 18 portions to the reactor holding the temperature 8 - 15°C. The mixture was stirred for half an hour after complete addition. The next one -third of the starting material solution (74mol), and more sodium borohydride (6.3 kg, 169 mol) were charged, followed by a half-hour stir, using the same procedure as before. This procedure was again repeated with the final one-third of the starting material solution (74 mol) and more sodium borohydride (6.3 kg, 169 mol). HPLC analysis then showed >99.9% conversion to the intermediate (5)--((5)-2,2-dimethyl-1,3-dioxolan-4-yl)-2-hydroxyethyl 4-methylbenzenesulfonate. Dichloromethane (200 kg) was charged to the reaction mixture. Sodium methoxide solution in methanol (43 kg, 30%, 239 mol) was dosed at 20-25°C for 60 minutes. After approximately half an hour, HPLC analysis showed 99.7% consumption of the intermediate alcohol. A solution of sodium acetate (25 kg) in water (230 L) was charged to the reaction mixture. The mixture was stirred for 10-15 minutes at 20-25°C. After separation for 15 minutes the lower organic phase was removed. The upper aqueous phase was extracted with dichloromethane (376 kg). The lower organic phase was removed, combining with the first organic phase, and the aqueous phase was discarded. Water (359 L) was charged to the combined organic phases. After stirring for 10-15 minutes at 20-25°C and settling for 15 minutes, the lower organic phase was transferred to a reactor containing sodium sulphate (63 kg). The volume of the mixture was reduced to 310 L by stripping off solvent, and then the sodium sulphate was filtered off. The filter cake was washed with dichloromethane (94 kg). The combined liquors were thoroughly mixed and then discharged to steel drums via a polypropylene bag filter to give (5)-2,2-dimethyl-4-((R)-oxiran-2-yl)-1,3-dioxolane solution in DCM (467.5 kg, 6.2%, 203 mol) as a clear yellow liquid. Yield: 91%. A sample, free from solvents, may be isolated on a small scale by evaporation of solvent and then distilling under vacuum. ¹H NMR (isolated sample, 400 MHz, d₆-DMSO) δ 4.01 (dd, J= 6.6, 8.2 Hz, 1H), 3.92 (m, 1H), 3.72 (dd, J= 5.8, 8.2 Hz, 1H), 3.03 (ddd, J=2.6, 4.1, 5.2 Hz, 1H), 2.77 (dd, J= 4.1, 5.0 Hz, 1H), 2.58 (dd, J= 2.6, 5.0 Hz, 1H), 1.34 (s, 3H), 1.27 (s, 3H). v) (R)-1-((S)-2,2-Dimethyl-1,3-dioxolan-4-yl)ethanol: From (5)-2,2-Dimethyl-4-((R)-oxiran-2-yl)-1,3-dioxolane solution in dichloromethane (465 kg, 6.2%, 200 mol), dichloromethane was distilled at 41-42°C and replaced by THF (129 kg). Distillation was continued at 60°C until a set volume in the reactor was reached (235 L). Lithium aluminium hydride (LAH) solution in THF (26.4 kg, 10%, 70 mol) was dosed to the reactor at 22°C and after subsequent stirring at 25°C for approximately one hour, GC analysis showed > 99.9 % consumption of the starting material. Small portions of water were added via a charging funnel at a rate which was adjusted to control temperature and foaming. A total of 2.6 litres of water (1 L per kg LAH) was added. Sodium hydroxide solution (2.6 kg, 15%, 1L per kg LAH) was added in the same manner as described for water. Water (7.9 L, 3L per kg LAH) was charged once more via the charging funnel using the same procedure as before. The slurry was filtered and the filter cake was washed with THF (36 kg). The filtrate was concentrated by stripping off THF, at a maximum temperature of 85°C, until condensation ceased. 2-MeTHF (129 kg) was charged to the reactor, and then solvent was distilled off to reach a solution volume of approximately 120 L. KF analysis showed <0.1% water. The solution was discharged via a cartridge filter to a PE-lined drum to give (R)- -((5)-2,2-dimethyl-1,3-dioxolan-4-yl)ethanol solution (103 kg, 27%, 187 mol) as a clear, light yellow liquid. Yield: 94%. A sample, free from solvents, may be isolated on a small scale by evaporation of solvent and then distilling under vacuum. 1H NMR (isolated sample, 400 MHz, d₆-DMSO) δ ppm 4.77 (d, J = 5.1 Hz, 1H), 3.95 (dd, J = 8.0, 6.2 Hz, 1H), 3.76 (dd, 8.0, 6.0 Hz, 1H), 3.70 (m, 1H), 3.46 (m, 1H), 1.29 (s, 3H), 1.25 (s, 3H), 1.07 (d, J = 6.2 Hz, 3H).

Alternatively Intermediate A [(R)-1-[(5)-2,2-dimethyl-1,3-dioxolan-4-yl]ethanol] may be prepares as follows:
Method A: 40g (1R)-1-[(4S)-2,2-dimethyl-1,3-dioxolan-4-yl]ethyl] 3,4,5 trimethoxybenzoate: (117.5mmol) was dissolved in 7 rel vol (280mL) methanol. To this solution was charged 20g 47%w/w aqueous sodium hydroxide (2 equivalents, 235 mmol) and the solution was heated to 50°C. The reaction went to completion typically within 1-2 hrs and was evaporated under reduced pressure. 2-Methyl-tetrahydrofurane (160mL, 4rel. vol) was charged and the mixture was evaporated again under reduced pressure to remove traces of methanol. Further 2-methyl-tetrahydrofurane (200mL, 5rel. vol) was charged and the resulting suspension was stirred at ambient temperature for 30 minutes before filtration. The filter cake was washed with 72mL (1.8 vol) 2-methyl tetrahydrofurane. The clear filtrate was concentrated again under reduced pressure to give the alcohol as colourless oil. Yield: 13.4g (78%)
Method B: 20g (1R)-1-[(4S)-2,2-dimethyl-1,3-dioxolan-4-yl]ethyl]-3,4,5-trimethoxybenzoate: (58.76mmol) was dissolved in 10 rel vol (200mL) 2-methyl tetrahydrofurane. To the clear solution was charged 4.24 mL methyl-tributylammonium chloride (75% aqueous solution, 1 1.75 mmol) followed by 9.89g (58.76mmol) of a 50% aqueous potassium hydroxide solution. The resulting mixture was agitated at 50°C and went to completion (<1% residual starting material) typically within 20hrs. The suspension was filtered and the clear filtrate was further dried by distillation under vacuum to approximately 1OOmL volume. The mixture was filtered, topped-up with 1OOmL 2-methyl tetrahydrofurane and concentrated again to 20mL total volume. The resulting clear solution was diuted with 30mL 2-methyl tetrahydrofurane that was previously used to rinse the vessel. Yield: 50mL solution in 2-methyl tetrahydrofurane containing 7.9g (92%th) (R)-1-[(S)-2,2-dimethyl-1,3-dioxolan-4-yl]ethanol. A sample, free from solvents, may be isolated on a small scale by evaporation of solvent and then distilling under vacuum. ¹H NMR (isolated sample, 400 MHz, d₆-DMSO) δ ppm 4.77 (d, J = 5.1 Hz, 1H), 3.95 (dd, J = 8.0, 6.2 Hz, 1H), 3.76 (dd, 8.0, 6.0 Hz, 1H), 3.70 (m, 1H), 3.46 (m, 1H), 1.29 (s, 3H), 1.25 (s, 3H), 1.07 (d, J = 6.2 Hz, 3H).

### (1R)-1-[(4S)-2,2-dimethyl-1,3-dioxolan-4-yl]ethyl]-3,4,5-trimethoxybenzoate was prepared as follows: [(1R,2S)-2,3,-dihydroxy-1-methyl-propyll 3-,4,5-trimethoxybenzoate

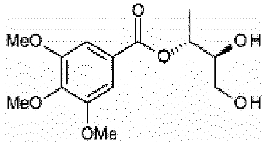

As the yield and enantiomeric excess of the product much depends on the level of residual water in solvents and reagents, the reaction requires the assistance of a drying agent, typically molecular sieves 3 Angstrom. The combined water content in solvents and reagents should not exceed 0.038 molar equivalents wrt E-crotyl alcohol. E-crotyl alcohol (20kg at 100% w/w, 277 mol) at approximately 20 °C was passed through a cartridge containing 3 Angstrom molecular sieve pellets (8 kg) at a constant rate over approximately 80 minutes into a dry collecting vessel. After a hold of approximately 32 minutes the cartridge was blown clear using pressurized nitrogen over approximately 31 minutes. Approximately 85% of the input E-crotyl alcohol was recovered as dried E-crotyl alcohol. A solution of dried E-crotyl alcohol (20 kg at 100%) w/w, 277 mol), L-(+)-diisopropyl tartrate (11.7 kg, 50 mol) and toluene (167 kg) at approximately -8 °C was charged with titanium isopropoxide (11.8 kg, 42 mol) and toluene (33 kg). The batch was agitated for approximately 30 minutes, before charging cumene hydroperoxide (87% w/w, 58.2 kg, 333 mol) and toluene (78 kg) over at least 4 hours. The batch was agitated for approximately 2 hours, before charging 3,4,5-trimethoxybenzoic acid (2.9 kg, 13.9 mol). The batch was then charged over approximately 1 hour to a slurry of titanium isopropoxide (7.9 kg, 28 mol), 3,4,5-trimethoxybenzoic acid (47.1 kg, 222 mol) and toluene (152 kg) at approximately 30 °C, followed by a line wash with toluene (17 kg). Titanium isopropoxide (23.7 kg, 83 mol) and toluene (40 kg) were then charged over approximately 2 hours, and the batch was then agitated for approximately 3 hours. The batch was cooled, and then charged with trimethylphosphite (17.2 kg, 139 mol) and toluene (35 kg). The batch was then warmed to approximately 30 °C, before washing twice with aqueous hydrochloric acid (10% w/w, 84 kg then 63 kg), and then three times with water (3 x 60 kg). The combined aqueous phases were then washed with 2-methyltetrahydrofuran (344 kg). The organic phases were combined and then washed with water (60 kg), before distilling under vacuum to a batch volume of approximately 260 ± 40 L. The temperature was adjusted to approximately 35 °C before hexanes (65 kg) were charged over at least 30 minutes. The batch was then seeded, and then agitated for at least 1 hour before cooling to approximately 0 °C over at least 3 hours. [Seeds may be obtained by removing a portion of the solution to a separate vessel and cooling this to or below the temperature of supersaturation. The precipitating solids can be filtered and returned directly to the main batch to function as seed crystals in a then controlled crystallisation.] Additional hexanes (325 kg) were charged over at least 2 hours and the slurry was aged for at least a further 1 hour before the solid was isolated by filtration. The solid was washed with hexanes (2 x 130 kg) and then dried to constant weight. Yield: 57% at 100% w/w. Strength = 93%) w/w [also contains 3,4,5-trimethoxybenzoic acid (TMBA)]. ¹H NMR [500 MHz, CDCl₃, with 2,3,5,6-tetrachloronitrobenzene (TCNB) as internal standard]; δ 7.71 (s, TCNB), 7.33 (s, residual TMBA), 7.26 (s, 2H), 7.24 (s, CDCl₃), 5.13 (m, 1H), 3.89 (m, 9H), 3.73 (m, 2H), 3.63 (1H, m), 1.44 (d, J= 6.6 Hz, 2H). Enantiomeric excess: typically 97%

### (1R)-1-[(4S)-2,2-dimethyl-1,3-dioxolan-4-yl]ethyl-3,4,5 trimethoxybenzoate:

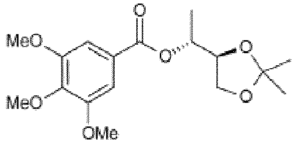

A solution of [(1R,2S)-2,3,-dihydroxy-1-methyl-propyl] 3,4,5-trimethoxybenzoate (43.8 kg at 100% w/w, 146 mol, approximately 97% enantiomeric excess) in 2-methyltetrahydrofuran (226 kg) at approximately 30 °C was charged with para-toluenesulfonic acid (0.6 kg, 3 mol). 2,2-Dimethoxypropane (38.0 kg, 365 mol) was then charged over approximately 40 minutes. The batch was agitated for approximately 2.5 hours before charging aqueous potassium bicarbonate solution (7% w/w, 167 kg). The temperature was adjusted to approximately 60 °C, before filtering the batch into a static vessel, to remove residual titanium species. The lower, aqueous, phase was removed and the batch was then washed with water (131 kg), before distilling under vacuum to a volume of approximately 130 ± 20 L. Isooctane (212 kg) was charged, and the solution was further distilled under vacuum to volume of approximately 240 ± 20 L. The batch was seeded at 60 °C and then agitated for at least 1 hour before cooling to approximately 5 °C over at least 8 hours. [Seeds may be obtained by removing a portion of the solution to a separate vessel and cooling this to or below the temperature of supersaturation. The precipitating solids can be filtered and returned directly to the main batch to function as seed crystals in a then controlled crystallisation.] The batch was aged for at least 2 hours before the solid was isolated by filtration. The solid was washed with hexanes (85 kg) at approximately -5 °C, and then dried to constant weight. Yield: 84% at 100% w/w. Strength = 100% w/w. ¹H NMR [400 MHz, CD<¾, with 2,3,5,6-tetrachloronitrobenzene (TCNB)]; δ 7.71 (s, TCNB), 7.28 (s, 2H), 7.24 (s, CDCl₃), 5.15 (m, 1H), 4.20 (m, 1H), 4.10 (m, 1H), 3.92 (m, 1H), 3.88 (s, 9H), 1.36 (m, 9H). Enantiomeric excess: typically > 99% ee (The isolation temperature maybe adjusted, depending on the enantioexcess of the starting [(1R,2S)-2,3,-dihydroxy-1-methyl-propyl] 3,4,5-trimethoxybenzoate, and the desired enantioexcess of the isolated (1R)-1-[(4S)-2,2-dimethyl-1,3-dioxolan-4-yl]ethyl-3,4,5 trimethoxybenzoate.)

### Intermediate B

### 3-Methylazetidine-1-sulfonamide

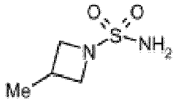

i) Benzyl 3-methylazetidin-1-ylsulfonylcarbamate: Isopropyl acetate (300 mL) was charged into a 1000 ml 3-neck flask and cooled to -10- 5°C. Sulfurisocyanatidic chloride (44.5 mL) was added at -5-10°C followed by the addition of phenylmethanol (50.5 mL) in isopropyl acetate (60 mL) at -5-10°C over 60 minutes. The mixture reacted at -5-10°C and was monitored by HPLC until the content of benzyl alcohol was <2% (after 1 hour at 0°C). A mixture of acetonitrile (300 mL), 3-methylazetidine hydrochloride (50 g) (Intermediate C, either commercially available or prepared as set out below) and triethylamine (162 mL) was stirred in a 2000 mL 3 -neck flask and to this was added the solution of the intermediate benzyl chlorosulfonylcarbamate in isopropyl acetate (360 mL) dropwise at <-5°C over 90 minutes. The mixture was allowed to warm to RT overnight. The reaction was quenched with acetic acid and pH was adjusted to 4∼5. The mixture was separated and the aqueous phase was washed with isopropyl acetate (500 ml) and then separated. The organic phase was combined and washed with 10% brine (2x120 ml), dried with anhydrous magnesium sulphate, filtered and the filter cake washed with isopropyl acetate (30 ml) and filtered to afford the sub-title product (145 g) as a red oil. ¹H NMR (500 MHz, CDCl₃) δ 11.43 (s, 1H), 7.57 (m, 5H), 5.18 (s, 2H), 4.08 - 3.96 (m, 2H), 3.60 - 3.50 (m, 2H), 2.67 - 2.54 (m, 1H), 1.22 - 1.11 (d, 3H). ii) 3-Methylazetidine-1-sulfonamide: The sub-title product of step (i) (132 g) split into 2 reactors was added to a stirred solution of 10% Pd/C (4.94 g) in ethanol (1000 mL). The mixture was hydrogenated at 3.00 bar for 16 hours. The solvent was evaporated to afford crude product. The crude product was purified by flash silica chromatography, elution 30% ethyl acetate in dichloromethane (stained with KMn04). Pure fractions were evaporated to dryness to afford the title product (60.3 g) as a white solid. ¹H NMR (500 MHz, CDCl₃) δ 6.82 (s, 2H), 3.75 (t, J = 8.0 Hz, 2H), 3.36 - 3.24 (m, 2H), 2.59 - 2.46 (m, 1H), 1.13 (d, J = 6.8 Hz, 3H).

### Intermediate C

### 3-Methylazetidine hydrochloride

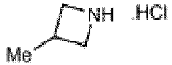

i) Benzyl 3-hydroxyazetidine-1-carboxylate: A solution of azetidin-3-ol (14.7 g) dissolved in THF (170 mL) and water (85 mL) was treated with potassium carbonate (37.1 g) under nitrogen. The mixture was stirred at RT for 30 minutes before cooling to 0 °C and adding benzyl carbonochloridate (20.0 mL) dropwise over 30 minutes at 0 °C. The resulting mixture was stirred at 20 °C for 60 hours. The reaction mixture was diluted with water (150 mL), and extracted with ethyl acetate (200 mL). The organic was dried over magnesium sulfate, filtered and evaporated to afford crude product as a colourless oil. The crude product was purified by flash silica chromatography, elution 50% ethyl acetate in isohexane to 100% ethyl acetate. Pure fractions were evaporated to dryness to afford the sub-title product (19.40 g, 69.8 %) as a colorless oil. ¹H NMR (500 MHz, CDCL₃) δ 7.41 - 7.28 (m, 5H), 5.09 (s, 2H), 4.70 - 4.54 (m, 1H), 4.23 (dd, J = 6.7, 9.9 Hz, 2H), 3.89 (dd, J = 4.4, 10.0 Hz, 2H), 2.34 (d, J = 6.1 Hz, 1H). ii) Benzyl 3-oxoazetidine-1-carboxylate: A solution of the sub-title product of step (i) (17.9 g) in DMSO (100 mL) was added drop wise over 15 minutes to a solution of pyridine sulphur trioxide (44.7 g) and triethylamine (39.3 mL) in DMSO (200 ml) at 0 °C (slight exotherm to 5C). The mixture was warmed to RT after 5 minutes and stirred for 16 hours. The mixture was poured into ice/water and extracted twice with ethyl acetate. The combined organics were washed with brine, dried over sodium sulfate and concentrated in vacuo to give crude product. The crude product was purified by flash silica chromatography, elution 100% dichloromethane. Pure fractions were evaporated to dryness to afford the sub-title product (17.9 g) as a pale yellow oil. ¹H NMR (500 MHz, CDCL₃) δ 7.40 - 7.31 (m, 5H), 5.17 (s, 2H), 4.78 (s, 4H). iii) Benzyl 3-methyleneazetidine-1-carboxylate: A suspension of methyltriphenylphosphonium bromide (93 g) and potassium tert-butoxide (29.3 g) in diethyl ether (700 mL) was stirred at RT for 20 minutes and heated at 35 °C for 1 hour under nitrogen. The bright yellow mixture was treated with the sub-title product of step (ii) (17.9 g) in diethyl ether (200 mL) dropwise over 1 hour at 35 °C (orange suspension formed). The resulting mixture was stirred at 35 °C for 12 hours. The mixture was cooled and filtered through a pad of celite and washed with diethyl ether. The filtrate was washed with water (300 mL), dried over magnesium sulfate, filtered and evaporated to afford crude product. The crude product was purified by flash silica chromatography, elution 10% ethyl acetate in isohexane to 50% ethyl acetate in isohexane (stained with KMn04). Pure fractions were evaporated to dryness to afford the sub -title product (14.1) as a colorless oil. ¹H NMR (400 MHz, CDCL₃) δ 7.39 - 7.28 (m, 5H), 5.12 (s, 2H), 5.01 (m, 2H), 4.57 (t, 4H). iv) 3-Methylazetidine hydrochloride: A solution of the sub-title product of step (iii) (14.1 g) dissolved in ethanol (100 mL) was treated with 10% Pd/C (JM type 87L) (1.48 g) under hydrogen. The resulting mixture was stirred at 20 °C for 40 hours at 4.50 bar pressure of hydrogen gas. The Cbz protecting group still attached so switched to palladium hydroxide on carbon (2 g) in ethanol (100 mL). The mixture was hydrogenated for a further 24 hours at 4.50 bar. The mixture was filtered through a pad of celite and filtrate cooled to 0 °C in an ice batch. 4M HCl in dioxane (26.0 mL) was added dropwise and the solution evaporated to dryness to give the title product (7.46 g) as a light brown oil. ¹H NMR (400 MHz, CDCL₃) δ 9.24 (s, 2H), 3.95 (ddd, J = 7.4, 9.8, 11.4 Hz, 2H), 3.55 - 3.45 (m, 2H), 2.85 (dt, J = 6.7, 14.2 Hz, 1H), 1.16 (d, 3H).

Preferred embodiments of WO 2013/008002 and the invention are as follows: The present invention provides the compound which is (a) a pyrimidine sulphonamide of formula (I) or (b) a pharmaceutically acceptable salt thereof.

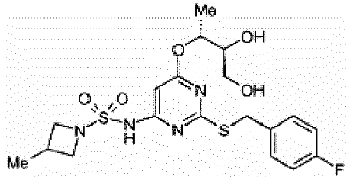

A representative compound of the invention demonstrates an unexpectedly long half-life in dog. A long half-life in pre-clinical species (such as the dog) suggests that a long half-life in human is attainable (Obach et al. (1997), J. Pharmacol. Exp. Ther., 283: 46-58). A half life in human in excess of 12 hours is commensurate with once a day dosing. In addition, in order to antagonize the target receptor in humans and therefore produce the desired biological effect, a compound should be present in the plasma in sufficient concentration to inhibit the receptor function, and this concentration must be maintained for a sufficient period to continue receptor inhibition between dosing intervals. Thus a compound should exhibit a combination of both high potency and long half life. A representative compound of the invention demonstrates the combination of high potency and long measured half life in dog. The minimum concentration needed to drive required effect is the Cmin and the maximum concentration reached in the plasma to maintain Cmin at the end of the dosing period (e.g. 24h for once-a-day) is the Cmax. Hence a small Cmax/Cmin ratio (driven by a long half-life) is beneficial, because high Cmax levels are more likely to cause unwanted effects. The compounds of invention are predicted to have a small Cmax/Cmin ratio. In a further aspect the compound of the invention are compound of formula (I) not in salt-form. Within the present invention it is to be understood that the compounds of the invention may exhibit the phenomenon of tautomerism and that the formulae within this specification can represent only one of the possible tautomeric forms. It is to be understood that the invention encompasses any tautomeric form and mixtures thereof and is not to be limited merely to any one tautomeric form utilised within the formulae. A pharmaceutically acceptable salt of compounds of the invention may include a salt prepared from a pharmaceutically acceptable non-toxic base, such as an inorganic or organic base. A salt derived from an inorganic base may be, for example, an aluminium, calcium, potassium, magnesium, sodium or zinc salt. A salt derived from an organic base may be, for example, a salt of a primary, secondary or tertiary amine. A pharmaceutically acceptable salt of compounds of the invention may be prepared in situ during the final isolation and purification of a compound, or by separately reacting the compound with a suitable base and isolating the salt thus formed. The compounds of the invention may exist as a solvate (such as a hydrate) and the present invention covers all such solvates. The compounds of the invention may exist as an in-vivo hydrolysable ester of the compound of formula (I). An in-vivo hydrolysable ester of a compound of the invention containing a hydroxy group is, for example, a pharmaceutically-acceptable ester which is hydrolysed in the human or animal body to produce the parent alcohol. An in-vivo hydrolysable ester of a compound of the invention containing a hydroxy group includes inorganic esters such as phosphate esters (including phosphoramidic cyclic esters) and a-acyloxyalkyl ethers and related compounds which as a result of the in-vivo hydrolysis of the ester breakdown to give the parent hydroxy group/s. Examples of a-acyloxyalkyl ethers include acetoxymethoxy and 2,2-dimethylpropionyloxy-methoxy. A selection of in-vivo hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. The compounds of the invention may exist in crystalline form. Thus, according to a further aspect of the invention, there is provided a substantially crystalline form of the compound of formula (I), or pharmaceutically acceptable salts thereof. When herein reference is made to compounds of the invention being crystalline, suitably the degree of crystallinity as determined by X-ray powder diffraction data is for example greater than about 60%, such as greater than about 80%, particularly greater than about 90%, more particularly greater than about 95%. In embodiments of the invention, the degree of crystallinity as determined by X-ray powder diffraction data is greater than about 98%, wherein the % crystallinity refers to the % by weight of the total sample mass which is crystalline.

It is stated hereinbefore that the compound of formula (I) may be produced in a crystalline form that is an anhydrate. By this we mean that the crystalline form contains less than 10% of hydrate form(s) (e.g. a monohydrate) of the compound of formula (I). According to a further aspect of the invention, there is provided a substantially crystalline anhydrate form of the compound of formula (I). In a still further aspect, the compound of formula (I) is not in the form of a salt. In a yet still further aspect, the compound of formula (I) is not in the form of a solvate, i.e. it is an "ansolvate". Hence, the term "anhydrate" encompasses "ansolvate". According to a further aspect of the invention, there is provided an anhydrate crystalline form of the compound of formula (I) which may be characterised by a differential scanning calorimetry curve, at a heating rate of 10°C per minute in a closed aluminium cup under a nitrogen atmosphere, exhibiting the following onset temperature of the melting endotherm of about 176°C. According to yet a further aspect of the invention, there is provided a crystalline form of the compound of formula (I) which may be characterised by an X-ray powder diffraction pattern, measured using a wavelength of X-rays 1.5418 A, comprising the following characteristic peaks with approximate 2-Theta values (in degrees). Crystalline Form A of N-(6-((2R,3S -3,4-dihydroxybutan-2-yloxy -2-(4-fluorobenzylthio)pyrimidin-4-yl)-3-methylazetidine-1 -sulfonamide (hereinafter 'Form A'): a characteristic differential scanning calorimetry curve, at a heating rate of 10°C per minute in a closed aluminium cup under a nitrogen atmosphere, exhibiting an onset temperature of the melting endotherm of about 176°C. In a further aspect Form A has an X-ray powder diffraction pattern, measured using a wavelength of X-rays 1.5418 A, with characteristic peaks at 2-Theta (in degrees) of 8.5, 9.7, 10.6, 17.1, 19.9, and 21.2. In a further aspect, Form A has an X-ray powder diffraction pattern, measured using a wavelength of X-rays 1.5418 A, with characteristic peaks at 2-Theta (in degrees) of 8.5, 9.7, 10.6, 17.1, 19.9, and 21.2. In another aspect, Form A has an X-ray powder diffraction pattern, measured using a wavelength of X-rays 1.5418 A, with at least three characteristic peaks at 2-Theta (in degrees) selected from 8.5, 9.7, 10.6, 17.1, 19.9, and 21.2. In a further aspect, Form A has an X-ray powder diffraction pattern, measured using a wavelength of X-rays 1.5418 A, with at least two characteristic peaks at 2-Theta (in degrees) selected from 8.5, 9.7, 10.6, 17.1, 19.9, and 21.2. In a still further aspect, Form A has an X-ray powder diffraction pattern, measured using a wavelength of X-rays 1.5418 A, with at least one characteristic peak at 2-Theta (in degrees) selected from 8.5, 9.7, 10.6, 17.1, 19.9, and 21.2. In another aspect, Form A comprises the characteristic X-ray powder diffraction pattern peaks, measured using a wavelength of X-rays 1.5418 A, as shown in Figure 1 of WO 2013/008002. In another aspect, Form A comprises the characteristic differential calorimetry curve substantially as shown in Figure 2 of WO 2013/008002. Suitably a crystalline modification of a compound according to the invention is substantially free from other crystalline modifications of the compound. Suitably, a described crystalline modification of a compound of formula (I) includes less than, for example, 20%, 15%, 10%), 5%‰, 3‰ or particularly, less than 1% by weight of other crystalline forms of that compound. Crystalline anhydrates of the compound of formula (I) may be prepared as described herein by crystallizing the compound of formula (I) from one or more suitable solvents or mixtures thereof. Anhydrate may be produced by crystallization from a solvent system which is substantially free of water (which may have been dried, and/or may be dried during the crystallization process). Solvent may be dried during the crystallization process, for example by decreasing the water content of a mixture of the compound to be crystallized in a suitable organic solvent / aqueous solvent system (e.g. by increasing the amount of organic solvent that is present and/or removal of water by formation of an azeoptrope, with successive distillations). However, crystalline anhydrates of the compound of formula (I) may also be prepared from water and/or water/alcohol mixtures. Compounds of the invention that are anhydrates typically contain no more than 2%, particularly 1%, more particularly 0.5% and more particularly 0.2% (w/w) water, whether such water is bound (crystal water or otherwise) or not. In order to ensure that crystalline forms as described herein are prepared in the absence of other crystalline forms, crystallisations may be carried out by seeding with nuclei and/or seed crystals of the desired crystalline form in the absence of nuclei and/or seed crystals of other crystalline forms. The skilled person will appreciate that the concentration in solution of the compound that is to be crystallised, and the solvent system that is used, may influence crystallisation temperatures and crystallisation times. Different crystalline forms may have different solubility in different organic solvents at any given temperature. In this respect, above-mentioned, or other, solvents may be employed as "antisolvents" (i.e. a solvent in which compounds of the invention are poorly soluble, but which is miscible with another solvent, in which compounds of the invention are more soluble), and may thus aid the crystallisation process. As may be appreciated by the skilled person, the crystalline form that is obtained depends upon both the kinetics and the thermodynamics of the crystallisation process. Under certain thermodynamic conditions (solvent system, temperature, pressure and concentration of the compound of the invention), one crystalline form may be more stable than another (or indeed any other). However, other crystalline forms that may have, in comparison, a relatively low thermodynamic stability, may be kinetically- favoured. Thus, in addition, kinetic factors, such as time, impurity profile, agitation, the presence of seeds, etc. may also influence which forms appear. Thus, the procedures discussed herein may be adapted by the skilled person as appropriate in order to obtain the particular crystalline form of the compound of formula (I). Compound of the invention may be dried using standard techniques. It will be appreciated by the skilled person that drying temperature and drying time may affect the solid state properties and/or the solid state form of compounds of the invention. For example, dehydration may occur at low humidity and/or elevated temperatures and/or reduced pressure. Hence, the crystalline anhydrates of compound of the invention may also be formed by dehydration of a hydrate. The preparation and characterisation of compounds of the invention may be prepared as described below, by adapting methods known in the art, or by using or adapting the preparative methods described in the Examples. Different crystalline forms of the compounds of the invention may be readily characterised using X-ray powder diffraction (XRPD) methods, for example as described hereinafter. Standard DSC techniques may also be used. Compounds of the invention may be prepared by following the process presented in Scheme 1 below and in the Examples described herein.

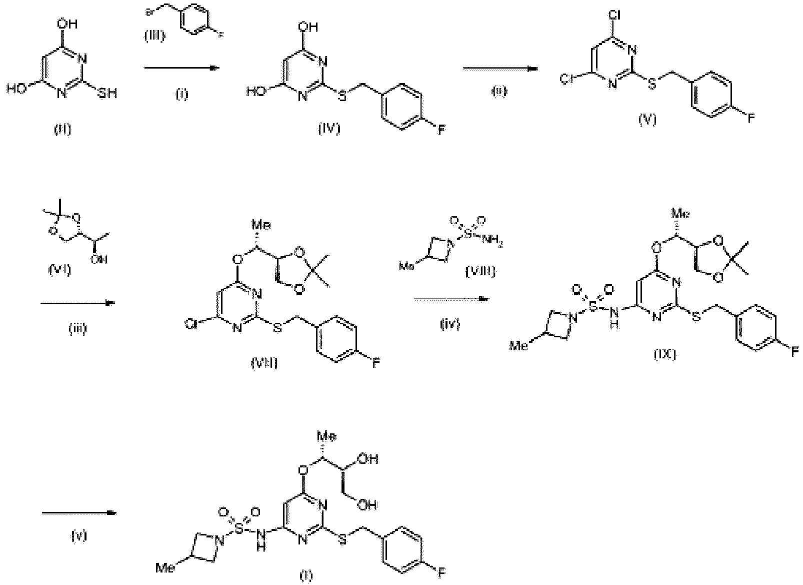

Scheme 1: (i) Sodium acetate, water, acetonitrile; (ii) Phosphorus oxychloride, benzyltriethylammonium chloride, dimethoxyethane; (iii) Sodium hydride, tetrahydrofuran; (iv) Cesium carbonate, dicyclohexyl(2',4',6'-triisopropylbiphenyl-2-yl)phosphine, tris(dibenzylideneacetone)dipalladium(0), dioxane; (v) Trifluoroacetic acid, dichloromethane.

The compounds of formula (II) and (III) are commercially available or may be synthesised using methods familiar to those skilled in the art.

The invention relates to an administration unit comprising crystalline form or pharmaceutical acceptable salt of N-(6-((2R,3S)-3,4-dihydroxybutan-2-yloxy)-2-(4-fluorobenzylthio)pyrimidin-4-yl)-3-methylazetidine-1-sulfonamide. The administration unit according to the invention is useful for treating various diseases and disorders which include but are not limited to of a chemokine mediated disease state, asthma, allergic rhinitis, chronic obstructive pulmonary disease, inflammatory bowel disease, irritable bowel syndrome, osteoarthritis, osteoporosis, rheumatoid arthritis or psoriasis.

### SPECIFIC INORMATION CONCERNING ASPECT (VIII) OF THE INVENTION

Aspect (viii) of the invention concerns forms of {drug8}, especially a solid form of 6-(Piperidin-4-yloxy)-2H-isoquinolin-1-one hydrochloride, namely to crystalline form of 6-(Piperidin-4-yloxy)-2H-isoquinolin-1-one hydrochloride selected from the group consisting of its hydrate, dihydrate, polymorph 1, polymorph 2, polymorph 3, polymorph 4, 1,4-dioxane solvate, acetonitrile solvate and methyl acetate solvate, which is useful for treating diseases associated with Rho-kinase and/or Rho-kinase mediated phosphorylation of myosin light chain phosphatase, in particular for the treatment and/or prevention of hypertension, pumonary hypertension, ocular hypertension, retinopathy, glaucoma, peripheral circuatory disorder, peripheral occlusive arterial disease (PAOD), coronary heart disease, angina pectoris, heart hypertrophy, heart failure, ischemic diseases, end organ damage inc!. ischemic organ failure, fibroid lung, fibroid liver, liver failure, nephropathy (including hypertension- induced, non-hypertension-induced, and diabetic nephropathies), renal failure, fibroid kidney, renal glomerulosclerosis, organ hypertrophy, asthma, chronic obstructive pulmonary disease (COPD), adult respiratory distress syndrome, thrombotic disorders, stroke, cerebral vasospasm, cerebral ischemia, pain, e.g. neuropathic pain; neuronal degeneration, spinal cord injury, Alzheimer's disease, premature birth, erectile dysfunction, endocrine dysfunctions, arteriosclerosis, prostatic hypertrophy, diabetes and complications of diabetes, metabolic syndrome, blood vessel restenosis, atherosclerosis, inflammation, autoimmune diseases, osteopathy such as osteoporosis, infection of digestive tracts with bacteria, sepsis, cancer development and progression, e.g. cancers of the breast, colon, prostate, ovaries, brain and lung and their metastases. The invention relates to crystalline solvates of 6-(Piperidin-4-yloxy)-2H- isoquinolin-1-one hydrochloride, which has the structure according to formula (I), 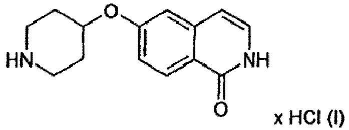 processes for their preparation and their use, in particular for the preparation of medicaments. 6-(Piperidin-4-yloxy)-2H-isoquinolin-1-one is known as a pharmaceutically active compound. 6-(Piperidin-4-yloxy)-2H-isoquinolin-1 -one is described as free base in WO 2007/065916. WO 2007/012421, WO 2008/077550 and WO 2009/080335 describe the synthesis of 6-(Piperidin-4-yloxy)-2H-isoquinolin-1-one and its hydrochloride but do not contain any evidence of a controlled, reproducible crystallization procedure. The described material is ony obtained by lyophilization. A crystalline form of 6-(Piperidin-4-yloxy)-2H-isoquinolin-1-one hydrochloride selected from the group consisting of its hydrate, dihydrate, polymorph 1, polymorph 2, polymorph 3, polymorph 4, 1,4-dioxane solvate, acetonitrile solvate and methyl acetate solvate is described in PCT/EP2012/062431, published as WO 2013/007518, which is incorporated by reference.

As used herein, crystalline form of 6-(Piperidin-4-yloxy)-2H-isoquinolin-1-one hydrochloride selected from the group consisting of its hydrate, dihydrate, polymorph 1, polymorph 2, polymorph 3, polymorph 4, 1,4-dioxane solvate, acetonitrile solvate and methyl acetate solvate is a crystalline form of 6-(Piperidin-4-yloxy)-2H-isoquinolin-1-one hydrochloride characterized as follows:

### Hydrate and Dihydrate

One embodiment the present invention is a crystalline hydrate of 6-(Piperidin-4-yloxy)-2H-isoquinolin-1-one hydrochloride (I). Another embodiment the present invention is a crystalline hydrate of compound (I) wherein the hydrate contains about 10.5 - 12.5 % water (w/w). Another embodiment of the present invention is a hydrate wherein the hydrate contains 1.85 - 2.2 molecules water per molecule 6-(Piperidin-4-yloxy)-2H- isoquinolin-1-one hydrochloride (I). Another embodiment of the present invention is a hydrate wherein the hydrate contains 2 molecules water per molecule 6-(Piperidin-4-yloxy)-2H-isoquinolin-1-one hydrochloride (I). A crystalline hydrate containng 1.85 - 2.2 molecules water per molecule is herein referred to as "dihydrate" and is an embodiment of the present invention. In another embodiment of the hydrate the water content is 10.5 - 11.4 % water (w/w). In a further embodiment the hydrate contains about 1.85 - 2.0 molecules water. In a further embodiment of the hydrate the water content is 1 1.4 % (w/w). In a further embodiment the hydrate contains 2.0 molecules water per molecule (I). Although the hydrate phase typically contains about 10 - 12.5% water it can also occur with lower water content. The crystal structure of the hydrate remains even if the dihydrate is dried and the remainiig water content is down to about 3 %. Water uptake is reversible if humidity in the environment is raised again. The water content in the isolated product depends on the drying conditions used during work up of the hydrate after crystallisation. In one embodiment the dihydrate has the property of having at least a characteristic reflection in an Xray powder diffractogram using CuKa radiation at 7.7 ± 0.2 degrees 2theta. In another embodiment the dihydrate has the property of having at least characteristic reflections in an X-ray powder diffractogram using CuKa radiation at 7.7 (strong), 15.2 (strong) and 16.8 (medium) degrees 2theta ± 0.2 degrees 2theta. In another embodiment the dihydrate has the property of having at least characteristic reflections in an X-ray powder diffractogram using CuKa radiation at 7.7 (strong), 15.2 (strong), 16.8 (medium), 22.4 (strong), 25.0 (strong) and 26.6 (strong) degrees 2theta ± 0.2 degrees 2theta. In another embodiment the dihydrate has the property of having at least characteristic reflections in an X-ray powder diffractogram using CuKa radiation at 7.7 (strong), 15.2 (strong), 16.8 (medium), 18.4 (medium), 20.4 (medium), 22.4 (strong), 25.0 (strong), 26.6 (strong) and 30.3 (mediun) degrees 2theta ± 0.2 degrees 2theta.

### Polymorph 1

Another aspect of the present invention relates to polymorph 1 of 6-(Piperidin-4-yloxy)- 2H-isoquinolin-1-one hydrochloride which has at least characteristic reflections in an X-ray powder diffractogram (XRPD) using CuKc radiation at 4.5 (medium), 15.4 (strong), 16.8 (strong), 21.7 (medium) and 24.7 (medium) degrees 2theta ± 0.2 degrees 2theta. In another aspect polymorph 1 has the property of having at least characteristic reflections in an X-ray powder diffractogram using CuKa radiation at 4.5 (medium), 15.4 (strong), 16.8 (strong), 19.8 (weak), 21.7 (medium), 22.5 (strong), 22.8 (Strong), 24.7 (medium) and 27.3 (medium) degrees 2theta ± 0.2 degrees 2theta.

### Polymorph 2

Another aspect of the present invention relates to polymorph 2 of compound (I) which has the property of having at least characteristic reflections in an X-ray powder diffractogram using CuKa radiation at 8.1 (medium), 15.8 (strong) and 16.5 (medium) degrees 2theta ± 0.2 degrees 2theta. In another aspect the polymorph has the property of having at least characteristic reflections in an X-ray powder diffractogram using CuKa radiation at 8.1 (Medium), 15.8 (Strong), 16.5 (Medium), 17.7 (Medium), 19.6 (Medium), 20.8 (Medium), 22.2 (medium), 25.0 (Medium), 26.6 (Medium) and 30.5 (Medium) degrees 2theta ± 0.2 degrees 2theta. In a further aspect the invention relates to polymorphs 1, 3 and 4 of compound (I), which unlike polymorph 2, can be directly obtained from compound (I) and which then can also be converted into the dihydrate. In one aspect the three anhydrous polymorphs are characterized by exhibiting in an X- ray powder diffractogram using CuKa radiation at least characteristic reflections at 1) 15.4 degrees 2theta and within each of the ranges selected from 2) 16.6 -16.8 and 3) 21.5 - 2.7 degrees 2theta ± 0.2 degrees 2theta.

### Polymorph 3

Another aspect of the present invention relates to polymorph 3 of 6-(Piperidtn-4-yloxy)-2H-isoquinolin-1-one hydrochloride which has the property of having at least characteristic reflections in an X-ray powder diffractogram using CuKoi radiation at 4.5 (medium), 15.4 (strong), 16.7 (strong), 21.7 (strong) and 25.5 (medium) degrees 2theta ± 0.2 degrees 2theta. In another aspect polymorph 3 has the property of having characteristic reflections in an X-ray powder diffractogram using CuKoi radiation at 4.5 (medium), 15.4 (strong), 16.7 (strong), 21.7 (strong), 22.0 (medium), 22.3 (medium) and 25.5 (medium) degrees 2theta ± 0.2 degrees 2theta.

### Polymorph 4

The invention further relates to polymorph 4 of 6-(Piperidin-4-yloxy)-2H-isoquinolin-1-one hydrochloride which has the property of having at least characteristic reflections in an X-ray powder diffractogram using CuKcti radiation at 15.4 (medium), 16.7 (medium), 21.5 (strong) and 30.7 (weak) degrees 2theta ± 0.2 degrees 2theta. In another aspect polymorph 4 has the property of having at least characteristic reflections in an X-ray powder diffractogram using CuKcti radiation at 15.4 (medium), 16.7 (medium), 16.9 (medium), 21.5 (strong), 21.9 (weak), 22.4 (medium), 23.2 (weak), 27.6 (weak) and 30.7 (weak) degrees 2theta ± 0.2 degrees 2theta.

### Solvates

Moreover, the present invention relates to a 1,4-dioxane solvate, a methyl acetate solvate, and an acetonitrile solvate of 6-(Piperidin-4-yloxy)-2H-isoquinolin-1 -one hydrochloride.

### 1,4-dioxane solvate

The 1,4-dioxane solvate according to the invention shows characteristic reflections in an X-ray powder diffractogram using CuKa radiation at 5.1 (strong) and 22.5 (strong) degrees 2theta ± 0.2 degrees 2theta. In another aspect the 1,4-dioxane solvate has the property of having at least characteristic reflections in an X-ray powder diffractogram using CuKa radiation at 15.1 (strong), 19.7 (medium), 20.3 (medium), 21,6 (medium), 22.5 (strong), 23.8 (medium, 24.9 (medium), and 30.2 (medium) degrees 2theta ± 0.2 degrees 2theta.

### Acetonitrile solvate

The acetonitrile solvate is another object of the present invention. This solvate shows at least characteristic reflections in an X-ray powder diffractogram using CuKa radiation at 6.8 (medium), 11.3 (medium) and 27.7 (strong) degrees 2theta ± 0.2 degrees 2theta. In another aspect the acetonitrile solvate has the property of having at least characteristic reflections in an X-ray powder diffractogram using CuKa radiation at 6.8 (medium), 11.3 (medium), 15.3 (strong), 20.9 (medium), 23.9 (strong), 24.0 (medium), 27.4 (medium), and 27.7 (strong) degrees 2theta ± 0.2 degrees 2theta.

### Methyl acetate solvate

The methyl acetate solvate according to the invention shows at least characteristic reflections in an X-ray powder diffractogram measured in suspension (capillary) using CuKa radiation in transmission mode at 15.0 (strong) and 23.7 (strong) degrees 2theta ± 0.2 degrees 2theta. In another aspect the methyl acetate solvate has the property of having at least characteristic reflections in an X-ray powder diffractogram using CuKa radiation at 6.9 (medium), 15.0 (strong), 20.8 (medium), 22.8 (medium), 23.7 (strong), 24.0 (medium), 25.1 (medium), and 28.0 (medium) degrees 2theta ± 0.2 degrees 2theta.

A crystalline form of 6-(Piperidin-4-yloxy)-2H-isoquinolin-1-one hydrochloride selected from the group consisting of its hydrate, dihydrate, polymorph 1, polymorph 2, polymorph 3, polymorph 4, 1,4-dioxane solvate, acetonitrile solvate and methyl acetate solvate can be obtained by the following procedures:
Compound (I) as starting material for making the polymorphs and solvates can be obtained as described in WO 2007/012421. Where the dihydrate is used or obtained, this is specified. 6-(Piperidin-4-yloxy)-2H-isoquinolin-1-one hydrochloride is abbreviated as "compound (I)". If not mentioned otherwise drying is carried out over night at reduced pressure (about <50 mbar) at 40°C.

### Formation of dihydrate,

a) 10 g crude 6-(Piperidin-4-yloxy)-2H-isoquinolin-1-one hydrochloride are dissolved in 25 mL water at 70C. The solution is cooled to 55°C and 75 mL acetone are added. The mixture is cooled to room temperature within 3 hours and left standing for two days for crystallization. After cooling (4°C) for 6 hours the product is isolated via filtration, washed with acetone/water (3:1) and dried in vacuum. 7.9 g (purity 97, 1 %)of 6-(Piperidin-4-yloxy)-2H-isoquinolin-1-one hydrochloride dihydrate are obtained. b) 8.5 g of crude 6-(Piperidin-4-yloxy)-2H-isoquinolin-1-one hydrochloride are dissolved in 21.5 mL water at 65°C. The temperature is lowered to 50°C in 1 h and 32.3 mL acetone are added in 30 min. The temperature is lowered to 40 °C and the mixture is stirred for 3 h. The reaction mixture is chilled to ambient temperature. The crystalline material is collected, washed with water/acetone (1/3) and dried to yield 4.54 g (purity > 99.9%) of 6-(Piperidin-4-yloxy)-2H-isoquinolin-1-one hydrochloride dihydrate. c) 0.205 g compound (I) (dihydrate) are dissolved in 20 mL ethanol and 3 mL water at about 65 °C. The solvent is allowed to evaporate from the stirred solution at the same temperature over night. d) 0.200 g of compound (I) (dihydrate) are dissolved in 20 mL ethanol and 4 mL water at 65°C. The solution is rapidly cooled to O. After 45 minutes the product is isolated by vacuum filtration and dried. In the same manner the dihydrate is obtained if ethanol is replaced by tetrahydrofuran or methyl ethyl ketone in examples c) and d). e) 0.204 g of compound (I) (dihydrate) are dissolved in 3mL water at 65°C. The solution is rapidly cooled to OX. After 30 minutes the product is isolated by vacuum filtration and dried.
Formation of polymorph 1: 1.2 g of 6-(Piperidin-4-yloxy)-2H-isoquinolin-1-one hydrochloride are suspended in isopropanol and stirred for 6h. The solid material (1.14 g) is isolated by filtration. 60.7 mg thereof are suspended in a mixture of 0.352 mL isopropanol and 0.647 mL methanoi. The mixture is heated until a clear solution is obtained. Upon cooling the crystalline product is obtained which is isolated by filtration.
Formation of polymorph 2: About 3 mg of compound (I) (dihydrate) are at 25°C exposed to a dry nitrogen atmosphere (stream of nitrogen) for at least 6 hours. After this treatment, the X-ray diffraction pattern of the sample corresponds to phase 2.
Formation of polymorph 3: a) 0.201 g of compound (I) (dihydrate) are dissolved in 20mL acetonitril and 3 mL water at 65 °C. The solvent is allowed to evaporate from the stirred solution at the same temperature overnight. Polymorph 3 and traces of the dihydrate are obtained. b) 0.208 g of compound (I) (dihydrate) are dissolved in 10 mL methanol at 65 °C. The stirred solution is rapidly cooled to 0°C. After 30 minutes the product is isolated by vacuum filtration and dried. c) 0.203 g of compound (I) (dihydrate) are suspended in 1.2 mL methanol at 20°C and stirred for 35 days. The product is isolated by vacuum filtration and dried. The same product (polymorph 3) is obtained, if the dihydrate of compound (I) is suspended in ethanol, 1 -propanol or 2-propanol.
Formation of polymorph 4: 0.202 g of compound (I) (dihydrate) are suspended in 2.0 mL 2-butanol at 20°C and stirred for 35 days. The product is isolated by vacuum filtration and dried.
Formation of methyl acetate solvate: 0.208 g of compound (I) (dihydrate) are suspended in 2,5 mL of methyl acetate. The solution is stirred in a closed vessel at room temperature for 35 days. The solid present in the suspension is the methyl acetate solvate as determined by XRPD in suspension. After vacuum filtration and drying the dihydrate containing forms 1 and 3 is obtained.
Formation of 1,4 dioxane solvate: 0.204 g of compound (I) (dihydrate) are dissolved in 2.5 mL of 1,4-dioxane at 20 °C for 35 days with continuous stirring. The solid present in the suspension is the methyl acetate solvate as determined by XRPD in suspension. After vacuum filtration and drying the solvate containing dihydrate are obtained.
Formation of acetonitrile solvate: 0.206 g of compound (I) (dihydrate) are suspended in 2.5 mL of acetonitrile. The suspension is stirred at 20 °C for 35 days. The solid present in the suspension is the acetonitrile solvate as determined by XRPD in suspension. The solid present in the suspension is isolated via vacuum filtration and dried overnight at reduced pressure at room temperature. After vacuum filtration and drying the dihydrate containing forms 1 and 3 is obtained.

In the context of the present invention, polymorph, polymorphic form, solvate etc. always refers to a polymorph, polymorphic form or solvate of 6-(Piperidin-4-yloxy)-2H∼ isoquinolin-1 -one hydrochloride. The terms "polymorph", "form" and "phase" may be used interchangeably herein. The anhydrous and solvent free forms as well as the hydrates and organic solvates of the present invention were obtained as outlined in the Examples provided below. The invention relates to an administration unit comprising crystalline form of 6-(Piperidin-4-yloxy)-2H-isoquinolin-1-one hydrochloride selected from the group consisting of its hydrate, dihydrate, polymorph 1, polymorph 2, polymorph 3, polymorph 4, 1,4-dioxane solvate, acetonitrile solvate and methyl acetate solvate. A hydrate is a solvate containing water. According to the invention the term of a hydrate of compound (i) includes all aqueous solvates of compound (I) where water is present in any ratio to compound (I). However, as acknowledged by the person skilled in the art, the presence of new solid solvates of a known chemical compound cannot be foreseen. The existence of crystalline phases (hydrates or solvates) cannot be foreseen. Also the conditions under which crystallization takes place to give a specific form, and the characteristics of the polymorphic forms and solvates cannot be predicted. Since properties such as the solubility and stability and consequently the suitability for use and storage of each polymorph and solvate may vary, identifying the existence of polymorphs is essential for providing pharmaceuticals with increased storage stability or predictable solubility profiles.

The administration unit according to the invention is useful for treating various diseases and disorders which include but are not limited to diseases associated with Rho-kinase and/or Rho-kinase mediated phosphorylation of myosin light chain phosphatase, in particular for the treatment and/or prevention of hypertension, pulmonary hypertension, ocular hypertension, retinopathy, glaucoma, peripheral circulatory disorder, peripheral occlusive arterial disease (PAOD), coronary heart disease, angina pectoris, heart hypertrophy, heart failure, ischemic diseases, end organ damage inc!. ischemic organ failure, fibroid lung, fibroid liver, liver failure, nephropathy (including hypertension- induced, non-hypertension-induced, and diabetic nephropathies), renal failure, fibroid kidney, renal glomerulosclerosis, organ hypertrophy, asthma, chronic obstructive pulmonary disease (COPD), adult respiratory distress syndrome, thrombotic disorders, stroke, cerebral vasospasm, cerebral ischemia, pain, e.g. neuropathic pain; neuronal degeneration, spinal cord injury, Alzheimer's disease, premature birth, erectile dysfunction, endocrine dysfunctions, arteriosclerosis, prostatic hypertrophy, diabetes and complications of diabetes, metabolic syndrome, blood vessel restenosis, atherosclerosis, inflammation, autoimmune diseases, osteopathy such as osteoporosis, infection of digestive tracts with bacteria, sepsis, cancer development and progression, e.g. cancers of the breast, colon, prostate, ovaries, brain and lung and their metastases.

### SPECIFIC INORMATION CONCERNING ASPECT (IX) OF THE INVENTION

Aspect (ix) of the invention concerns forms of {drug9}, especially a crystalline polymorph of 6-(Piperidin-4-yloxy)-2H-isoquinolin-1-one hydrochloride wherein the polymorph exhibits in an X-ray powder diffractogram using CuKa radiation at least a characteristic reflection within the two ranges selected from 1) 15.4 -15.8 and 2) 16.5 -16.8 ± 0.2 degrees 2theta. The invention relates to crystalline polymorphs of 6-(Piperidin-4-yloxy)-2H-isoquinolin-1-one hydrochloride, namely to a crystalline form of 6-(Piperidin-4-yloxy)-2H-isoquinolin-1-one hydrochloride wherein the polymorph exhibits in an Xray powder diffractogram using CuKa radiation at least a characteristic reflection within the two ranges selected from 1) 15.4 - 15.8 and 2) 16.5 -16.8 ± 0.2 degrees 2theta, preferably selected from the group consisting of its hydrate, dihydrate, polymorph 1, polymorph 2, polymorph 3, polymorph 4, 1,4-dioxane solvate, acetontrile solvate and methyl acetate solvate. The invention relates to crystalline solvates of 6-(Piperidin-4-yloxy)-2H- isoquinolin-1-one hydrochloride, which has the structure according to formula (I), 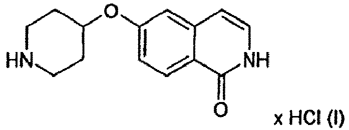 processes for their preparation and their use, in particular for the preparation of medicaments. 6-(Piperidin-4-yloxy)-2H-isoquinolin-1-one is known as a pharmaceutically active compound. 6-(Piperidin-4-yloxy)-2H-isoquinolin-1-one is described as free base in WO 2007/065916. WO2007/012421, WO 2008/077550 and WO 2009/080335 describe the synthesis of 6-(Piperidin-4-yloxy)-2H-isoquinolin-1-one and its hydrochloride but do not contain any evidence of a controlled, reproducible crystallization procedure. The described material is only obtained by lyophilization. The invention relates to a solid form of 6-(Piperidin-4-yloxy)-2H-isoquinolin-1-one hydrochloride, namely to crystalline polymorph of 6-(Piperidin-4-yloxy)-2H-isoquinolin-1-one hydrochloride wherein the polymorph exhibits in an X-ray powder diffractogram using CuKa radiation at least a characteristic reflection within the two ranges selected from 1) 15.4 -15.8 and 2) 16.5 -16.8 ± 0.2 degrees 2theta, which is useful for treating diseases associated with Rho-kinase and/or Rho-kinase mediated phosphorylation of myosin light chain phosphatase, in particular for the treatment and/or prevention of hypertension, pulmonary hypertension, ocular hypertension, retinopathy, glaucoma, peripheral circulatory disorder, peripheral occlusive arterial disease (PAOD), coronary heart disease, angina pectoris, heart hypertrophy, heart failure, ischemic diseases, end organ damage inc!. ischemic organ failure, fibroid lung, fibroid liver, liver failure, nephropathy (including hypertension- induced, non-hypertension-induced, and diabetic nephropathies), renal failure, fibroid kidney, renal glomerulosclerosis, organ hypertrophy, asthma, chronic obstructive pulmonary disease (COPD), adult respiratory distress syndrome, thrombotic disorders, stroke, cerebral vasospasm, cerebral ischemia, pain, e.g. neuropathic pain; neuronal degeneration, spinal cord injury, Alzheimer's disease, premature birth, erectile dysfunction, endocrine dysfunctions, arteriosclerosis, prostatic hypertrophy, diabetes and complications of diabetes, metabolic syndrome, blood vessel restenosis, atherosclerosis, inflammation, autoimmune diseases, osteopathy such as osteoporosis, infection of digestive tracts with bacteria, sepsis, cancer development and progression, e.g cancers of the breast, colon, prostate, ovaries, brain and lung and their metastases. The crystalline polymorph of 6-(Piperidin-4-yloxy)-2H-isoquinolin-1-one hydrochloride wherein the polymorph exhibits in an X-ray powder diffractogram using CuKa radiation at least a characteristic reflection within the two ranges selected from 1) 15.4 -15.8 and 2) 16.5 -16.8 ± 0.2 degrees 2theta is described in PCT/EP2012/062444, published as WO 2013/007519, which is incorporated by reference.

As used herein, unless expressly stated otherwise, the crystalline polymorph of 6-(Piperidin-4-yloxy)-2H-isoquinolin-1-one hydrochloride wherein the polymorph exhibits in an X-ray powder diffractogram using CuKa radiation at least a characteristic reflection within the two ranges selected from 1) 15.4 -15.8 and 2) 16.5 -16.8 ± 0.2 degrees 2theta refers to a polymorph preferably selected from the group consisting of its hydrate, dihydrate, polymorph 1, polymorph 2, polymorph 3, polymorph 4, 1,4-dioxane solvate, acetonitrile solvate and methyl acetate solvate. As used herein, crystalline polymorph of 6-(Piperidin-4-yloxy)-2H-isoquinolin-1-one hydrochloride wherein the polymorph exhibits in an X-ray powder diffractogram using CuKa radiation at least a characteristic reflection within the two ranges selected from 1) 15.4 -15.8 and 2) 16.5 -16.8 ± 0.2 degrees 2theta is a crystalline form of 6-(Piperidin-4-yloxy)-2H-isoquinolin-1-one hydrochloride that is preferably selected from the group consisting of its hydrate, dihydrate, polymorph 1, polymorph 2, polymorph 3, polymorph 4, 1,4-dioxane solvate, acetonitrile solvate and methyl acetate solvate and that is characterized as follows:
General: In one embodiment the present invention relates to a crystalline polymorph of 6-(Piperidin-4-yloxy)-2H-isoquinolin-1-one hydrochloride wherein the polymorph exhibits in an X-ray powder diffractogram using CuKa radiation at least a characteristic reflection within the two ranges selected from 1) 15.4 -15.8 and 2) 16.5 -16.8 ± 0.2 degrees 2theta. In a further embodiment the crystalline form of 6-(Piperidin-4-yloxy)-2H-isoquinolin- 1 -one hydrochloride exhibits in an X-ray powder diffractogram using CuKa radiation an additional characteristic reflection at 21.5 - 21.7 degrees 2theta ±0.2 degrees 2theta.
Hydrate: One embodiment the present invention is a crystalline hydrate of 6-(Piperidin-4- yloxy)-2H-isoquinolin-1 -one hydrochloride (I). Another embodiment the present invention Is a crystalline hydrate of compound (I) wherein the hydrate contains about 10.5 -12.5 % water (w/w).Another embodiment, of the present invention is a hydrate wherein the hydrate contains 1.85 -2.2 molecules water per molecule 6-(Piperidin-4-yloxy)-2H-isoquinolin-1-one hydrochloride (I). Another embodiment, of the present invention is a hydrate wherein the hydrate contains 2 molecules water per molecule 6-(Piperidin-4-yloxy)-2H-isoquinolin-1-one hydrochloride (I). A crystalline hydrate containing 1.85 -2.2 molecules water per molecule is herein referred to as "dihydrate" and is an embodiment of the present invention. In another embodiment of the hydrate the water content is 10.5 - 1 1.4 % water (w/w). In a further embodiment the hydrate contains about 1.85 - 2.0 molecules water. In a further embodiment of the hydrate the water content is 1 1.4 % (w/w). In a further embodiment the hydrate contains 2.0 molecules water per molecule (I). Although the hydrate phase typically contains about 10-12.5% water it can also occur with lower water content. The crystal structure of the hydrate remains even if the dihydrate is dried and the remaining water content is down to about 3 %. Water uptake is reversible if humidity in the environment is raised again. The water content in the isolated product depends on the drying conditions used during work up of the hydrate after crystallisation.In one embodiment the dihydrate has the property of having at least a characteristic reflection in an X-ray powder diffractogram using CuKa radiation at 7.7 ± 0.2 degrees 2theta. In another embodiment the dihydrate has the property of having at least characteristic reflections in an X-ray powder diffractogram using CuKa radiation at 7.7 (strong), 15.2 (strong) and 16.8 (medium) degrees 2theta ± 0.2 degrees 2theta. In another embodiment the dihydrate has the property of having at least characteristic reflections in an X-ray powder diffractogram using CuKa radiation at 7.7 (strong), 15.2 (strong), 16.8 (medium), 18.4 (medium), 20.4 (medium), 22.4 (strong), 25.0 (strong), 26.6 (strong) and 30.3 (medium) degrees 2theta ± 0.2 degree 2theta.
Polymorph 1: Another aspect of the present invention relates to polymorph 1 of 6-(Piperidin-4-yloxy)-2H-isoquinolin-1 -one hydrochloride which has at least characteristic reflections in an X-ray powder diffractogram (XRPD) using CuKai radiation at 4.5 (medium), 15.4 (strong), 16.8 (strong), 21.7 (medium) and 24.7 (medium) degrees 2theta ± 0,2 degrees 2theta. In another aspect polymorph 1 has the property of having at least characteristic reflections in an X-ray powder diffractogram using CuKa radiation at 4.5 (medium), 15.4 (strong), 16.8 (strong), 21.7 (medium), 22.8 (Strong) and 24.7 (medium) degrees 2theta ±0.2 degrees 2theta. In another aspect polymorph 1 has the property of having at least characteristic reflections in an X-ray powder diffractogram using CuKa radiation at 4.5 (medium), 15.4 (strong), 16.8 (strong), 19.8 (weak), 21.7 (medium), 22,5 (strong), 22.8 (Strong), 24.7 (medium) and 27.3 (medium) degrees 2theta ± 0.2 degrees 2theta.
Polymorph 2: Another aspect of the present invention relates to polymorph 2 of compound (I) which has the property of having at least characteristic reflections in an X-ray powder diffractogram using CuKa radiation at 8.1 (medium), 15.8 (strong) and 16.5 (medium) deg 2theta ± 0.2 degrees 2theta. In another aspect the polymorph 2 has the property of having at least characteristic reflections in an X-ray powder diffractogram using CuKa radiation at 8.1 (Medium), 15.8 (Strong), 16.5 (Medium), 22,2 (medium), 25.0 (Medium) and 26.6 (Medium) degrees 2theta ± 0.2 degrees 2theta. In another aspect the polymorph 2 has the property of having at least characteristic reflections in an X-ray powder diffractogram using CuKa radiation at 8.1 (Medium), 15.8 (Strong), 16.5 (Medium), 17.7 (Medium), 19.6 (Medium), 20.8 (Medium), 22.2 (medium), 25.0 (Medium), 26.6 (Medium) and 30.5 (Medium) degrees 2theta + 0.2 degrees 2theta.
Polymorph 3: Another aspect of the present invention relates to polymorph 3 of 6-(Piperidin-4-yioxy)-2H∼isoquinolin-1-one hydrochloride which has the property of having at least characteristic reflections in an X-ray powder diffractogram using CuKa radiation at 4.5 (medium), 15.4 (strong), 16.7 (strong), 21.7 (strong) and 25.5 (medium) degrees 2theta ± 0.2 degrees 2theta. In another aspect polymorph 3 has the property of having characteristic reflections in an X-ray powder diffractogram using CuKa radiation at 4.5 (medium), 15.4 (strong), 16.7 (strong), 21.7 (strong), 22.3 (medium) and 25.5 (medium) degrees 2theta + 0.2 degrees 2theta. In another aspect polymorph 3 has the property of having characteristic reflections in an X-ray powder diffractogram using CuKai radiation at 4.5 (medium), 15.4 (strong), 16.7 (strong), 21.7 (strong), 22.0 (medium), 22.3 (medium) and 25.5 (medium) degrees 2theta ± 0.2 degrees 2theta.
Polymorph 4: The present invention further relates to polymorph 4 of 6-(Piperidin-4-yloxy)-2H- isoquinolin-1-one hydrochloride which has the property of having at least characteristic reflections in an X-ray powder diffractogram using CuKa radiation at 15.4 (medium), 16.7 (medium), 21.5 (strong) and 30.7 (weak) degrees 2theta ± 0.2 degrees 2theta. In another aspect polymorph 4 has the property of having at least characteristic reflections in an X-ray powder diffractogram using CuKa radiation at 15.4 (medium), 16.7 (medium), 16.9 (medium), 21.5 (strong), 22,4 (medium), and 30.7 (weak) degrees 2theta ± 0.2 degrees 2theta. In another aspect polymorph 4 has the property of having at least characteristic reflections in an X-ray powder diffractogram using CuKai radiation at 15.4 (medium), 16.7 (medium), 16.9 (medium), 21.5 (strong), 21.9 (weak), 22.4 (medium), 23.2 (weak), 27.6 (weak) and 30.7 (weak) degrees 2theta ± 0.2 degrees 2theta. Solvates: Moreover, the present invention relates to a 1,4-dioxane solvate, a methyl acetate solvate and an acetonitrile solvate of 6-(Piperidin-4-yloxy)-2H-isoquinolin-1-one hydrochloride.
1,4-dioxane solvate: The 1,4-dioxane solvate according to the invention shows characteristic reflections in an X-ray powder diffractogram using CuKa radiation at 15.1 (strong) and 22.5 (strong) degrees 2theta ± 0.2 degrees 2theta.

In another aspect the 1,4-dioxane solvate has the property of having at least characteristic reflections in an X-ray powder diffractogram using CuKa radiation at 15.1 (strong), 19.7 (medium), 20.3 (medium), 21.6 (medium), 22.5 (strong), 23.8 (medium, 24.9 (medium), and 30.2 (medium) degrees 2theta ± 0.2 degrees 2theta.
Acetonitrile solvate: The acetonitrile solvate is another object of the present invention. This solvate shows at least characteristic reflections in an X-ray powder diffractogram using CuKa radiation at 6.8 {medium), 11.3 (medium) and 27.7 (strong) degrees 2theta + 0.2 degrees 2theta. In another aspect the acetonitrile solvate has the property of having at least characteristic reflections in an X-ray powder diffractogram using CuKa radiation at 6.8 (medium), 11.3 (medium), 15.3 (strong), 20.9 (medium), 23.9 (strong), 24.0 (medium), 27.4 (medium), and 27.7 (strong) degrees 2theta ± 0.2 degrees 2theta.
Methyl acetate solvate: The methyl acetate solvate according to the invention shows at least characteristic reflections in an X-ray powder diffractogram measured in suspension (capillary) using CuKa radiation in transmission mode at 5.0 (strong) and 23.7 (strong) degrees 2theta ± 0.2 degrees 2theta. In another aspect the methyl acetate solvate has the property of having at least characteristic reflections in an X-ray powder diffractogram using CuKa radiation at 6.9 (medium), 15.0 (strong), 20.8 (medium), 22.8 (medium), 23.7 (strong), 24.0 (medium), 25.1 (medium), and 28.0 (medium) degrees 2theta ± 0.2 degrees 2theta.

The crystalline polymorph of 6-(Piperidin-4-yloxy)-2H-isoquinolin-1-one hydrochloride wherein the polymorph exhibits in an X-ray powder diffractogram using CuKa radiation at least a characteristic reflection within the two ranges selected from 1) 15.4 -15.8 and 2) 16.5 -16.8 ± 0.2 degrees 2theta, which is preferably selected from the group consisting of its hydrate, dihydrate, polymorph 1, polymorph 2, polymorph 3, polymorph 4, 1,4-dioxane solvate, acetonitrile solvate and methyl acetate solvate, can be obtained by the following procedures:
Compound (I) as starting material for making the polymorphs and solvates can be obtained as described in WO 2007/012421. Where the dihydrate is used or obtained, this is specified. 6-(Piperidin-4-yloxy)-2H-isoquinolin-1-one hydrochloride is abbreviated as "compound (I)". If not mentioned otherwise drying is carried out over night at reduced pressure (about <50 mbar) at 40°C.
Formation of dihydrate: a) 10 g crude 6-(Piperidin-4-yloxy)-2H-isoquinolin-1-one hydrochloride are dissolved in 25 mL water at 70C. The solution is cooled to 55°C and 75 mL acetone are added. The mixture is cooled to room temperature within 3 hours and left standing for two days for crystallization. After cooling (4°C) for 6 hours the product is isolated via filtration, washed with acetone/water (3:1) and dried in vacuum. 7.9 g (purity 97, 1 %)of 6-(Piperidin-4-yloxy)-2H-isoquinolin-1-one hydrochloride dihydrate are obtained, b) 8.5 g of crude 6-(Piperidin-4-yloxy)-2H-isoquinolin-1-one hydrochloride are dissolved in 21.5 mL water at 65°C. The temperature is lowered to 50°C in 1 h and 32.3 mL acetone are added in 30 mini. The temperature is lowered to 40 °C and the mixture is stirred for 3 h. The reaction mixture is chilled to ambient temperature. The crystalline material is collected, washed with water/acetone (1/3) and dried to yield 4.54 g (purity > 99.9%) of 6-(Piperidin-4-yloxy)-2H-isoquinolin-1-one hydrochloride dihydrate. c) 0.205 g compound (I) (dihydrate) are dissolved in 20 mL ethanol and 3 mL water at about 65 °C. The solvent is allowed to evaporate from the stirred solution at the same temperature over night. d) 0.200 g of compound (I) (dihydrate) are dissolved in 20 mL ethanol and 4 mL water at 65°C. The solution is rapidly cooled to O. After 45 minutes the product is isolated by vacuum filtration and dried. In the same manner the dihydrate is obtained if ethanol is replaced by tetrahydrofuran or methyl ethyl ketone in examples c) and d). e) 0.204 g of compound (I) (dihydrate) are dissolved in 3mL water at 65°C. The solution is rapidly cooled to OX. After 30 minutes the product is isolated by vacuum filtration and dried.
Formation of polymorph 1: 1.2 g of 6-(Piperidin-4-yloxy)-2H-isoquinolin-1-one hydrochloride are suspended in isopropanol and stirred for 6h. The solid material (1.14 g) is isolated by filtration. 60.7 mg thereof are suspended in a mixture of 0.352 mL isopropanol and 0.647 mL methanoi. The mixture is heated until a clear solution is obtained. Upon cooling the crystalline product is obtained which is isolated by filtration.
Formation of polymorph 2: About 3 mg of compound (I) (dihydrate) are at 25°C exposed to a dry nitrogen atmosphere (stream of nitrogen) for at least 6 hours. After this treatment, the X-ray diffraction pattern of the sample corresponds to phase 2.
Formation of polymorph 3: a) 0.201 g of compound (I) (dihydrate) are dissolved in 20mL acetonitril and 3 mL water at 65 °C. The solvent is allowed to evaporate from the stirred solution at the same temperature overnight. Polymorph 3 and traces of the dihydrate are obtained. b) 0.208 g of compound (I) (dihydrate) are dissolved in 10 mL methanol at 65 °C. The stirred solution is rapidly cooled to 0°C. After 30 minutes the product is isolated by vacuum filtration and dried. c) 0.203 g of compound (I) (dihydrate) are suspended in 1.2 mL methanol at 20°C and stirred for 35 days. The product is isolated by vacuum filtration and dried. The same product (polymorph 3) is obtained, if the dihydrate of compound (I) is suspended in ethanol, 1 -propanol or 2-propanol. 5) Formation of polymorph 4: 0.202 g of compound (I) (dihydrate) are suspended in 2.0 mL 2-butanol at 20°C and stirred for 35 days. The product is isolated by vacuum filtration and dried.
Formation of methyl acetate solvate: 0.208 g of compound (I) (dihydrate) are suspended in 2,5 mL of methyl acetate. The solution is stirred in a closed vessel at room temperature for 35 days. The solid present in the suspension is the methyl acetate solvate as determined by XRPD in suspension. After vacuum filtration and drying the dihydrate containing forms 1 and 3 is obtained.
Formation of 1,4 dioxane solvate: 0.204 g of compound (I) (dihydrate) are dissolved in 2.5 mL of 1,4-dioxane at 20 °C for 35 days with continuous stirring. The solid present in the suspension is the methyl acetate solvate as determined by XRPD in suspension. After vacuum filtration and drying the solvate containing dihydrate are obtained.
Formation of acetonitrile solvate: 0.206 g of compound (I) (dihydrate) are suspended in 2.5 mL of acetonitrile. The suspension is stirred at 20 °C for 35 days. The solid present in the suspension is the acetonitrile solvate as determined by XRPD in suspension. The solid present in the suspension is isolated via vacuum filtration and dried overnight at reduced pressure at room temperature. After vacuum filtration and drying the dihydrate containing forms 1 and 3 is obtained.

The invention relates to an administration unit comprising crystalline polymorph of 6-(Piperidin-4-yloxy)-2H-isoquinolin-1-one hydrochloride wherein the polymorph exhibits in an X-ray powder diffractogram using CuKa radiation at least a characteristic reflection within the two ranges selected from 1) 15.4 -15.8 and 2) 16.5 -16.8 ± 0.2 degrees 2theta. The administration unit according to the invention is useful for treating various diseases and disorders which include but are not limited to diseases associated with Rho-kinase and/or Rho-kinase mediated phosphorylation of myosin light chain phosphatase, in particular for the treatment and/or prevention of hypertension, pulmonary hypertension, ocular hypertension, retinopathy, glaucoma, peripheral circulatory disorder, peripheral occlusive arterial disease (PAOD), coronary heart disease, angina pectoris, heart hypertrophy, heart failure, ischemic diseases, end organ damage inc!. ischemic organ failure, fibroid lung, fibroid liver, liver failure, nephropathy (including hypertension- induced, non-hypertension-induced, and diabetic nephropathies), renal failure, fibroid kidney, renal glomerulosclerosis, organ hypertrophy, asthma, chronic obstructive pulmonary disease (COPD), adult respiratory distress syndrome, thrombotic disorders, stroke, cerebral vasospasm, cerebral ischemia, pain, e.g. neuropathic pain; neuronal degeneration, spinal cord injury, Alzheimer's disease, premature birth, erectile dysfunction, endocrine dysfunctions, arteriosclerosis, prostatic hypertrophy, diabetes and complications of diabetes, metabolic syndrome, blood vessel restenosis, atherosclerosis, inflammation, autoimmune diseases, osteopathy such as osteoporosis, infection of digestive tracts with bacteria, sepsis, cancer development and progression, e.g. cancers of the breast, colon, prostate, ovaries, brain and lung and their metastases.

### SPECIFIC INORMATION CONCERNING ASPECT (XI) OF THE INVENTION

Aspect (xi) of the invention concerns forms of {drug11}, especially a form of Compound of the formula (I) or a stereoisomer or pharmaceutically acceptable salt from thereof, namely to amorphous solid dispersion comprising Compound of the formula (I) or a stereoisomer or pharmaceutically acceptable salt from thereof and a polymer wherein the polymer is selected fromgroup consisting of PVP, PVP-vinylacetatcopolymer, HPMC-AS and HPMC, which is useful for treating inflammatory dieseases, osteoarthritis, aneurysm, fever, cardiovascular effects, Crohn's disease, congestive heart failure, autoimmune diseases, HIV-infection, HIV-associated dementia, psoriasis, idiopathic pulmonary fibrosis, cancer. Compound of the formula (I) or a stereoisomer or pharmaceutically acceptable salt from thereof is described by formula (I):

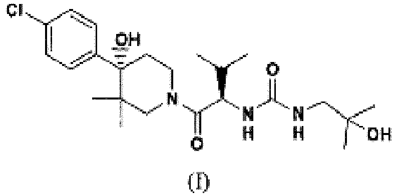

Compound of the formula (I) or a stereoisomer or pharmaceutically acceptable salt from thereof and / or amorphous solid dispersion comprising Compound of the formula (I) or a stereoisomer or pharmaceutically acceptable salt from thereof and a polymer wherein the polymer is selected fromgroup consisting of PVP, PVP-vinylacetatcopolymer, HPMC-AS and HPMC is described in WO 2013/013114, which is incorporated by reference.

The invention relates to an administration unit comprising amorphous solid dispersion comprising Compound of the formula (I) or a stereoisomer or pharmaceutically acceptable salt from thereof and a polymer wherein the polymer is selected fromgroup consisting of PVP, PVP-vinylacetatcopolymer, HPMC-AS and HPMC. The administration unit according to the invention is useful for treating various diseases and disorders which include but are not limited to inflammatory dieseases, osteoarthritis, aneurysm, fever, cardiovascular effects, Crohn's disease, congestive heart failure, autoimmune diseases, HIV-infection, HIV-associated dementia, psoriasis, idiopathic pulmonary fibrosis, cancer. Preferred embodiments of the administration unit are the claims of WO 2013/013114 and cited as items 1 to 15 here:
1. An amorphous solid dispersion comprising Compound (I) of the formula 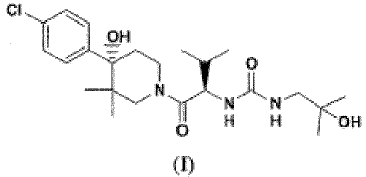 or a stereoisomer or pharmaceutically acceptable salt from thereof and a polymer wherein the polymer is selected from the group consisting of PVP, P VP-vinyl acetate copolymer, HPMC-AS and HPMC.
2. The dispersion according to item 1 wherein the ratio of Compound I to polymer is in the range of from about 99 to about 80% Compound I and from about 1 to about 20% polymer.
3. The dispersion according to item 2 wherein the ratio of Compound I to polymer is in the range of from about 95 to about 85% Compound I and from about 5 to about 15% polymer.
4. The dispersion according to item 3 wherein the ratio of Compound I to polymer is in the range of from about 93 to about 88% Compound I and from about 7 to about 12% polymer.
5. The dispersion according to item 1 prepared by hot-melt extrusion, lyophilization or spray-drying.
6. The dispersion according to item 5 prepared by spray-drying.
7. The dispersion of item 1, wherein said dispersion exhibits less than about 5% crystallization when stored at 25 °C/60% RH for at least 2 years.
8. The dispersion of item 1, wherein said dispersion exhibits less than about 3% degradation of Compound I when stored at 25 °C/60% RH for at least two years.
9. The dispersion according to item 1 wherein the polymer is PVP.
10. The dispersion according to item 1 wherein the polymer is HPMC-AS.
11. A pharmaceutical composition comprised of a pharmaceutically acceptable carrier and a therapeutically effective amount of the dispersion according to item 1.
12. The composition of item 11, wherein said composition is tabletted,
13. The composition of item 11, wherein said composition is prepared in capsule form.
14. A method for treating a disorder comprising administering to a patient in need thereof a therapeutically effective amount of the amorphous solid dispersion according to item 1; wherein said disorder is selected from osteoarthritis, aneurysm, fever, cardiovascular effects, Crohn's disease, congestive heart failure, autoimmune diseases, HIV-infection, HIV-associated dementia, psoriasis, idiopathic pulmonary fibrosis, transplant arteriosclerosis, physically- or chemically-induced brain trauma, neuropathic pain, inflammatory bowel disease, alveolitis, ulcerative colitis, systemic lupus erythematosus, nephrotoxic serum nephritis, glomerulonephritis, asthma, multiple sclerosis, arthrosclerosis, rheumatoid arthritis, restenosis, organ transplantation, multiple myeloma, colorectal cancer, hepatocellular cancer and other cancers.
15. A method for treating inflammatory diseases comprising administering to a patient in need thereof a therapeutically effective amount of the amorphous solid dispersion formulation according to item 1.

Preferred embodiments are described also in WO 2013/013114 and cited here: The present invention provides amorphous solid dispersions of Compound I that have an unexpectedly advantageous pharmaceutical profile. The dispersions of the present invention exhibit superior oral bioavailability and are surprisingly stable both in suspension and in the solid state. In one embodiment, the present invention comprises an amorphous solid dispersion composition or stereoisomers thereof, wherein the polymer used is selected from the group consisting of PVP, P VP-vinyl acetate copolymer, HPMC-AS and HPMC, particularly PVP and HPMC-AS, and more particularly PVP. In another embodiment there is provided the dispersion wherein the w/w% of Compound I to polymer is in the range of from about 99 to about 80% Compound I and from about 1 to about 20% polymer. In a further embodiment, there is provided the dispersion wherein the w/w% of Compound I to polymer is in the range of from about 95 to about 85% Compound I and from about 5 to about 15% polymer. In another embodiment, the w/w% of Compound I to polymer is in the range of from about 93 to about 88% Compound I and from about 7 to about 12% polymer. In yet another embodiment, the dispersion is prepared by hot-melt extrusion, lyophilization, or spray-drying methods, particularly by spray-drying. In another embodiment, a dispersion is provided that is stable in suspension for at least about 5 days. In a further embodiment the dispersion exhibits less than about 5% crystallization when stored at 25 °C/60% RH for at least 2 years. In yet another embodiment Compound I contained within the dispersion exhibits less than about 3% degradation when stored at 25 °C/60% RH for at least two years. A pharmaceutical composition comprised of a pharmaceutically acceptable carrier and a therapeutically effective amount of the dispersion. Another embodiment provides an orally bioavailable composition comprising the dispersion. In a further embodiment the composition is tabletted. A preferred tabletted embodiment comprises the dispersion of the present invention in a weight range of 10-75%, preferably 20-60%, and most preferably 45-55%. A preferred tabletted embodiment comprises one or more fillers, for example lactose and/or microcrystalline cellulose, in a total weight range percent of 10-86%, more preferably 23-76%, and most preferably 35-68%. A preferred tabletted embodiment comprises a disintegrant, for example ac-di-sol, in a weight percent range of 4-10%, preferably 4-9%, and more preferably 5-7%. A preferred tabletted embodiment comprises one or more flow-aids, for example Cab-o-Sil MSP and/or Syloid, in a total weight percent range of 0.1- 3%, and preferably 0.25-2%. A preferred tabletted embodiment comprises a lubricant, for example Mg stearate, in a weight percent range of 0.25- 2.0%, preferably 0.25-1.0%, and more preferably 0.25 - 0.75%. A more preferred tabletted embodiment comprises (in weight percent ranges): 27 - 55% of the dispersion of the present invention, 35-68%, of one or more fillers, 5-7% of a disintegrant, 0.25-2.0% one or more flow-aids, and 0.25-0.75% of a lubricant. In a further embodiment the composition is prepared in capsule form.

In another embodiment, the present invention is directed to i) a method for treating disorders, comprising administering to a patient in need thereof a therapeutically effective amount of an amorphous solid dispersion formulation containing compound (I); ii) use of an amorphous solid dispersion of Compound 1 for use in treating a disorder; or iii) use of an amorphous solid dispersion formulation of Compound 1 in the preparation of a medicament for the treatment of a disorder, wherein said disorder is selected from osteoarthritis, aneurysm, fever, cardiovascular effects, Crohn's disease, congestive heart failure, autoimmune diseases, HIV-infection, HIV-associated dementia, psoriasis, idiopathic pulmonary fibrosis, transplant arteriosclerosis, physically- or chemically-induced brain trauma, neuropathic pain, inflammatory bowel disease, alveolitis, ulcerative colitis, systemic lupus erythematosus, nephrotoxic serum nephritis, glomerulonepliritis, asthma, multiple sclerosis, arthrosclerosis, rheumatoid arthritis, restenosis, organ transplantation, psoriatic arthritis, multiple myeloma, allergies, for example, skin and mast cell degranulation in eye conjunctiva, hepatocellular carcinoma, colorectal cancer, osteoporosis, renal fibrosis, and other cancers, preferably, Crohn's disease, psoriasis, inflammatory bowel disease, systemic lupus erythematosus, multiple sclerosis, rheumatoid arthritis, multiple myeloma, allergies, for example, skin and mast cell degranulation in eye conjunctiva, hepatocellular carcinoma, osteoporosis and renal fibrosis. In a further embodiment, the present invention is directed to i) a method for treating inflammatory diseases, comprising administering to a patient in need thereof a therapeutically effective amount of an amorphous solid dispersion formulation containing Compound (I); ii) use of an amorphous solid dispersion of Compound 1 for use in treating inflammatory diseases; or iii) use of an amorphous solid dispersion formulation of Compound 1 in the preparation of a medicament for the treatment of a inflammatory disease comprising administering to a patient in need thereof a therapeutically effective amount of an amorphous solid dispersion formulation containing compound (I).

The invention may be embodied in other specific forms without departing from the spirit or essential attributes thereof. This invention also encompasses all combinations of alternative aspects of the invention noted herein. It is understood that any and all embodiments of the present invention may be taken in conjunction with any other embodiment to describe additional embodiments of the present invention. Furthermore, any elements of an embodiment may be combined with any and all other elements from any of the embodiments to describe additional embodiments. In order to prepare the compositions hereinabove described, various preparation means available to the skilled artisan may be utilized. The amorphous dispersions of the present invention may be prepared by hot-melt extrusion, lyophilization, or spray-drying. In order to prepare the compositions described herein it is preferred to utilize spray drying procedures available in the art. Preferred spray drying reaction conditions comprise the use of acetone, methanol, or ethanol solutions (2-40 w/v%) with an inlet temperature of the spray dry apparatus of typically about 70-175 °C. Typically, the spray-dried material has particle size wherein 90% of the particles are under 50 µm. Figures 1-3 of WO 2013/013114 confirm the non-crystalline, amorphous nature of the present invention. The dispersions of this invention are surprisingly stable, as illustrated in Example 2 of WO 2013/013114.

In particular, aqueous suspensions of these dispersions are unexpectedly stable as optical microscopy confirms that no crystallization occurs in suspensions of the dispersions, even after 8 days at room temperature (see Example 2 of WO 2013/013114). Also, the dispersions of the present invention are chemically and physically stable. For example based on the data given in Example 2 of WO 2013/013114, it is expected that Compound I contained within the dispersion exhibits less than 3% degradation when stored at 25 °C/60% RH for at least 2 years. In addition, the dispersions are expected to exhibit less than about 5% crystallization when stored at 25 °C/60% RH for at least 2 years.

The compositions of the invention herein described according to the various embodiments may then be tabletted using equipment and procedures available in the art. Furthermore, when desired or necessary, suitable additional binders, lubricants, disintegrating agents, and coloring agents can also be incorporated into the tabletting mixture. Suitable binders include starch, gelatin, natural sugars such as glucose or beta-lactose, com sweeteners, natural and synthetic gums such as acacia, tragacanth or sodium alginate, carboxymethylcellulose, polyethylene glycol, and the like. Lubricants used in these dosage forms include sodium oleate, magnesium stearate, and the like. Disintegrators include, without limitation, starch, methyl cellulose, agar, bentonite, xanthan gum, and the like. Tablets are formulated, for example, by preparing a powder mixture, granulating or slugging, adding a lubricant and disintegrant, and pressing into tablets. A powder mixture is prepared by mixing the compound, suitable comminuted, with a diluent or base as described above, and optionally, with a binder such as carboxymethylcellulose, an alginate, gelating, or polyvinyl pyrrolidone, a solution retardant such as paraffin, a resorption accelerator such as a quaternary salt and/or and absorption agent such as bentonite, kaolin, or dicalcium phosphate. The powder mixture can be granulated by wetting with a binder such as syrup, starch paste, acadia mucilage, or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc, or mineral oil. The lubricated mixture is then compressed into tablets. The compounds of the present invention can also be combined with a free flowing inert carrier and compressed into tablets directly without going through the granulating or slugging steps. A clear or opaque protective coating consisting of a sealing coat of shellac, a coating of sugar or polymeric material, and a polish coating of wax can be provided. Dyestuffs can be added to these coatings to distinguish different unit dosages.

By way of non-limiting example, tablets containing about 1 to about1OOO mg, preferably about 50 to about600 mg and preferably about 100 to about 500 mg of Compound I may be made using the compositions herein described. Other dosage units are within the scope of the invention hereof. In particular, tablets containing spray-dried composition containing Compound I and a polymer, including PVP and HPMC- AS have shown improved in vitro dissolution rates, good in vivo oral bioavailability in dogs, and good chemical/physical stability.

Further, a method of inhibiting CCR1 activity in a patient comprises administering to the patient a therapeutically effective amount of a pharmaceutical tablet according to one or more of the embodiments hereinabove described. The term "therapeutically effective amount" means the total amount of the active component of the method that is sufficient to show a patient benefit, i.e., symptomatic or disease modifymg treatment. When applied to an individual active ingredient, administered alone, the term refers to that ingredient alone. When applied to a combination, the term refers to combined amounts of the active ingredients that result in the therapeutic effect, whether administered in combination, serially or simultaneously. This invention also encompasses all combinations of alternative aspects of the invention noted herein. It is understood that any and all embodiments of the present invention may be taken in conjunction with any other embodiment to describe additional embodiments of the present invention. Furthermore, any elements of an embodiment may be combined with any and all other elements from any of the embodiments to describe additional embodiments. The foregoing description is merely illustrative and should not be understood to limit the scope or underlying principles of the invention in any way. Indeed, various modifications of the invention, in addition to those shown and described herein, will become apparent to those skilled in the art from the following examples and the foregoing description. Such modifications are also intended to fall within the scope of the appended claims.

### DEFINITIONS

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th Edition, p. 1418, Mack Publishing Company, Easton, PA (1985), the disclosure of which is hereby incorporated by reference. Said references are incorporated herein by reference. In addition, compound of the formula I are, subsequent to their preparation, preferably isolated and purified to obtain a composition containing an amount by weight equal to or greater than 99% formula I compound ("substantially pure" compound I), which is then used or formulated as described herein. Such "substantially pure" compounds of the formula I are also contemplated herein as part of the present invention. All stereoisomers of the compounds of the instant invention are contemplated, either in admixture or in pure or substantially pure form. The compounds of the present invention can have asymmetric centers at any of the carbon atoms including any one of the R substituents and/or exhibit polymorphism. Consequently, compounds of formula I can exist in Enantiomeric, or diastereomeric forms, or in mixtures thereof. The processes for preparation can utilize racemates, enantiomers, or diastereomers as starting materials. When diastereomeric or enantiomeric products are prepared, they can be separated by conventional methods for example, chromatographic or fractional crystallization. "Stable compound" and "stable structure" are meant to indicate a compound that is sufficiently robust to survive isolation to a useful degree of purity from a reaction mixture, and formulation into an efficacious therapeutic agent. The present invention is intended to embody stable compounds. "Therapeutically effective amount" is intended to include an amount of a compound of the present invention alone or an amount of the combination of compounds claimed or an amount of a Compound of the present invention in combination with other active ingredients effective to inhibit MIP-Iα or effective to treat or prevent inflammatory disorders. As used herein, "treating" or "treatment" cover the treatment of a disease-state in a mammal, particularly in a human, and include; (a) preventing the disease-state from occurring in a mammal, in particular, when such mammal is predisposed to the disease-state but has not yet been diagnosed as having it; (b) inhibiting the disease-state, i.e., arresting it development; and/or (c) relieving the disease-state, i.e., causing regression of the disease state.

The synthesis of Compound I may be prepared as described in patent application US 2009/0326010 A1 assigned to the Applicant of WO 2013/013114 which published on December 31, 2009 in reaction schemes, descriptions and examples thereof, as well by relevant literature procedures that are known to one skilled in the art.

### PREFERRED EMBODIMENTS CONCERNING ASPECTS (I), (II), (III), (IV), (V), (VI), (VII), (VIII), (IX), (X), (XI), (XII), (XIII), (XIV) AND (XV) OF THE INVENTION

The invention relates to an administration unit comprising (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.

Preferably, the administration unit according to the invention comprises a therapeutically effective amount of (v) {drug5}, (vii) {drug7}, (viii) {drug8}, (ix) {drug9}, or (xi) {drug11}, respectively , wherein at least 10 % by weight of said therapeutically effective amount of (v) {drug5}, (vii) {drug7}, (viii) {drug8}, (ix) {drug9}, or(xi) {drug11}, respectively are present as (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}. Preferably, at least 20 % by weight, or at least 30 % by weight, or at least 40 % by weight, or at least 50 % by weight, or at least 60 % by weight, or at least 70 % by weight, or at least 80 % by weight, or at least 90 % by weight of said therapeutically effective amount of (v) {drug5}, (vii) {drug7}, (viii) {drug8}, (ix) {drug9}, or (xi) {drug11}, respectively are present as (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}. Preferably, at least 95 % by weight, or at least 96 % by weight, or at least 97 % by weight, or at least 98 % by weight, or at least 99 % by weight, or at least 99.5 % by weight, or at least 99.8 % by weight, or at least 99.9 % by weight of said therapeutically effective amount of (v) {drug5}, (vii) {drug7}, (viii) {drug8}, (ix) {drug9}, or (xi) {drug11}, respectively are present as (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.

The total content of (v) {drug5}, (vii) {drug7}, (viii) {drug8}, (ix) {drug9}, or (xi) {drug11}, respectively as well as the relative content of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; can be determined by standard analysis known to the skilled artisan. Suitable methods include but are not limited to thermal analysis, HPLC and XRPD.

In a preferred embodiment of the administration unit according to the invention, not more than 90 % by weight of said therapeutically effective amount of (v) {drug5}, (vii) {drug7}, (viii) {drug8}, (ix) {drug9}, or (xi) {drug11}, respectively are amorphous. Preferably, not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (v) {drug5}, (vii) {drug7}, (viii) {drug8}, (ix) {drug9}, or (xi) {drug11}, respectively are amorphous. Preferably, not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (v) {drug5}, (vii) {drug7}, (viii) {drug8}, (ix) {drug9}, or (xi) {drug11}, respectively are amorphous.

The total content of (v) {drug5}, (vii) {drug7}, (viii) {drug8}, (ix) {drug9}, or (xi) {drug11}, respectively as well as the relative content of amorphous (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; can be determined by standard analysis known to the skilled artisan. Suitable methods include but are not limited to thermal analysis, HPLC and XRPD (see e.g. K.D. Harris, Powder diffraction crystallography of molecular solids, Top Curr Chem., 2012, 315:133-77).

In a preferred embodiment of the administration unit according to the invention, not more than 90 % by weight of said therapeutically effective amount of (v) {drug5}, (vii) {drug7}, (viii) {drug8}, (ix) {drug9}, or (xi) {drug11}, respectively are polymorphs of (v) {drug5}, (vii) {drug7}, (viii) {drug8}, (ix) {drug9}, or (xi) {drug11}, respectively other than (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}. Preferably, not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (v) {drug5}, (vii) {drug7}, (viii) {drug8}, (ix) {drug9}, or (xi) {drug11}, respectively are polymorphs of (v) {drug5}, (vii) {drug7}, (viii) {drug8}, (ix) {drug9}, or (xi) {drug11}, respectively other than (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}. Preferably, not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (v) {drug5}, (vii) {drug7}, (viii) {drug8}, (ix) {drug9}, or (xi) {drug11}, respectively are polymorphs of (v) {drug5}, (vii) {drug7}, (viii) {drug8}, (ix) {drug9}, or (xi) {drug11}, respectively other than (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.

The total content of (v) {drug5}, (vii) {drug7}, (viii) {drug8}, (ix) {drug9}, or (xi) {drug11}, respectively as well as the relative content of polymorphs other than (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; can be determined by standard analysis known to the skilled artisan. Suitable methods include but are not limited to thermal analysis, HPLC and XRPD (see e.g. N. Chieng, T. Rades, J.Aaltonen, An overview of recent studies on the analysis of pharmaceutical polymorphs, J Pharm Biomed Anal. 2011, 25:55(4):618-44; R. Hilfiker, Polymorplism, Wiley-VCH, 1st ed. 2006; H.G. Brittain, Polymorphism in Pharmaceutical Solids (Drugs and the Pharmaceutical Sciences), Informa Healthcare, 2nd ed. 2009).

In a preferred embodiment of the administration unit according to the invention, the therapeutically effective amount of (v) {drug5}, (vii) {drug7}, (viii) {drug8}, (ix) {drug9}, or (xi) {drug11}, respectively is 0.1 µg to 2 g of (v) {drug5}, (vii) {drug7}, (viii) {drug8}, (ix) {drug9}, or (xi) {drug11}, respectively. Preferably, the therapeutically effective amount of (v) {drug5}, (vii) {drug7}, (viii) {drug8}, (ix) {drug9}, or (xi) {drug11}, respectively is 1 µg to 2.5 µg of (v) {drug5}, (vii) {drug7}, (viii) {drug8}, (ix) {drug9}, or (xi) {drug11}, respectively, or 2.5 µg to 5 µg of (v) {drug5}, (vii) {drug7}, (viii) {drug8}, (ix) {drug9}, or (xi) {drug11}, respectively , or 5 µg to 10 µg of (v) {drug5}, (vii) {drug7}, (viii) {drug8}, (ix) {drug9}, or (xi) {drug11}, respectively , or 10 µg to 25 µg of (v) {drug5}, (vii) {drug7}, (viii) {drug8}, (ix) {drug9}, or (xi) {drug11}, respectively , or 25 µg to 50 µg of (v) {drug5}, (vii) {drug7}, (viii) {drug8}, (ix) {drug9}, or (xi) {drug11}, respectively , or 50 µg to 100 µg of (v) {drug5}, (vii) {drug7}, (viii) {drug8}, (ix) {drug9}, or (xi) {drug11}, respectively , or 100 µg to 250 µg of (v) {drug5}, (vii) {drug7}, (viii) {drug8}, (ix) {drug9}, or (xi) {drug11}, respectively , or 250 µg to 500 µg of (v) {drug5}, (vii) {drug7}, (viii) {drug8}, (ix) {drug9}, or (xi) {drug11}, respectively , or 500 µg to 1 mg of (v) {drug5}, (vii) {drug7}, (viii) {drug8}, (ix) {drug9}, or (xi) {drug11}, respectively , or 1 mg to 2.5 mg of (v) {drug5}, (vii) {drug7}, (viii) {drug8}, (ix) {drug9}, or (xi) {drug11}, respectively , or 2.5 mg to 5 mg of (v) {drug5}, (vii) {drug7}, (viii) {drug8}, (ix) {drug9}, or (xi) {drug11}, respectively , or 5 mg to 10 mg of (v) {drug5}, (vii) {drug7}, (viii) {drug8}, (ix) {drug9}, or (xi) {drug11}, respectively , or 10 mg to 25 mg of (v) {drug5}, (vii) {drug7}, (viii) {drug8}, (ix) {drug9}, or (xi) {drug11}, respectively , or 25 mg to 50 mg of (v) {drug5}, (vii) {drug7}, (viii) {drug8}, (ix) {drug9}, or (xi) {drug11}, respectively, or 50 mg to 100 mg of (v) {drug5}, (vii) {drug7}, (viii) {drug8}, (ix) {drug9}, or (xi) {drug11}, respectively , or 100 mg to 250 mg of (v) {drug5}, (vii) {drug7}, (viii) {drug8}, (ix) {drug9}, or (xi) {drug11}, respectively , or 250 mg to 500 mg of (v) {drug5}, (vii) {drug7}, (viii) {drug8}, (ix) {drug9}, or (xi) {drug11}, respectively , or 500 mg to 1 g of (v) {drug5}, (vii) {drug7}, (viii) {drug8}, (ix) {drug9}, or (xi) {drug11}, respectively , or 1 g to 1.25 g of (v) {drug5}, (vii) {drug7}, (viii) {drug8}, (ix) {drug9}, or (xi) {drug11}, respectively, or 1.25 g to 1.5 g of (v) {drug5}, (vii) {drug7}, (viii) {drug8}, (ix) {drug9}, or (xi) {drug11}, respectively , or 1.5 g to 1.75 g of (v) {drug5}, (vii) {drug7}, (viii) {drug8}, (ix) {drug9}, or (xi) {drug11}, respectively , or 1.75 g to 2 g of (v) {drug5}, (vii) {drug7}, (viii) {drug8}, (ix) {drug9}, or (xi) {drug11}, respectively.

Preferably, the administration unit according to the invention comprises 0.1 µg to 2 g of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}. Preferably, it comprises 1 µg to 2.5 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 2.5 µg to 5 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 5 µg to 10 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 10 µg to 25 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 25 µg to 50 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 50 µg to 100 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 100 µg to 250 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 250 µg to 500 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 500 µg to 1 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 1 mg to 2.5 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 2.5 mg to 5 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 5 mg to 10 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 10 mg to 25 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 25 mg to 50 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 50 mg to 100 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 100 mg to 250 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 250 mg to 500 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 500 mg to 1 g of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 1 g to 1.25 g of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 1.25 g to 1.5 g of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 1.5 g to 1.75 g of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 1.75 g to 2 g of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.

Preferably, the administration unit according to the invention contains (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; as substantially the only form of (v) {drug5}, (vii) {drug7}, (viii) {drug8}, (ix) {drug9}, or (xi) {drug11}, respectively , i.e. preferably contains neither non-crystalline forms of (v) {drug5}, (vii) {drug7}, (viii) {drug5}, (ix) {drug9}, or (xi) {drug11}, respectively nor crystalline forms other than (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.

The administration unit according to the invention and its constituents, i.e. (v) {drug5}, (vii) {drug7}, (viii) {drug5}, (ix) {drug9}, or(xi) {drug11}, respectively and (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; respectively, as well as pharmaceutical excipients, can be characterized by analytic methods that are known to the skilled artisan and described in Ph. Eur. and USP (see e.g. H.G. Brittain, S.J. Bodanowich, D.E. Bugay, J. DeVoncentis, G. Lewen, A.W. Newman, Physical characterization of pharmaceutical solids, Pharm Res. 1991, 8(8):963-73; D.E. Bugay, Characterization of the solid-state: spoectroscopic techniques, Adv Drug Deliv Rev., 2001, 48(1):43-65; P.A. Tishmack, D.E. Bugay, S.R. Byrn, Solid-state nuclear magnetic resonance spectroscopy - pharmaceutical application, J Pharm Sci, 2003, 92(3):441-74; C.J. Lee, C.J. Strachan, P.J. Manson, T. Rades, Characterization of the bulk properties of pharmaceutical solids using nonlinear optics - a review, J Pharm Pharmacol., 2007, 59(2):241-50); R.A. Storey, Solid State Characterization of Pharmaceuticals, Wiley-Blackwell, 2011).

In a preferred embodiment, the administration unit according to the invention contains (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; in form of particles, wherein the particle size distribution of X90 is about 500 µm or less, i.e. a particle size distribution (preferably by volume) such that at least 90 % of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; has a particle size smaller than 500 µm. Preferably, for (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i} (ix) {drug9a}; (xi) {drug11a}, {drug11b}, {drug11c}, or {drug11d}; the particle size distribution of X90 is about 480 µm or less, or about 460 µm or less, or about 440 µm or less, or about 420 µm or less, or about 400 µm or less, or about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 60 µm or less, or about 40 µm or less, or about 20 µm or less.

In a preferred embodiment, the administration unit according to the invention contains (v) {drag5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; in form of particles, wherein the particle size distribution of X50 is about 400 µm or less, i.e. a particle size distribution (preferably by volume) such that at least 50 % of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; has a particle size smaller than 400 µm. Preferably, for (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; the particle size distribution of X50 is about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 70 µm or less, or about 60 µm or less, or about 50 µm or less. Preferably, for (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; the particle size distribution of X50 is about 45 µm or less, or about 40 µm or less, or about 35 µm or less, or about 30 µm or less, or about 25 µm or less, or about 20 µm or less, or about 15 µm or less, or about 10 µm or less.

According to USP, the following parameters may be defined based on the cumulative distribution. QR(X) = cumulative distribution of particles with a dimension less than or equal to X [µm] where the subscript R reflects the distribution type: 0 Number, 1 Length, 2 Area, 3 Volume. Therefore, by definition: QR(X) = 0.90 when X = X90; QR(X) = 0.50 when X = X50; and QR(X) = 0.10 when X = X10.

In preferred embodiments, the administration unit according to the invention contains (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; in form of particles having a cumulative distribution by volume basis Q3(X) (X in µm) of Q3(355) <0.50; or Q3(180) <0.50 and Q3(355) ≥0.50; or Q3(125) <0.50 and Q3(180) ≥0.50; or Q3(125) ≥0.50.

In preferred embodiments, the administration unit according to the invention contains (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; in form of particles having a cumulative distribution by volume basis Q3(X) (X in µm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50. In preferred embodiments, the administration unit according to the invention contains (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; in form of particles having a cumulative distribution by volume basis Q3(X) (X in µm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

The indicated particle size properties are determined by laser-diffraction method, in particular low angle laser light scattering, i.e. Fraunhofer diffraction. Alternatively, the particle size properties can be also determined by microscopy (e.g. electron microscopy or scanning electron microscopy). The powder fineness is preferably determined in accordance with USP35 <811>. The results of the particle size distribution determined by different techniques can be correlated with one another.

Preferably, the administration unit according to the invention is solid, semisolid or liquid.

Preferably, the administration unit according to the invention is for administration once daily, or twice daily, or thrice daily, or four times daily, or five times daily, or six times daily.

Preferably, the administration unit according to the invention is monolithic or multiparticulate.

In a preferred embodiment of the administration unit according to the invention, (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; is homogeneously distributed over the administration unit.

Excipient composition and homogeneity can be determined by standard analysis known to the skilled artisan. Suitable methods include but are not limited to near-infrared chemical imaging.

In another preferred embodiment of the administration unit according to the invention, (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; is not homogeneously distributed over the administration unit.

In a preferred embodiment, the administration unit according to the invention provides controlled release of (v) {drug5}, (vii) {drug7}, (viii) {drug8}, (ix) {drug9}, or (xi) {drug11}, respectively. Preferably, the administration unit according to the invention comprises a controlled release matrix or a controlled release coating.

In a preferred embodiment, under in vitro conditions (paddle apparatus, 75 rpm, 900 mL artificial gastric juice, pH 1.2) the administration unit according to the invention has released after 1 hour not more than 90 % of (v) {drug5}, (vii) {drug7}, (viii) {drug8}, (ix) {drug9}, or (xi) {drug11}, respectively. Preferably, the administration unit according to the invention has released after 1 hour not more than 80 % of (v) {drug8}, (vii) {drug7}, (viii) {drug8}, (ix) {drug9}, or (xi) {drug11}, respectively , or not more than 70 % of (v) {drug5}, (vii) {drug7}, (viii) {drug8}, (ix) {drug9}, or (xi) {drug11}, respectively , or not more than 60 % of (v) {drug5}, (vii) {drug7}, (viii) {drug8}, (ix) {drug9}, or (xi) {drug11}, respectively , or not more than 50 % of (v) {drug5}, (vii) {drug7}, (viii) {drug8}, (ix) {drug9}, or (xi) {drug11}, respectively , or not more than 40 % of (v) {drug5}, (vii) {drug7}, (viii) {drug8}, (ix) {drug9}, or (xi) {drug11}, respectively , or not more than 30 % of (v) {drug5}, (vii) {drug7}, (viii) {drug8}, (ix) {drug9}, or (xi) {drug11}, respectively , or not more than 20 % of (v) {drug5}, (vii) {drug7}, (viii) {drug8}, (ix) {drug9}, or (xi) {drug11}, respectively , or not more than 10 % of (v) {drug5}, (vii) {drug7}, (viii) {drug8}, (ix) {drug9}, or (xi) {drug11}, respectively.

In a preferred embodiment, under in vitro conditions (paddle apparatus, 75 rpm, 900 mL artificial gastric juice, pH 1.2) the administration unit according to the invention has released after 2 hours not more than 90 % of (v) {drug5}, (vii) {drug7}, (viii) {drug8}, (ix) {drug9}, or (xi) {drug11}, respectively. Preferably, the administration unit according to the invention has released after 2 hours not more than 80 % of (v) {drug8}, (vii) {drug7}, (viii) {drug8}, (ix) {drug9}, or (xi) {drug11}, respectively , or not more than 70 % of (v) {drug5}, (vii) {drug7}, (viii) {drug8}, (ix) {drug9}, or (xi) {drug11}, respectively , or not more than 60 % of (v) {drug5}, (vii) {drug7}, (viii) {drug8}, (ix) {drug9}, or (xi) {drug11}, respectively , or not more than 50 % of (v) {drug5}, (vii) {drug7}, (viii) {drug8}, (ix) {drug9}, or (xi) {drug11}, respectively , or not more than 40 % of (v) {drug5}, (vii) {drug7}, (viii) {drug8}, (ix) {drug9}, or (xi) {drug11}, respectively , or not more than 30 % of (v) {drug5}, (vii) {drug7}, (viii) {drug8}, (ix) {drug9}, or (xi) {drug11}, respectively , or not more than 20 % of (v) {drug5}, (vii) {drug7}, (viii) {drug8}, (ix) {drug9}, or (xi) {drug11}, respectively , or not more than 10 % of (v) {drug5}, (vii) {drug7}, (viii) {drug8}, (ix) {drug9}, or (xi) {drug11}, respectively.

In a preferred embodiment, under in vitro conditions (paddle apparatus, 75 rpm, 900 mL artificial gastric juice, pH 1.2) the administration unit according to the invention has released after 4 hours not more than 90 % of (v) {drug5}, (vii) {drug7}, (viii) {drug8}, (ix) {drug9}, or (xi) {drug11}, respectively. Preferably, the administration unit according to the invention has released after 4 hours not more than 80 % of (v) {drug5}, (vii) {drug7}, (viii) {drug8}, (ix) {drug9}, or (xi) {drug11}, respectively , or not more than 70 % of (v) {drug5}, (vii) {drug7}, (viii) {drug8}, (ix) {drug9}, or (xi) {drug11}, respectively , or not more than 60 % of (v) {drug5}, (vii) {drug7}, (viii) {drug8}, (ix) {drug9}, or (xi) {drug11}, respectively , or not more than 50 % of (v) {drug5}, (vii) {drug7}, (viii) {drug8}, (ix) {drug9}, or (xi) {drug11}, respectively , or not more than 40 % of (v) {drug5}, (vii) {drug7}, (viii) {drug8}, (ix) {drug9}, or (xi) {drug11}, respectively , or not more than 30 % of (v) {drug5}, (vii) {drug7}, (viii) {drug8}, (ix) {drug9}, or (xi) {drug11}, respectively, or not more than 20 % of (v) {drug5}, (vii) {drug7}, (viii) {drug8}, (ix) {drug9}, or (xi) {drug11}, respectively , or not more than 10 % of (v) {drug5}, (vii) {drug7}, (viii) {drug8}, (ix) {drug9}, or (xi) {drug11}, respectively.

In a preferred embodiment, under in vitro conditions (paddle apparatus, 75 rpm, 900 mL artificial gastric juice, pH 1.2) the administration unit according to the invention has released after 8 hours not more than 90 % of (v) {drug5}, (vii) {drug7}, (viii) {drug8}, (ix) {drug9}, or (xi) {drug11}, respectively. Preferably, the administration unit according to the invention has released after 8 hours not more than 80 % of (v) {drug5}, (vii) {drug7}, (viii) {drug8}, (ix) {drug9}, or (xi) {drug11}, respectively , or not more than 70 % of (v) {drug5}, (vii) {drug7}, (viii) {drug8}, (ix) {drug9}, or (xi) {drug11}, respectively , or not more than 60 % of (v) {drug5}, (vii) {drug7}, (viii) {drug8}, (ix) {drug9}, or (xi) {drug11}, respectively , or not more than 50 % of (v) {drug5}, (vii) {drug7}, (viii) {drug8}, (ix) {drug9}, or (xi) {drug11}, respectively , or not more than 40 % of (v) {drug5}, (vii) {drug7}, (viii) {drug8}, (ix) {drug9}, or (xi) {drug11}, respectively , or not more than 30 % of (v) {drug5}, (vii) {drug7}, (viii) {drug8}, (ix) {drug9}, or (xi) {drug11}, respectively, or not more than 20 % of (v) {drug5}, (vii) {drug7}, (viii) {drug8}, (ix) {drug9}, or (xi) {drug11}, respectively , or not more than 10 % of (v) {drug5}, (vii) {drug7}, (viii) {drug8}, (ix) {drug9}, or (xi) {drug11}, respectively. In another preferred embodiment, the administration unit according to the invention provides immediate release of (v) {drug5}, (vii) {drug7}, (viii) {drug8}, (ix) {drug9}, or (xi) {drug11}, respectively.

In a preferred embodiment, under in vitro conditions (paddle apparatus, 75 rpm, 900 mL artificial gastric juice, pH 1.2) the administration unit according to the invention has released after 0.5 hours at least 10 % of (v) {drug8}, (vii) {drug7}, (viii) {drug8}, (ix) {drug9}, or (xi) {drug11}, respectively. Preferably, the administration unit according to the invention has released after 0.5 hours at least 20 % of (v) {drug5}, (vii) {drug7}, (viii) {drug8}, (ix) {drug9}, or (xi) {drug11}, respectively , or at least 30 % of (v) {drug5}, (vii) {drug7}, (viii) {drug8}, (ix) {drug9}, or (xi) {drug11}, respectively , or at least 40 % of (v) {drug5}, (vii) {drug7}, (viii) {drug8}, (ix) {drug9}, or (xi) {drug11}, respectively , or at least 50 % of (v) {drug5}, (vii) {drug7}, (viii) {drug8}, (ix) {drug9}, or (xi) {drug11}, respectively , or at least 60 % of (v) {drug5}, (vii) {drug7}, (viii) {drug8}, (ix) {drug9}, or (xi) {drug11}, respectively , or at least 70 % of (v) {drug5}, (vii) {drug7}, (viii) {drug8}, (ix) {drug9}, or (xi) {drug11}, respectively , or at least 80 % of (v) {drug5}, (vii) {drug7}, (viii) {drug8}, (ix) {drug9}, or (xi) {drug11}, respectively , or at least 90 % of (v) {drug5}, (vii) {drug7}, (viii) {drug8}, (ix) {drug9}, or (xi) {drug11}, respectively.

Preferably, the administration unit according to the invention has a total weight of 10 mg to 3 g. Preferably, the administration unit according to the invention has a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.

In a preferred embodiment, the administration unit according to the invention is for systemic or topical administration.

In another preferred embodiment, the administration unit according to the invention is for parenteral administration.

Preferably, the administration unit according to the invention is for oral, buccal, opthalmic, nasal, rectal, transmucosal, or intestinal administration. Preferably, it is for oral administration.

Preferably, the administration unit according to the invention is selected from the group consisting of tablets, capsules, effervescent tablets, orodispersible tablets, dragees, sachets, drops, suspensions, and powders. Preferably, it is a tablet. Preferably, the administration unit according to the invention is round or oblong; and/or is planar or biconvex.

In a preferred embodiment, the administration unit according to the invention has a round cross-section with a diameter of 1 mm to 20 mm. Preferably, the diameter of the round cross-section is 1 mm to 3 mm, or 3 mm to 5 mm, or 5 mm to 8 mm, or 8 mm to 10 mm, or 10 mm to 13 mm, or 13 mm to 15 mm, or 15 mm to 18 mm, or 18 mm to 20 mm.

In a preferred embodiment, the administration unit according to the invention is oblong and has an aspect ratio of at least 1.1:1. Preferably, the aspect ratio is at least 4:3, or at least 21/2:1, or at least 3:2, or at least 16:10, or at least □:1, or at least 5:3, or at least 16:9.

In a preferred embodiment, the administration unit according to the invention has been manufactured by direct compression. In another preferred embodiment, the administration unit according to the invention has been manufactured by granulation and subsequent compression of granules. Preferably, granulation is wet granulation or dry granulation.

Preferably, the administration unit according to the invention has been compacted at a pressure of 10 MPa to 10,000 MPa. Preferably, the administration unit according to the invention has been compacted at a pressure of 10 MPa to 25 MPa, or 25 MPa to 50 MPa, or 50 MPa to 100 MPa, or 100 MPa to 250 MPa, or 250 MPa to 500 MPa, or 500 MPa to 1000 MPa, or 1000 MPa to 2500 MPa, or 2500 MPa to 5000 MPa, or 5000 MPa to 10,000 MPa.

In a preferred embodiment, the administration unit according to the invention comprises a film coating. Preferably, the film coating is enteric. Suitable enteric coating materials are known to the skilled artisan and include but are not limited to methyl acrylate-methacrylic acid copolymers, cellulose acetate succinate, hydroxy propyl methyl cellulose phthalate, hydroxy propyl methyl cellulose acetate succinate (hypromellose acetate succinate), polyvinyl acetate phthalate (PVAP), methyl methacrylate-methacrylic acid copolymers, sodium alginate and stearic acid.

In a preferred embodiment of the administration unit according to the invention, the film coating comprises a cellulose ether (e.g. hydroxypropylmethyl cellulose) or a synthetic polymer (e.g. polyvinyl alcohol).

In a preferred embodiment of the administration unit according to the invention, the film coating has an average thickness of 0.01 µm to 0.03 µm, or 0.03 to 0.05 µm, or 0.05 to 0.1 µm, or 0.1 to 0.25 µm, or 0.25 to 0.5 µm, or 0.5 to 1.0 µm, or 1.0 µm to 2.5 µm, or 2.5 µm to 5.0 µm, or 5.0 µm to 10 µm, or 10 µm to 25 µm, or 25 µm to 50 µm, or 50 µm to 100 µm, or 100 µm to 250 µm, or 250 µm to 500 µm, or 500 µm to 1000 µm.

In a preferred embodiment, the administration unit according to the invention has a tablet porosity of not more than 90 %. Preferably, it has a tablet porosity of not more than 80 %, or not more than 70 %, or not more than 60 %, or not more than 50 %, or not more than 40 %, or not more than 30 %, or not more than 20 %, or not more than 10 %. Preferably, it has a tablet porosity of not more than 5 %, or not more than 4 %, or not more than 3 %, or not more than 2 %, or not more than 1 %, or not more than 0.5 %, or not more than 0.2 %, or not more than 0.1 %.

The most common strength test in pharmaceutical applications is the diametral compression test, which is used to calculate the radial tensile strength of a tablet (Fell, J.T., Newton, J.M., 1970. Determination of tablet strength by diametral compression test. J. Pharm. Sci. 59, 688-691). In order to calculate the radial tensile strength, the stress conditions have to be such that the tablet fails in tension. During radial tensile strength measurements, the fracture occurs through a predetermined diametral cross section of the tablet. Therefore, the radial tensile strength is likely to reflect the average strength of tablet rather than the strength of the weakest plane in the tablet.

In a preferred embodiment, the administration unit according to the invention has a radial tensile strength of 0.1 to 100 MPa. Preferably, it has a radial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

Another method for determining mechanical strength is to measure the axial tensile strength. The force necessary to break the tablet is obtained by pulling the tablet parallel to the applied force during the formation of the tablet and this force is then used to calculate the axial tensile strength (Nyström, C., Malmqvist, K., Mazur, J., Alex, W., Hölzer, A.W., 1978. Measurement of axial and radial tensile strength of tablets and their relation to capping. Acta Pharm. Suec. 15, 226-232). During axial tensile strength measurements, the fracture will occur through the weakest plane in the tablet. Consequently, this method allows detection of capping tendencies in a tablet.

In a preferred embodiment, the administration unit according to the invention has an axial tensile strength of 0.1 to 100 MPa. Preferably, it has an axial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

In a preferred embodiment, the administration unit according to the invention has an average pore size of 0.001 µm to 1000 µm. Preferably, it has an average pore size of 0.001 µm to 0.003 µm, or 0,003 µm to 0.005 µm, or 0.005 µm to 0.01 µm, or 0.01 µm to 0.025 µm, or 0.025 µm to 0.05 µm, or 0.05 µm to 0.1 µm, or 0.1 µm to 0.25 µm, or 0.25 µm to 0.5 µm, or 0.5 µm to 1 µm, or 1 µm to 2.5 µm, or 2.5 µm to 5 µm, or 5 µm to 10 µm, or 10 µm to 25 µm, or 25 µm to 50 µm, or 50 µm to 100 µm, or 100 µm to 250 µm, or 250 µm to 500 µm, or 500 µm to 1000 µm.

The pore size distribution of a tablet may be assessed by methods that are known to the skilled artisan and include but are not limited to gas adsorption (e.g. Stanley-Wood, N.G., Johansson, M.E., 1980. Variation of intra- and inter-particle porosity with degree of compaction. Analyst 105, 1104-1112; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86) or mercury porosimetry (e.g. Stanley-Wood, N.G., Johansson, M.E., 1980. Variation of intra- and inter-particle porosity with degree of compaction. Analyst 105, 1104-1112; Juppo, A.M., 1996. Relationship between breaking force and pore structure of lactose, glucose and mannitol tablets. Int. J. Pharm.127, 95-102; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86). These techniques are complementary, in that mercury porosimetry can be used to measure larger pores (the lower size limit is about 0.003 µm in diameter) while gas adsorption allows measurement of smaller pores. For further details it is also referred to S. Lowell et al., Characterization of Porous Solids and Powders: Surface Area, Pore Size and Density (Particle Technology Series), Springer, 2010.

In a preferred embodiment, the administration unit according to the invention has a pore size distribution such that at least 10 % of the pores have a pore size of not more than 0.1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 0.1 µm.

In another preferred embodiment, the administration unit according to the invention has a pore size distribution such that at least 10 % of the pores have a pore size of not less than 0.1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 0.1 µm.

In preferred embodiment, the administration unit according to the invention has a pore size distribution such that at least 10 % of the pores have a pore size of not more than 1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 1 µm.

In another preferred embodiment, the administration unit according to the invention has a pore size distribution such that at least 10 % of the pores have a pore size of not less than 1 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 1 µm.

In a preferred embodiment, the administration unit according to the invention has a pore size distribution such that at least 10 % of the pores have a pore size of not more than 10 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 10 µm.

In another preferred embodiment, the administration unit according to the invention has a pore size distribution such that at least 10 % of the pores have a pore size of not less than 10 µm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 10 µm.

In a preferred embodiment, the administration unit according to the invention has a water content of not more than 5 % by weight, relative to the total weight of the tablet. Preferably, it has a water content of not more than 4 % by weight, or not more than 3 % by weight, or not more than 2 % by weight, or not more than 1 % by weight, or not more than 0.5 % by weight, or not more than 0.1 % by weight, relative to the total weight of the tablet.

In a preferred embodiment, the administration unit according to the invention has a true density of 0.60 to 1.40 g cm-3. Preferably, it has a true density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit according to the invention has an apparent density of 0.60 to 1.40 g cm-3. Preferably, it has an apparent density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit according to the invention disintegrates within 6000 seconds. Preferably, it disintegrates within 5500 seconds, or within 5000 seconds, or within 4500 seconds, or within 4000 seconds, or within 3500 seconds, or within 3000 seconds, or within 2500 seconds, or within 2000 seconds, or within 1500 seconds, or within 1000 seconds, or within 750 seconds, or within 500 seconds, or within 250 seconds.

In a preferred embodiment, the administration unit according to the invention is a capsule. Preferably, it comprises a multitude of particulates comprising (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}. Preferably, it comprises at least 2 particulates, or at least 3 particulates, or at least 4 particulates, or at least 5 particulates, or at least 6 particulates, or at least 7 particulates, or at least 8 particulates, or at least 9 particulates, or at least 10 particulates.

In a preferred embodiment of the administration unit according to the invention, the capsule material comprises hard gelatine. In a preferred embodiment, the administration unit according to the invention comprises at least 2 different types of particulates that differ from each other in at least one property selected from the group consisting of nature of excipients, content of excipients, content of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; content of (v) {drug5}, (vii) {drug7}, (viii) {drug8}, (ix) {drug9}, or (xi) {drug11}, respectively , water content, total weight, size, and shape.

In a preferred embodiment, the administration unit according to the invention passes the storage stability test according to Ph. Eur. under accelerated storage conditions at 40°C and 75 % rel. humidity. Preferably, it has a storage stability for at least 3 months, or at least 4 months, or at least 5 months, or at least 6 months, or at least 7 months, or at least 8 months, or at least 9 months, or at least 10 months, or at least 11 months, or at least 12 months according to Ph. Eur. under accelerated storage conditions at 40°C and 75 % rel. humidity.

In a preferred embodiment, the administration unit according to the invention provides the peak plasma level within 0.1 hour to 36 hours after administration. Preferably, it provides the peak plasma level within 0.1 hour to 1 hour, or 1 hour to 2 hours, or 2 hours to 3 hours, or 3 hours to 4 hours, or 4 hours to 5 hours, or 5 hours to 6 hours, or 6 hours to 7 hours, or 7 hours to 8 hours, or 8 hours to 9 hours, or 9 hours to 10 hours, or 10 hours to 11 hours, or 11 hours to 12 hours, or 12 hours to 13 hours, or 13 hours to 14 hours, or 14 hours to 15 hours, or 15 hours to 16 hours, or 16 hours to 17 hours, or 17 hours to 18 hours, or 18 hours to 19 hours, or 19 hours to 20 hours, or 20 hours to 21 hours, or 21 hours to 22 hours, or 22 hours to 23 hours, or 23 hours to 24 hours, after administration.

Preferably, the administration unit according to the invention comprises one or more excipients independently selected from the group consisting of antiadherents, binders, disintegrants, fillers, diluents, flavors, colors, lubricants, glidants, sorbents, surfactants, preservatives and sweeteners.

In a preferred embodiment of the administration unit according to the invention, the antiadherent is selected from the group consisting of silicon dioxide, talc, talc waxes, stearates (e.g. magnesium, calcium and sodium), stearic acid, stearowet, boric acid, sodium chloride, DL-leucine, sodium oleate, sodium benzoate, sodium acetate, sodium lauryl sulfate, and magnesium lauryl sulfate. Magnesium stearate is particularly preferred.

In a preferred embodiment of the administration unit according to the invention, the binder is selected from the group consisting of saccharides and their derivatives; proteins; gums; silicates; and synthetic polymers. Preferably, the binder is a saccharide or a derivative thereof selected from the group consisting of disaccharides (e.g. sucrose, glucose or lactose); polysaccharides and their derivatives (e.g. starch, pregelatinized starch, cellulose, microcrystalline cellulose, polysaccharide acids, cellulose ethers [e.g. methyl cellulose, ethyl cellulose, hydroxyethyl cellulose, hydroxypropyl cellulose, hydroxypropylmethyl cellulose or carboxymethylcellulose sodium]); alginic acid and salts thereof (e.g. sodium alginate); and sugar alcohols (e.g. xylitol, sorbitol or maltitol); or wherein the binder is a protein selected from gelatin; or wherein the binder is a gum (e.g. acacia gum, guar gum, or tragacanth gum); or wherein the binder is a silicate (e.g. magnesium aluminum silicate or bentonite); or wherein the binder is a synthetic polymer selected from the group consisting of polyvinylpyrrolidone, polyvinylpyrrolidone-vinylacetate copolymer, polymethacrylate polymer, polyvinylalcohol, and polyethylene glycol.

In a preferred embodiment of the administration unit according to the invention, the disintegrant is selected from the group consisting of cross-linked polymers (e.g. cross-linked polyvinylpyrrolidone [crospovidone] or cross-linked sodium carboxymethyl cellulose [croscarmellose sodium]), starches (e.g. corn or potato), pregelatinized starch, modified starch (e.g. sodium starch glycolate), algenic acid, alginates (e.g. sodium alginate), carboxymethylcellulose, microcrystalline cellulose, clays (e.g. magnesium aluminum silicate), and gums (e.g. agar, guar, locust bean, karaya, pectin, or tragacanth gum).

In a preferred embodiment of the administration unit according to the invention, the filler (or diluent) is selected from the group consisting of plant cellulose, microcrystalline cellulose, inorganic phosphates (e.g. dibasic calcium phosphate or tricalcium phosphate), inorganic carbonates such as calcium carbonate, inorganic sulfates such as calcium sulfate dihydrate, sugars and sugar alcohols (e.g. lactose, glucose, inositol, mannitol, sorbitol, sucrose, dextrose, maltodextrins, and fructose), magnesium stearate, calcium lactate trihydrate, starch (e.g. corn, wheat, maize, potato or rice starch), and modified starch (e.g. carboxymethyl starch).

In a preferred embodiment of the administration unit according to the invention, the lubricant is hydrophilic or hydrophobic. Preferably, the lubricant is selected from the group consisting of talc, silica, stearin, fatty acids (e.g. stearic acid) or fatty acid derivatives, metal stearates (e.g. magnesium stearate, calcium stearate, or zinc stearate), glyceryl monostearate, glyceryl palmitostearate, glyceryl behenate, sodium lauryl sulfate, magnesium lauryl sulfate, sodium stearyl fumarate, hydrogenated vegetable oil, polyalkylene glycols (e.g. polyethylene glycol), starch, light mineral oil, sodium benzoate, and sodium chloride.

In a preferred embodiment of the administration unit according to the invention, the glidant is selected from the group consisting of fumed silica, cornstarch, talc, and magnesium carbonate.

In a preferred embodiment of the administration unit according to the invention, the surfactant is selected from the group consisting of sodium lauryl sulfate, polyethylene-polypropylene glycol co-polymers, poly(oxyethylene)-poly(oxypropylene)-block-copolymers (e.g. poloxamers).

In a preferred embodiment of the administration unit according to the invention, the preservative is selected from the group consisting of antioxidants (e.g. vitamin A, vitamin C, vitamin E, retinyl palmitate, selenium, ascorbyl palmitate, sodium ascorbate), amino acids (e.g. cysteine or methionine), citric acid and its salts (e.g. sodium citrate), synthetic preservatives (e.g. parabens such as methyl paraben or propyl paraben; butylated hydroxyanisole, butylated hydroxytoluene, monothioglycerol, propyl gallate), hypophosphorous acid, sodium bisulfite, sodium formaldehyde sulfoxylate, and sodium metabisulfite.

In a preferred embodiment, the administration unit according to the invention passes the friability test according to Ph. Eur. Preferably, the weight loss in the friability test according to Ph. Eur. is not more than 2.0 % by weight, or not more than 1.5 % by weight, or not more than 1.0 % by weight, or not more than 0.5 % by weight, or not more than 0.4 % by weight, or not more than 0.3 % by weight, or not more than 0.2 % by weight, or not more than 0.1 % by weight.

In a preferred embodiment, the administration unit according to the invention passes the test uniformity of content of single-dose preparations according to Ph. Eur.

In a preferred embodiment, the administration unit according to the invention does not comprise any active pharmaceutical ingredient other than (v) {drug5}, (vii) {drug7}, (viii) {drug8}, (ix) {drug9}, or (xi) {drug11}, respectively.

In a preferred embodiment, the administration unit according to the invention is for storage not above 50 °C. Preferably, it is for storage not above 25 °C.

In a preferred embodiment, the administration unit according to the invention is not identical with any of the administration units that are individualized in the experimental section of WO 2012/164286, WO 2012/166420, WO 2013/001294, WO 2013/003249, WO 2013/003386, WO 2012/172449, WO 2013/008002, WO 2013/007518, WO 2013/007519, WO 2013/012909, WO 2013/013114, WO 2013/011402, WO 2013/012118, WO 2013/016155, and WO 2013/016156, which is incorporated by reference.

The invention also relates to the administration unit according to the invention for use in the treatment of a disorder or a disease in a mammal. Preferably, the mammal is a human. Preferably, the mammal is an adult.

Preferably, the mammal does not suffer from liver damage and/or kidney damage.

Preferably, the mammal is not pregnant.

Preferably, the mammal is not allergic against (v) {drug5}, (vii) {drug7}, (viii) {drug8}, (ix) {drug9}, or (xi) {drug11}, respectively.

In a preferred embodiment, the administration unit according to the invention is administered orally with a liquid. Preferably, the liquid is water, milk, juice or lemonade.

In a preferred embodiment of the administration unit according to the invention, the administered daily dose of (v) {drug5}, (vii) {drug7}, (viii) {drug8}, (ix) {drug9}, or (xi) {drug11}, respectively is 0.0001 mg/kg body weight to 100 mg/kg body weight. Preferably, the administered daily dose of (v) {drug5}, (vii) {drug7}, (viii) {drug8}, (ix) {drug9}, or (xi) {drug11}, respectively is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.

In a preferred embodiment of the administration unit according to the invention, the administered daily dose of (v) {drug5}, (vii) {drug7}, (viii) {drug8}, (ix) {drug9}, or (xi) {drug11}, respectively is 0.001 mg to 5000 mg. Preferably, the administered daily dose of (v) {drug5}, (vii) {drug7}, (viii) {drug8}, (ix) {drug9}, or (xi) {drug11}, respectively is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.

The invention also relates to a packaging comprising one or more administration units according to the invention.

In a preferred embodiment, the packaging according to the invention comprises a material selected from the group consisting of paper, cardboard, paperboard, metal foil and plastic foil.

In a preferred embodiment, the packaging according to the invention is a blister packaging.

A preferred blister packaging includes but is not limited to PVC-blister, PVDC-blister, PVC/PVDC-blister, moisture-proof packaging material such as aluminium foil blister pack, alu/alu blister, transparent or opaque polymer blister with pouch.

An alternative packaging according to the invention is a polypropylene tube, glass bottle and HDPE bottle optionally containing a child-resistant feature. The primary packaging material may comprise a desiccant such as molecular sieve or silica gel to improve chemical stability of (v) {drug5}, (vii) {drug7}, (viii) {drug8}, (ix) {drug9}, or (xi) {drug11}, respectively. Opaque packaging such as colored blister materials, tubes, brown glass bottles or the like can be used to prolong shelflife of (v) {drug5}, (vii) {drug7}, (viii) {drug8}, (ix) {drug9}, or (xi) {drug11}, respectively by reduction of photodegradation.

In a preferred embodiment, the packaging according to the invention comprises a multitude of at least 2, or at least 3, or at least 4, or at least 5, or at least 6, or at least 7, or at least 8, or at least 9, or at least 10 administration units according to the invention.

In a preferred embodiment of the packaging according to the invention, the administration units do not substantially differ in at least one property selected from the group consisting of nature of excipients, content of excipients, content of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; content of (v) {drug5}, (vii) {drug7}, (viii) {drug8}, (ix) {drug9}, or (xi) {drug11}, respectively , water content, total weight, release profile, disintegration time, size, shape, porosity, apparent density, true density, average pore size, pore size distribution, axial tensile strength, radial tensile strength, friability, and storage stability. Preferably, the administration units differ in said at least one property by not more than 5 %, or not more than 4 %, or not more than 3 %, or not more than 2 %, or not more than 1 %, or not more than 0.5 %, or not more than 0.1 %. Preferably, all administration units are substantially identical.

The invention also relates to the packaging according to the invention for use in the treatment of a disorder or a disease, wherein one or more administration units of said multitude are administered on a first day, and one or more administration units of said multitude are administered on a second day following said first day.

In a preferred embodiment of the packaging according to the invention, one or more administration units of said multitude are administered on one or more subsequent days following said second day. Preferably, the time interval between the administration of an administration unit and the administration of a subsequent administration unit is not more than 48 hours. Preferably, the time interval is not more than 36 hours, or not more than 24 hours, or not more than 12 hours, or not more than 8 hours, or not more than 6 hours, or not more than 4 hours.

In a preferred embodiment of the packaging according to the invention, the administration units are administered on not more than 30 subsequent days. Preferably, the administration units are administered on not more than 25 subsequent days, or on not more than 20 subsequent days, or on not more than 14 subsequent days, or on not more than 10 subsequent days, or on not more than 7 subsequent days, or on not more than 3 subsequent days.

In a preferred embodiment of the packaging according to the invention, the administered daily dose of (v) {drug5}, (vii) {drug7}, (viii) {drug8}, (ix) {drug9}, or (xi) {drug11}, respectively is 0.0001 mg/kg body weight to 100 mg/kg body weight. Preferably, the administered daily dose of (v) {drug5}, (vii) {drug7}, (viii) {drug8}, (ix) {drug9}, or (xi) {drug11}, respectively is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.

In a preferred embodiment of the packaging according to the invention, the administered daily dose of (v) {drug5}, (vii) {drug7}, (viii) {drug8}, (ix) {drug9}, or (xi) {drug11}, respectively is 0.001 mg to 5000 mg. Preferably, the administered daily dose of (v) {drug5}, (vii) {drug7}, (viii) {drug8}, (ix) {drug9}, or (xi) {drug11}, respectively is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.

In a preferred embodiment, the packaging according to the invention does not comprise any administration unit comprising an active pharmaceutical ingredient other than (v) {drug5}, (vii) {drug7}, (viii) {drug8}, (ix) {drug9}, or (xi) {drug11}, respectively.

The invention also relates to a method for manufacturing an administration unit according to the invention or a packaging according to the invention, comprising the steps:
(a) providing a quantity of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; that exceeds the dose of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; to be contained in a single administration unit by a factor of at least 10;
(b) mixing the quantity of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; with one or more pharmaceutical excipients;
(c) dividing the mixture obtained in step (b) in fractions each containing the dose of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; to be contained in a single administration unit;
(d) optionally, forming the fractions obtained in step (c) to administration units; and
(e) optionally, packing the fractions obtained in step (c) and the administration units obtained in step (d), respectively. In a preferred embodiment of the method according to the invention, in step (a) the quantity of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; exceeds the dose of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; to be contained in a single administration unit by a factor of at least 25, or at least 50, or at least 100, or at least 250, or at least 500, or at least 1000, or at least 2500, or at least 5000, or at least 10,000.

The invention also relates to particles of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; having a particle size distribution X90 of about 500 µm or less, i.e. a particle size distribution (preferably by volume) such that at least 90 % of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; has a particle size smaller than 500 um. Preferably, for (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; the particle size distribution of X90 is about 480 µm or less, or about 460 µm or less, or about 440 µm or less, or about 420 µm or less, or about 400 µm or less, or about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 60 µm or less, or about 40 µm or less, or about 20 µm or less.

In a preferred embodiment, the particles of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; according to the invention have a particle size distribution X50 of about 400 µm or less, i.e. a particle size distribution (preferably by volume) such that at least 50 % of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; has a particle size smaller than 400 µm. Preferably, for (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; the particle size distribution of X50 is about 380 µm or less, or about 360 µm or less, or about 340 µm or less, or about 320 µm or less, or about 300 µm or less, or about 280 µm or less, or about 260 µm or less, or about 240 µm or less, or about 220 µm or less, or about 200 µm or less, or about 180 µm or less, or about 160 µm or less, or about 140 µm or less, or about 120 µm or less, or about 100 µm or less, or about 80 µm or less, or about 70 µm or less, or about 60 µm or less, or about 50 µm or less. Preferably, for (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; the particle size distribution of X50 is about 45 µm or less, or about 40 µm or less, or about 35 µm or less, or about 30 µm or less, or about 25 µm or less, or about 20 µm or less, or about 15 µm or less, or about 10 µm or less.

In preferred embodiments, the particles of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; have a cumulative distribution by volume basis Q3(X) (X in µm) of Q3(355) <0.50; or Q3(180) <0.50 and Q3(355) ≥0.50; or Q3(125) <0.50 and Q3(180) ≥0.50; or Q3(125) ≥0.50.

In preferred embodiments, the particles of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; have a cumulative distribution by volume basis Q3(X) (X in µm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.

In preferred embodiments, the particles of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; have a cumulative distribution by volume basis Q3(X) (X in µm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

The indicated particle size properties are determined by laser-diffraction method, in particular low angle laser light scattering, i.e. Fraunhofer diffraction. Alternatively, the particle size properties can be also determined by microscopy (e.g. electron microscopy or scanning electron microscopy). The powder fineness is preferably determined in accordance with USP35 <811>. The results of the particle size distribution determined by different techniques can be correlated with one another.

Suitable milling and grinding techniques that are suitable for obtaining a specific particle size distribution are known to the skilled artisan (see e.g. N. Rasenack, B.W. Müller, Micron-size drug particles: common and novel micronization techniques, Pharm Dev Technol., 2004, 9(1):1-13; A. Martin, M.J. Cocero, Micronization processes with supercritical fluids: fundamentals and mechanisms, Adv Drug Deliv Rev. 2008, 60(3):339-50; S.C. Gad, Pharmaceutical Manufacturing Handbook: Production and Processes (Pharmaceutical Development Series), Wiley Interscience, 1st ed. 2008; A.J. Hickey, Pharmaceutical Process Engineering (Drugs and the Pharmaceutical Sciences), Informa Healthcare, 2nd ed. 2009; B. Nickerson, Sample Preparation of Pharmaceutical Dosage Forms: Challenges and Strategies for Sample Preparation and Extraction, Springer, 1st ed. 2011; D. Dragoman, Optical Characteriaztion of Solids, Springer, 1st ed. 2010; and D. Schulze; Powders and Bulk Solids: Behavior, Characterization, Storage and Flow, Springer, 2008).

### PREFERRED EMBODIMENTS OF THE INVENTION ACCORDING TO ITEMS 1 TO 137

1. An administration unit comprising (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.
2. The administration unit according to item 1, comprising a therapeutically effective amount of (v) {drug5}, (vii) {drug7}, (viii) {drug8}, (ix) {drug9}, or (xi) {drug11}, respectively , wherein at least 10 % by weight of said therapeutically effective amount of (v) {drug5}, (vii) {drug7}, (viii) {drug8}, (ix) {drug9}, or (xi) {drug11}, respectively are present as (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.
3. The administration unit according to item 2, wherein at least 20 % by weight, or at least 30 % by weight, or at least 40 % by weight, or at least 50 % by weight, or at least 60 % by weight, or at least 70 % by weight, or at least 80 % by weight, or at least 90 % by weight of said therapeutically effective amount of (v) {drug5}, (vii) {drug7}, (viii) {drug8}, (ix) {drug9}, or (xi) {drug11}, respectively are present as (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.
4. The administration unit according to any of items 2 to 3, wherein at least 95 % by weight, or at least 96 % by weight, or at least 97 % by weight, or at least 98 % by weight, or at least 99 % by weight, or at least 99.5 % by weight, or at least 99.8 % by weight, or at least 99.9 % by weight of said therapeutically effective amount of (v) {drug5}, (vii) {drug7}, (viii) {drug8}, (ix) {drug9}, or (xi) {drug11}, respectively are present as (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.
5. The administration unit according to any of items 2 to 4, wherein not more than 90 % by weight of said therapeutically effective amount of (v) {drug5}, (vii) {drug7}, (viii) {drug8}, (ix) {drug9}, or (xi) {drug11}, respectively are amorphous.
6. The administration unit according to item 5, wherein not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (v) {drug5}, (vii) {drug7}, (viii) {drug8}, (ix) {drug9}, or (xi) {drug11}, respectively are amorphous.
7. The administration unit according to item 5 or 6, wherein not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (v) {drug5}, (vii) {drug7}, (viii) {drug8}, (ix) {drug9}, or (xi) {drug11}, respectively are amorphous.
8. The administration unit according to any of items 2 to 7, wherein not more than 90 % by weight of said therapeutically effective amount of (v) {drug5}, (vii) {drug7}, (viii) {drug8}, (ix) {drug9}, or (xi) {drug11}, respectively are polymorphs of (v) {drug5}, (vii) {drug7}, (viii) {drug8}, (ix) {drug9}, or (xi) {drug11}, respectively other than (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.
9. The administration unit according to item 8, wherein not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (v) {drug5}, (vii) {drug7}, (viii) {drug8}, (ix) {drug9}, or (xi) {drug11}, respectively are polymorphs of (v) {drug5}, (vii) {drug7}, (viii) {drug8}, (ix) {drug9}, or (xi) {drug11}, respectively other than (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.
10. The administration unit according to item 8 or 9, wherein not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (v) {drug5}, (vii) {drug7}, (viii) {drug8}, (ix) {drug9}, or (xi) {drug11}, respectively are polymorphs of (v) {drug5}, (vii) {drug7}, (viii) {drug8}, (ix) {drug9}, or (xi) {drug11}, respectively other than (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.
11. The administration unit according to any of items 2 to 10, wherein the therapeutically effective amount of (v) {drug5}, (vii) {drug7}, (viii) {drug8}, (ix) {drug9}, or (xi) {drug11}, respectively is 0.1 µg to 2 g of (v) {drug5}, (vii) {drug7}, (viii) {drug8}, (ix) {drug9}, or (xi) {drug11}, respectively.
12. The administration unit according to item 11, wherein the therapeutically effective amount of (v) {drug5}, (vii) {drug7}, (viii) {drug8}, (ix) {drug9}, or (xi) {drug11}, respectively is 1 µg to 2.5 µg of (v) {drug5}, (vii) {drug7}, (viii) {drug8}, (ix) {drug9}, or (xi) {drug11}, respectively , or 2.5 µg to 5 µg of (v) {drug5}, (vii) {drug7}, (viii) {drug8}, (ix) {drug9}, or (xi) {drug11}, respectively , or 5 µg to 10 µg of (v) {drug5}, (vii) {drug7}, (viii) {drug8}, (ix) {drug9}, or (xi) {drug11}, respectively , or 10 µg to 25 µg of (v) {drug5}, (vii) {drug7}, (viii) {drug8}, (ix) {drug9}, or (xi) {drug11}, respectively , or 25 µg to 50 µg of (v) {drug5}, (vii) {drug7}, (viii) {drug8}, (ix) {drug9}, or (xi) {drug11}, respectively , or 50 µg to 100 µg of (v) {drug5}, (vii) {drug7}, (viii) {drug8}, (ix) {drug9}, or (xi) {drug11}, respectively , or 100 µg to 250 µg of (v) {drug5}, (vii) {drug7}, (viii) {drug8}, (ix) {drug9}, or (xi) {drug11}, respectively , or 250 µg to 500 µg of (v) {drug5}, (vii) {drug7}, (viii) {drug8}, (ix) {drug9}, or (xi) {drug11}, respectively , or 500 µg to 1 mg of (v) {drug5}, (vii) {drug7}, (viii) {drug8}, (ix) {drug9}, or (xi) {drug11}, respectively , or 1 mg to 2.5 mg of (v) {drug5}, (vii) {drug7}, (viii) {drug8}, (ix) {drug9}, or (xi) {drug11}, respectively , or 2.5 mg to 5 mg of (v) {drug5}, (vii) {drug7}, (viii) {drug8}, (ix) {drug9}, or (xi) {drug11}, respectively , or 5 mg to 10 mg of (v) {drug5}, (vii) {drug7}, (viii) {drug8}, (ix) {drug9}, or (xi) {drug11}, respectively , or 10 mg to 25 mg of (v) {drug5}, (vii) {drug7}, (viii) {drug8}, (ix) {drug9}, or (xi) {drug11}, respectively , or 25 mg to 50 mg of (v) {drug5}, (vii) {drug7}, (viii) {drug8}, (ix) {drug9}, or (xi) {drug11}, respectively , or 50 mg to 100 mg of (v) {drug5}, (vii) {drug7}, (viii) {drug8}, (ix) {drug9}, or (xi) {drug11}, respectively , or 100 mg to 250 mg of (v) {drug5}, (vii) {drug7}, (viii) {drug8}, (ix) {drug9}, or (xi) {drug11}, respectively , or 250 mg to 500 mg of (v) {drug5}, (vii) {drug7}, (viii) {drug8}, (ix) {drug9}, or (xi) {drug11}, respectively , or 500 mg to 1 g of (v) {drug5}, (vii) {drug7}, (viii) {drug8}, (ix) {drug9}, or (xi) {drug11}, respectively , or 1 g to 1.25 g of (v) {drug5}, (vii) {drug7}, (viii) {drug8}, (ix) {drug9}, or (xi) {drug11}, respectively , or 1.25 g to 1.5 g of (v) {drug5}, (vii) {drug7}, (viii) {drug8}, (ix) {drug9}, or (xi) {drug11}, respectively , or 1.5 g to 1.75 g of (v) {drug5}, (vii) {drug7}, (viii) {drug8}, (ix) {drug9}, or (xi) {drug11}, respectively , or 1.75 g to 2 g of (v) {drug5}, (vii) {drug7}, (viii) {drug8}, (ix) {drug9}, or (xi) {drug11}, respectively.
13. The administration unit according to any of items 1 to 12, comprising 0.1 µg to 2 g of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.
14. The administration unit according to item 13, comprising 1 µg to 2.5 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 2.5 µg to 5 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug01b}, {drug11c}, or {drug11d}; or 5 µg to 10 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {dmg11d}; or 10 µg to 25 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 25 µg to 50 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 50 µg to 100 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 100 µg to 250 µg of(v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 250 µg to 500 µg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 500 µg to 1 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 1 mg to 2.5 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 2.5 mg to 5 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 5 mgto 10 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 10 mg to 25 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 25 mg to 50 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drag11a}, {drug11b}, {drug11c}, or {drug11d}; or 50 mg to 100 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 100 mg to 250 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 250 mg to 500 mg of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 500 mg to 1 g of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 1 g to 1.25 g of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 1.25 g to 1.5 g of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 1.5 g to 1.75 g of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; or 1.75 g to 2 g of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.
15. The administration unit according to any of items 1 to 14, which is solid, semisolid or liquid.
16. The administration unit according to any of items 1 to 15, which is for administration once daily, or twice daily, or thrice daily, or four times daily, or five times daily, or six times daily.
17. The administration unit according to any of items 1 to 16, which is monolithic or multiparticulate.
18. The administration unit according to any of items 1 to 17, wherein (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; is homogeneously distributed over the administration unit.
19. The administration unit according to any of items 1 to 17, wherein (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; is not homogeneously distributed over the administration unit.
20. The administration unit according to any of items 1 to 19, providing controlled release of (v) {drug5}, (vii) {drug7}, (viii) {drug8}, (ix) {drug9}, or (xi) {drug11}, respectively.
21. The administration unit according to item 20, comprising a controlled release matrix or a controlled release coating.
22. The administration unit according to any of items 1 to 19, providing immediate release of (v) {drug5}, (vii) {drug7}, (viii) {drug8}, (ix) {drug9}, or (xi) {drug11}, respectively.
23. The administration unit according to any of items 1 to 22, having a total weight of 10 mg to 3 g.
24. The administration unit according to item 23, having a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.
25. The administration unit according to any of items 1 to 24, which is for systemic or topical administration.
26. The administration unit according to any of items 1 to 25, which is for parenteral administration.
27. The administration unit according to any of items 1 to 26, which is for oral, buccal, opthalmic, nasal, rectal, transmucosal, or intestinal administration.
28. The administration unit according to any of items 1 to 27, which is for oral administration.
29. The administration unit according to item 28, which is selected from the group consisting of tablets, capsules, effervescent tablets, orodispersible tablets, dragees, sachets, drops, suspensions, and powders.
30. The administration unit according to any of items 1 to 29, which is a tablet.
31. The administration unit according to item 30, which is round or oblong; and/or which is planar or biconvex.
32. The administration unit according to any of items 30 to 31, having been manufactured by direct compression.
33. The administration unit according to any of items 30 to 31, having been manufactured by granulation and subsequent compression of granules.
34. The administration unit according to item 33, wherein granulation is wet granulation or dry granulation.
35. The administration unit according to any of items 30 to 34, having been compacted at a pressure of 10 MPa to 10,000 MPa.
36. The administration unit according to item 35, having been compacted at a pressure of 10 MPa to 25 MPa, or 25 MPa to 50 MPa, or 50 MPa to 100 MPa, or 100 MPa to 250 MPa, or 250 MPa to 500 MPa, or 500 MPa to 1000 MPa, or 1000 MPa to 2500 MPa, or 2500 MPa to 5000 MPa, or 5000 MPa to 10,000 MPa.
37. The administration unit according to any of items 30 to 36, comprising a film coating.
38. The administration unit according to item 37, wherein the film coating is enteric.
39. The administration unit according to any of items 37 to 38, wherein the film coating comprises a cellulose ether (e.g. hydroxypropylmethyl cellulose) or a synthetic polymer (e.g. polyvinyl alcohol).
40. The administration unit according to any of items 37 to 39, wherein the film coating has an average thickness of 0.01 to 1000 µm.
41. The administration unit according to item 40, wherein the film coating has an average thickness of 0.01 µm to 0.03 µm, or 0.03 to 0.05 µm, or 0.05 to 0.1 µm, or 0.1 to 0.25 µm, or 0.25 to 0.5 µm, or 0.5 to 1.0 µm, or 1.0 µm to 2.5 µm, or 2.5 µm to 5.0 µm, or 5.0 µm to 10 µm, or 10 µm to 25 µm, or 25 µm to 50 µm, or 50 µm to 100 µm, or 100 µm to 250 µm, or 250 µm to 500 µm, or 500 µm to 1000 µm.
42. The administration unit according to any of items 30 to 41, having a tablet porosity of not more than 90 %.
43. The administration unit according to item 42, having a tablet porosity of not more than 80 %, or not more than 70 %, or not more than 60 %, or not more than 50 %, or not more than 40 %, or not more than 30 %, or not more than 20 %, or not more than 10 %.
44. The administration unit according to item 42 to 43, having a tablet porosity of not more than 5 %, or not more than 4
   %, or not more than 3 %, or not more than 2 %, or not more than 1 %, or not more than 0.5 %, or not more than 0.2 %, or not more than 0.1 %.
45. The administration unit according to any of items 30 to 44, having a radial tensile strength of 0.1 to 100 MPa.
46. The administration unit according to item 45, having a radial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.
47. The administration unit according to any of items 30 to 46, having an axial tensile strength of 0.1 to 100 MPa.
48. The administration unit according to item 47, having an axial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.
49. The administration unit according to any of items 30 to 48, having an average pore size of 0.001 µm to 1000 µm.
50. The administration unit according to item 49, having an average pore size of 0.001 µm to 0.003 µm, or 0,003 µm to 0.005 µm, or 0.005 µm to 0.01 µm, or 0.01 µm to 0.025 µm, or 0.025 µm to 0.05 µm, or 0.05 µm to 0.1 µm, or 0.1 µm to 0.25 µm, or 0.25 µm to 0.5 µm, or 0.5 µm to 1 um, or 1 µm to 2.5 µm, or 2.5 µm to 5 µm, or 5 µm to 10 µm, or 10 µm to 25 µm, or 25 µm to 50 µm, or 50 µm to 100 µm, or 100 µm to 250 µm, or 250 µm to 500 µm, or 500 µm to 1000 µm.
51. The administration unit according to any of items 30 to 50, having a pore size distribution such that at least 10 % of the pores have a pore size of not more than 0.1 µm.
52. The administration unit according to item 51, having a pore size distribution such that at least 20 %, or at least 30 %, or at least 40 %, or at least 50 %, or at least 60 %, or at least 70 %, or at least 80 %, or at least 90 % of the pores have a pore size of not more than 0.1 µm.
53. The administration unit according to any of items 30 to 52, having a pore size distribution such that at least 10 % of the pores have a pore size of not less than 0.1 µm.
54. The administration unit according to item 53, having a pore size distribution such that at least 20 %, or at least 30 %, or at least 40 %, or at least 50 %, or at least 60 %, or at least 70 %, or at least 80 %, or at least 90 % of the pores have a pore size of not less than 0.1 µm.
55. The administration unit according to any of items 30 to 54, having a pore size distribution such that at least 10 % of the pores have a pore size of not more than 1 µm.
56. The administration unit according to item 55, having a pore size distribution such that at least 20 %, or at least 30 %, or at least 40 %, or at least 50 %, or at least 60 %, or at least 70 %, or at least 80 %, or at least 90 % of the pores have a pore size of not more than 1 µm.
57. The administration unit according to any of items 30 to 56, having a pore size distribution such that at least 10 % of the pores have a pore size of not less than 1 µm.
58. The administration unit according to item 57, having a pore size distribution such that at least 20 %, or at least 30 %, or at least 40 %, or at least 50 %, or at least 60 %, or at least 70 %, or at least 80 %, or at least 90 % of the pores have a pore size of not less than 1 µm.
59. The administration unit according to any of items 30 to 58, having a pore size distribution such that at least 10 % of the pores have a pore size of not more than 10 µm.
60. The administration unit according to item 59, having a pore size distribution such that at least 20 %, or at least 30 %, or at least 40 %, or at least 50 %, or at least 60 %, or at least 70 %, or at least 80 %, or at least 90 % of the pores have a pore size of not more than 10 µm.
61. The administration unit according to any of items 30 to 60, having a pore size distribution such that at least 10 % of the pores have a pore size of not less than 10 µm.
62. The administration unit according to item 61, having a pore size distribution such that at least 20 %, or at least 30 %, or at least 40 %, or at least 50 %, or at least 60 %, or at least 70 %, or at least 80 %, or at least 90 % of the pores have a pore size of not less than 10 µm.
63. The administration unit according to any of items 30 to 62, having a water content of not more than 5 % by weight, relative to the total weight of the tablet.
64. The administration unit according to item 63, having a water content of not more than 4 % by weight, or not more than 3 % by weight, or not more than 2 % by weight, or not more than 1 % by weight, or not more than 0.5 % by weight, or not more than 0.1 % by weight, relative to the total weight of the tablet.
65. The administration unit according to any of items 30 to 64, having a true density of 0.60 to 1.40 g cm-3.
66. The administration unit according to item 65, having a true density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.
67. The administration unit according to any of items 30 to 66, having an apparent density of 0.60 to 1.40 g cm-3.
68. The administration unit according to item 67, having an apparent density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.
69. The administration unit according to any of items 30 to 68, disintegrating within 6000 seconds.
70. The administration unit according to item 69, disintegrating within 5500 seconds, or within 5000 seconds, or within 4500 seconds, or within 4000 seconds, or within 3500 seconds, or within 3000 seconds, or within 2500 seconds, or within 2000 seconds, or within 1500 seconds, or within 1000 seconds, or within 750 seconds, or within 500 seconds, or within 250 seconds.
71. The administration unit according to any of items 1 to 29, which is a capsule.
72. The administration unit according to item 71, comprising a multitude of particulates comprising (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}.
73. The administration unit according to any of items 71 to 72, comprising at least 2 particulates, or at least 3 particulates, or at least 4 particulates, or at least 5 particulates, or at least 6 particulates, or at least 7 particulates, or at least 8 particulates, or at least 9 particulates, or at least 10 particulates.
74. The administration unit according to any of items 71 to 73, wherein the capsule material comprises hard gelatine.
75. The administration unit according to any of items 71 to 74, comprising at least 2 different types of particulates that differ from each other in at least one property selected from the group consisting of nature of excipients, content of excipients, content of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; content of (v) {drug5}, (vii) {drug7}, (viii) {drug8}, (ix) {drug9}, or (xi) {drug11}, respectively, water content, total weight, size, and shape.
76. The administration unit according to any of items 1 to 75, passing the storage stability test according to Ph. Eur. under accelerated storage conditions at 40°C and 75 % rel. humidity.
77. The administration unit according to item 76, having a storage stability for at least 3 months, or at least 4 months, or at least 5 months, or at least 6 months, or at least 7 months, or at least 8 months, or at least 9 months, or at least 10 months, or at least 11 months, or at least 12 months according to Ph. Eur. under accelerated storage conditions at 40°C and 75 % rel. humidity.
78. The administration unit according to any of items 1 to 77, providing the peak plasma level within 0.1 hour to 36 hours after administration.
79. The administration unit according to item 78, providing the peak plasma level within 0.1 hour to 1 hour, or 1 hour to 2 hours, or 2 hours to 3 hours, or 3 hours to 4 hours, or 4 hours to 5 hours, or 5 hours to 6 hours, or 6 hours to 7 hours, or 7 hours to 8 hours, or 8 hours to 9 hours, or 9 hours to 10 hours, or 10 hours to 11 hours, or 11 hours to 12 hours, or 12 hours to 13 hours, or 13 hours to 14 hours, or 14 hours to 15 hours, or 15 hours to 16 hours, or 16 hours to 17 hours, or 17 hours to 18 hours, or 18 hours to 19 hours, or 19 hours to 20 hours, or 20 hours to 21 hours, or 21 hours to 22 hours, or 22 hours to 23 hours, or 23 hours to 24 hours, after administration.
80. The administration unit according to any of items 1 to 79, comprising one or more excipients independently selected from the group consisting of antiadherents, binders, disintegrants, fillers, flavors, colors, lubricants, glidants, sorbents, surfactants, preservatives and sweeteners.
81. The administration unit according to item 80, wherein the antiadherent is selected from the group consisting of silicon dioxide, talc, talc waxes, stearates (e.g. magnesium, calcium or sodium), stearic acid, stearowet, boric acid, sodium chloride, DL-leucine, sodium oleate, sodium benzoate, sodium acetate, sodium lauryl sulfate, and magnesium lauryl sulfate.
82. The administration unit according to any of items 80 to 81, wherein the binder is selected from the group consisting of saccharides and their derivatives; proteins; gums; silicates; and synthetic polymers.
83. The administration unit according to item 82, wherein the binder is a saccharide or a derivative thereof selected from the group consisting of disaccharides (e.g. sucrose, glucose or lactose); polysaccharides and their derivatives (e.g. starch, pregelatinized starch, cellulose, microcrystalline cellulose, polysaccharide acids, cellulose ethers [e.g. methyl cellulose, ethyl cellulose, hydroxyethyl cellulose, hydroxypropyl cellulose, hydroxypropylmethyl cellulose or carboxymethylcellulose sodium]); alginic acid and salts thereof (e.g. sodium alginate); and sugar alcohols (e.g. xylitol, sorbitol or maltitol); or wherein the binder is a protein selected from gelatin; or wherein the binder is a gum (e.g. acacia gum, guar gum, or tragacanth gum); or wherein the binder is a silicate (e.g. magnesium aluminum silicate or bentonite); or wherein the binder is a synthetic polymer selected from the group consisting of polyvinylpyrrolidone, polyvinylpyrrolidone-vinylacetate copolymer, polymethacrylate polymer, polyvinylalcohol, and polyethylene glycol.
84. The administration unit according to any of items 80 to 83, wherein the disintegrant is selected from the group consisting of cross-linked polymers (e.g. cross-linked polyvinylpyrrolidone [crospovidone] or cross-linked sodium carboxymethyl cellulose [croscarmellose sodium]), starches (e.g. corn or potato), pregelatinized starch, modified starch (e.g. sodium starch glycolate), algenic acid, alginates (e.g. sodium alginate), carboxymethylcellulose, microcrystalline cellulose, clays (e.g. magnesium aluminum silicate), and gums (e.g. agar, guar, locust bean, karaya, pectin, or tragacanth gum).
85. The administration unit according to any of items 80 to 84, wherein the filler is selected from the group consisting of plant cellulose, microcrystalline cellulose, inorganic phosphates (e.g. dibasic calcium phosphate or tricalcium phosphate), inorganic carbonates such as calcium carbonate, inorganic sulfates such as calcium sulfate dihydrate, sugars and sugar alcohols (e.g. lactose, glucose, inositol, mannitol, sorbitol, sucrose, dextrose, maltodextrins, and fructose), magnesium stearate, calcium lactate trihydrate, starch (e.g. corn, wheat, maize, potato or rice starch), and modified starch (e.g. carboxymethyl starch).
86. The administration unit according to any of items 80 to 85, wherein the lubricant is hydrophilic or hydrophobic.
87. The administration unit according to item 86, wherein the lubricant is selected from the group consisting of talc, silica, stearin, fatty acids (e.g. stearic acid) or fatty acid derivatives, metal stearates (e.g. magnesium stearate, calcium stearate, or zinc stearate), glyceryl monostearate, glyceryl palmitostearate, glyceryl behenate, sodium lauryl sulfate, magnesium lauryl sulfate, sodium stearyl fumarate, hydrogenated vegetable oil, polyalkylene glycols (e.g. polyethylene glycol), starch, light mineral oil, sodium benzoate, and sodium chloride.
88. The administration unit according to any of items 80 to 87, wherein the glidant is selected from the group consisting of fumed silica, cornstarch, talc, and magnesium carbonate.
89. The administration unit according to any of items 80 to 88, wherein the preservative is selected from the group consisting of antioxidants (e.g. vitamin A, vitamin C, vitamin E, retinyl palmitate, selenium, ascorbyl palmitate, sodium ascorbate), amino acids (e.g. cysteine or methionine), citric acid and its salts (e.g. sodium citrate), synthetic preservatives (e.g. parabens such as methyl paraben or propyl paraben; butylated hydroxyanisole, butylated hydroxytoluene, monothioglycerol, propyl gallate), hypophosphorous acid, sodium bisulfite, sodium formaldehyde sulfoxylate, and sodium metabisulfite.
90. The administration unit according to any of items 1 to 89, passing the friability test according to Ph. Eur.
91. The administration unit according to item 90, wherein the weight loss in the friability test according to Ph. Eur. is not more than 2.0 % by weight, or not more than 1.5 % by weight, or not more than 1.0 % by weight, or not more than 0.5 % by weight, or not more than 0.4 % by weight, or not more than 0.3 % by weight, or not more than 0.2 % by weight, or not more than 0.1 % by weight.
92. The administration unit according to any of items 1 to 91, passing the test uniformity of content of single-dose preparations according to Ph. Eur.
93. The administration unit according to any of items 1 to 92, not comprising any active pharmaceutical ingredient other than (v) {drug5}, (vii) {drug7}, (viii) {drug8}, (ix) {drug9}, or (xi) {drug11}, respectively.
94. The administration unit according to any of items 1 to 93, which is for storage not above 50 °C.
95. The administration unit according to item 94, which is for storage not above 25 °C.
96. The administration unit according to any of items 1 to 95, with the proviso that the administration unit is not identical with any of the administration units that are individualized in the experimental section of WO 2012/164286, WO 2012/166420, WO 2013/001294, WO 2013/003249, WO 2013/003386, WO 2012/172449, WO 2013/008002, WO 2013/007518, WO 2013/007519, WO 2013/012909, WO 2013/013114, WO 2013/011402, WO 2013/012118, WO 2013/016155, and WO 2013/016156.
97. The administration unit according to any of items 1 to 96 for use in the treatment of a disorder or a disease in a mammal.
98. The administration unit according to item 97, wherein the mammal is a human.
99. The administration unit according to any of items 97 to 98, wherein the mammal is an adult.
100. The administration unit according to any of items 97 to 99 wherein the mammal does not suffer from liver damage.
101. The administration unit according to any of items 97 to 100, wherein the mammal does not suffer from kidney damage.
102. The administration unit according to any of items 97 to 101, wherein the mammal is not pregnant.
103. The administration unit according to any of items 97 to 102, wherein the mammal is not allergic against (v) {drug5}, (vii) {drug7}, (viii) {drug8}, (ix) {drug9}, or (xi) {drug11}, respectively.
104. The administration unit according to any of items 97 to 103, which is administered orally with a liquid.
105. The administration unit according to item 104, wherein the liquid is water, milk, juice or lemonade.
106. The administration unit according to any of items 97 to 105, wherein the administered daily dose of (v) {drug5}, (vii) {drug7}, (viii) {drug8}, (ix) {drug9}, or (xi) {drug11}, respectively is 0.0001 mg/kg body weight to 100 mg/kg body weight.
107. The administration unit according to item 106, wherein the administered daily dose of (v) {drug5}, (vii) {drug7}, (viii) {drug8}, (ix) {drug9}, or (xi) {drug11}, respectively is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.
108. The administration unit according to any of items 97 to 107, wherein the administered daily dose of (v) {drug5}, (vii) {drug7}, (viii) {drug8}, (ix) {drug9}, or (xi) {drug11}, respectively is 0.001 mg to 5000 mg.
109. The administration unit according to item 108, wherein the administered daily dose of (v) {drug5}, (vii) {drug7}, (viii) {drug8}, (ix) {drug9}, or (xi) {drug11}, respectively is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 1999 mg.
110. A packaging comprising one or more administration units according to any of items 1 to 109.
111. The packaging according to item 110, comprising a material selected from the group consisting of paper, cardboard, paperboard, metal foil and plastic foil.
112. The packaging according to any of items 110 to 111, which is a blister packaging.
113. The packaging according to any of items 110 to 112, comprising a multitude of at least 2, or at least 3, or at least 4, or at least 5, or at least 6, or at least 7, or at least 8, or at least 9, or at least 10 administration units according to any of items 1 to 109.
114. The packaging according to item 113, wherein the administration units do not substantially differ in at least one property selected from the group consisting of nature of excipients, content of excipients, content of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; content of (v) {drug5}, (vii) {drug7}, (viii) {drug8}, (ix) {drug9}, or (xi) {drug11}, respectively , water content, total weight, release profile, disintegration time, size, shape, porosity, apparent density, true density, average pore size, pore size distribution, axial tensile strength, radial tensile strength, friability, and storage stability.
115. The packaging according to item 114, wherein the administration units differ in said at least one property by not more than 5 %, or not more than 4 %, or not more than 3 %, or not more than 2 %, or not more than 1 %, or not more than 0.5 %, or not more than 0.1 %.
116. The packaging according to any of items 113 to 115, wherein all administration units are substantially identical.
117. The packaging according to any of items 113 to 116 for use in the treatment of a disorder or a disease, wherein one or more administration units of said multitude are administered on a first day, and one or more administration units of said multitude are administered on a second day following said first day.
118. The packaging according to item 117, wherein one or more administration units of said multitude are administered on one or more subsequent days following said second day.
119. The packaging according to any of items 117 to 118, wherein the time interval between the administration of an administration unit and the administration of a subsequent administration unit is not more than 48 hours.
120. The packaging according to item 119, wherein the time interval is not more than 36 hours, or not more than 24 hours, or not more than 12 hours, or not more than 8 hours, or not more than 6 hours, or not more than 4 hours.
121. The packaging according to any of items 117 to 120, wherein the administration units are administered on not more than 30 subsequent days.
122. The packaging according to item 121, wherein the administration units are administered on not more than 25 subsequent days, or on not more than 20 subsequent days, or on not more than 14 subsequent days, or on not more than 10 subsequent days, or on not more than 7 subsequent days, or on not more than 3 subsequent days.
123. The packaging according to any of items 117 to 122, wherein the administered daily dose of (v) {drug5}, (vii) {drug7}, (viii) {drug8}, (ix) {drug9}, or (xi) {drug11}, respectively is 0.0001 mg/kg body weight to 100 mg/kg body weight.
124. The packaging according to item 123, wherein the administered daily dose of (v) {drug5}, (vii) {drug7}, (viii) {drug8}, (ix) {drug9}, or (xi) {drug11}, respectively is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.
125. The packaging according to any of items 117 to 124, wherein the administered daily dose of (v) {drug5}, (vii) {drug7}, (viii) {drug8}, (ix) {drug9}, or (xi) {drug11}, respectively is 0.001 mg to 5000 mg.
126. The packaging according to item 125, wherein the administered daily dose of (v) {drug5}, (vii) {drug7}, (viii) {drug8}, (ix) {drug9}, or (xi) {drug11}, respectively is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.
127. The packaging according to any of items 117 to 126, not comprising any administration unit comprising an active pharmaceutical ingredient other than (v) {drug5}, (vii) {drug7}, (viii) {drug8}, (ix) {drug9}, or (xi) {drug11}, respectively.
128. A method for manufacturing an administration unit according to any of items 1 to 109 or a packaging according to any of items 110 to 127, comprising the steps: (a) providing a quantity of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; that exceeds the dose of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; to be contained in a single administration unit by a factor of at least 10; (b) mixing the quantity of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; with one or more pharmaceutical excipients; (c) dividing the mixture obtained in step (b) in fractions each containing the dose of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; to be contained in a single administration unit; (d) optionally, forming the fractions obtained in step (c) to administration units; and (e) optionally, packing the fractions obtained in step (c) and the administration units obtained in step (d), respectively.
129. The method according to item 133, wherein in step (a) the quantity of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; exceeds the dose of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; to be contained in a single administration unit by a factor of at least 25, or at least 50, or at least 100, or at least 250, or at least 500, or at least 1000, or at least 2500, or at least 5000, or at least 10,000.
130. Particles of (v) {drug5a}; (vii) {drug7a} or {drug7b}; (viii) {drug8a}, {drug8b}, {drug8c}, {drug8d}, {drug8e}, {drug8f}, {drug8g}, {drug8h}, or {drug8i}; (ix) {drug9a}; (xi) {drug11a}, {drug11b}, {drug11c}, or {drug11d}; having a particle size distribution X90 of 500 µm or less.
131. The particles of (v) {drug5a}; (vii) {drug7a} or {dmg7b}; (viii) {drug8a}, {drug8b}, {drug8c}, {drug8d}, {dmg8e}, {dmg8f}, {dmg8g}, {drug8h}, or {drug8i}; (ix) {drug9a}; (xi) {drug11a}, {drug11b}, {drug11c}, or {drug11d}; according to item 130, having a particle size distribution X90 of 480 µm or less, or 460 µm or less, or 440 µm or less, or 420 µm or less, or 400 µm or less, of 380 µm or less, or 360 µm or less, or 340 µm or less, or 320 µm or less, or 300 µm or less, of 280 µm or less, or 260 µm or less, or 240 µm or less, or 220 µm or less, or 200 um or less, of 180 µm or less, or 160 µm or less, or 140 µm or less, or 120 µm or less, or 100 µm or less, or 80 µm or less, or 60 µm or less, or 40 µm or less, or 20 µm or less.
132. The particles of (v) {drug5a}; (vii) {drug7a} or {dmg7b}; (viii) {drug8a}, {drug8b}, {drug8c}, {drug8d}, {dmg8e}, {dmg8f}, {dmg8g}, {drug8h}, or {drug8i}; (ix) {drug9a}; (xi) {drug11a}, {drug11b}, {drug11c}, or {drug11d}; according to any of items 130 to 131, having a particle size distribution X50 of 400 µm or less.
133. The particles of (v) {drug5a}; (vii) {drug7a} or {dmg7b}; (viii) {drug8a}, {drug8b}, {drug8c}, {drug8d}, {dmg8e}, {dmg8f}, {dmg8g}, {drug8h}, or {drug8i}; (ix) {drug9a}; (xi) {drug11a}, {drug11b}, {drug11c}, or {drug11d}; according to item 132, having a particle size distribution X50 of 380 µm or less, or 360 µm or less, or 340 µm or less, or 320 µm or less, or 300 µm or less, of 280 µm or less, or 260 µm or less, or 240 µm or less, or 220 µm or less, or 200 µm or less, 180 µm or less, or 160 µm or less, or 140 µm or less, or 120 µm or less, or 100 µm or less, or 80 µm or less, or 70 µm or less, or 60 µm or less, or 50 µm or less.
134. The particles of (v) {drug5a}; (vii) {drug7a} or {dmg7b}; (viii) {drug8a}, {drug8b}, {drug8c}, {drug8d}, {dmg8e}, {dmg8f}, {dmg8g}, {drug8h}, or {drug8i}; (ix) {drug9a}; (xi) {drug11a}, {drug11b}, {drug11c}, or {drug11d}; according to item 133, having a particle size distribution X50 of 45 µm or less, or 40 µm or less, or 35 µm or less, or 30 µm or less, or 25 µm or less, or 20 µm or less, or 15 µm or less, or 10 µm or less.
135. The particles of (v) {drug5a}; (vii) {drug7a} or {dmg7b}; (viii) {drug8a}, {drug8b}, {drug8c}, {drug8d}, {dmg8e}, {dmg8f}, {dmg8g}, {drug8h}, or {drug8i}; (ix) {drug9a}; (xi) {drug11a}, {drug11b}, {drug11c}, or {drug11d}; according to any of items 130 to 134, having a cumulative distribution by volume basis Q3(X) (X in µm) of Q3(355) <0.50; or Q3(180) <0.50 and Q3(355) ≥0.50; or Q3(125) <0.50 and Q3(180) ≥0.50; or Q3(125) ≥0.50.
136. The particles of (v) {drug5a}; (vii) {drug7a} or {dmg7b}; (viii) {drug8a}, {drug8b}, {drug8c}, {drug8d}, {dmg8e}, {dmg8f}, {dmg8g}, {drug8h}, or {drug8i}; (ix) {drug9a}; (xi) {drug11a}, {drug11b}, {drug11c}, or {drug11d}; according to any of items 130 to 135, having a cumulative distribution by volume basis Q3(X) (X in µm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.
137. The particles of (v) {drug5a}; (vii) {drug7a} or {dmg7b}; (viii) {drug8a}, {drug8b}, {drug8c}, {drug8d}, {dmg8e}, {dmg8f}, {dmg8g}, {drug8h}, or {drug8i}; (ix) {drug9a}; (xi) {drug11a}, {drug11b}, {drug11c}, or {drug11d}; according to any of items 130 to 136, having a cumulative distribution by volume basis Q3(X) (X in µm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

## Claims

1. An administration unit comprising 0.1 µg to 2 g of a crystalline form or pharmaceutical acceptable salt of N-(6-((2R,3S)-3,4-dihydroxybutan-2-yloxy)-2-(4-fluorobenzylthio)pyrimidin-4-yl)-3-methylazetidine-1-sulfonamide

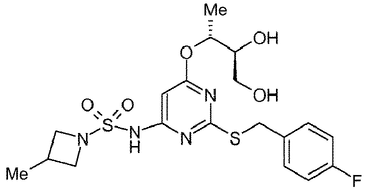

2. The administration unit according to claim 1, comprising 1 µg to 2.5 µg or 2.5 µg to 5 µg; or 5 µg to 10 µg; or 10 µg to 25 µg; or 25 µg to 50 µg; or 50 µg to 100 µg; or 100 µg to 250 µg; or 250 µg to 500 µg; or 500 µg to 1 mg; or 1 mg to 2.5 mg; or 2.5 mg to 5 mg; or 5 mg to 10 mg; or 10 mg to 25 mg; or 25 mg to 50 mg; or 50 mg to 100 mg; or 100 mg to 250 mg; or 250 mg to 500 mg; or 500 mg to 1 g; or 1 g to 1.25 g; or 1.25 g to 1.5 g; or 1.5 g to 1.75 g; or 1.75 g to 2 g of the crystalline form or pharmaceutical acceptable salt of N-(6-((2R,3S)-3,4-dihydroxybutan-2-yloxy)-2-(4-fluorobenzylthio)pyrimidin-4-yl)-3-methylazetidine-1-sulfonamide.

3. The administration unit according to any of claims 1 to 2, which is solid, semisolid or liquid.

4. The administration unit according to any of claims 1 to 3, which is for administration once daily, or twice daily, or thrice daily, or four times daily, or five times daily, or six times daily.

5. The administration unit according to any of claims 1 to 4, having a total weight of 10 mg to 3 g.

6. The administration unit according to claim 5, having a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.

7. The administration unit according to any of claims 1 to 6, which is for oral administration.

8. The administration unit according to claim 8, which is selected from the group consisting of tablets, capsules, effervescent tablets, orodispersible tablets, dragees, sachets, drops, suspensions, and powders.

9. The administration unit according to any of claims 1 to 8 for use in the treatment of a disorder or a disease in a mammal.

10. The administration unit according to any of claims 1 to 8, wherein the crystalline form or pharmaceutical acceptable salt of N-(6-((2R,3S)-3,4-dihydroxybutan-2-yloxy)-2-(4-fluorobenzylthio)pyrimidin-4-yl)-3-methylazetidine-1-sulfonamide has at least one peak at 2-Theta (in degrees) selected from 8.5, 9.7, 10.6, 17.1, 19.9, and 212

11. A packaging comprising one or more administration units according to any of claims 1 to 10.

12. The packaging according to claim 11, comprising a material selected from the group consisting of paper, cardboard, paperboard, metal foil and plastic foil.

13. The packaging according to any of claims 11 to 12, comprising a multitude of at least 2, or at least 3, or at least 4, or at least 5, or at least 6, or at least 7, or at least 8, or at least 9, or at least 10 administration units according to any of claims 1 to 10.

14. The packaging according to claim 13, wherein the administration units do not substantially differ in at least one property selected from the group consisting of nature of excipients, content of excipients, content of crystalline form or pharmaceutical acceptable salt of N-(6-((2R,3S)-3,4-dihydroxybutan-2-yloxy)-2-(4-tluorobenzylthio)pyrimidin-4-yl)-3-methylazetidine-1-sulfonamide; content of N-(6-((2R,3S)-3,4-dihydroxybutan-2-yloxy)-2-(4-fluorobenzyl-thio)pyrimidin-4-yl)-3-methylazetidine-1-sulfonamide, water content, total weight, release profile, disintegration time, size, shape, porosity, apparent density, true density, average pore size, pore size distribution, axial tensile strength, radial tensile strength, friability, and storage stability.

15. The packaging according to any of claims 13 to 15, wherein all administration units are substantially identical.
